# EUROPEAN PATENT SPECIFICATION

(11) **EP 2 678 315 B1**
(45) Date of publication and mention of the grant of the patent: **16.12.2015**
(21) Application number: 12704845.2
(22) Date of filing: 23.02.2012
(51) Int. Cl.: C07D 213/40, C07D 213/82, C07D 401/12, C07D 405/12, C07D 413/10, C07D 413/14, C07D 413/12, A61K 31/44, A61P 1/00, A61P 17/06, A61P 25/14, A61P 25/16, A61P 25/18, A61P 25/28, A61P 25/34

(54) **(Pyridin-4-yl)benzylamides as allosteric modulators of alpha 7 nAChR**
(Pyridin-4-yl-)benzylamide als allosterische Modulatoren von alpha-7-nAChR
(Pyridin-4-yl)benzylamides en tant que modulateurs allostériques d'alpha-7-nAChR

(30) Priority: 25.02.2011 EP 11155937
(43) Date of publication of application: 01.01.2014
(73) Proprietor: Janssen Pharmaceutica, N.V., 2340 Beerse (BE)
(72) Inventor: DE BOECK, Benoît, Christian, Albert, Ghislain, B-2340 Beerse (BE); ROMBOUTS, Geert, B-2340 Beerse (BE); LEENAERTS, Joseph, Elisabeth, B-2340 Beerse (BE); MACDONALD, Gregor, James, B-2340 Beerse (BE)
(74) Representative: Quaghebeur, Luc
(86) International application number: PCT/EP2012/053047
(87) International publication number: WO 2012/113850

(56) References cited:
- EP-A1- 1 845 081
- EP-A1- 2 284 159
- WO-A1-2006/096358
- WO-A1-2009/135944

## Description

### Field of the invention

The present invention relates to (pyridine-4-yl)benzylamides and pharmaceutically acceptable salts thereof, processes for preparing them, pharmaceutical compositions containing them and their use in therapy. The invention particularly relates to allosteric modulators of nicotinic acetylcholine receptors, such allosteric modulators having the capability to increase the efficacy of nicotinic receptor agonists.

### Background Prior Art

Allosteric modulators of nicotinic acetylcholine alpha 7 receptors have been disclosed in WO-2007/031440, WO-2007/118903, WO-2009/050186, WO-2009/050185, WO-2009/115547 and WO-2009/135944.

WO-2006/096358 discloses azabicycloalkane derivates as nicotinic acetylcholine receptor agonists.

### Background of the invention

Cholinergic receptors normally bind the endogenous neurotransmitter acetylcholine (ACh), thereby triggering the opening of ion channels. ACh receptors in the mammalian central nervous system can be divided into muscarinic (mAChR) and nicotinic (nAChR) subtypes based on the agonist activities of muscarine and nicotine, respectively. The nicotinic acetylcholine receptors are ligand-gated ion-channels containing five subunits. Members of the nAChR subunit gene family have been divided into two groups based on their amino acid sequences; one group containing so-called alpha subunits, and a second group containing beta subunits. Three kinds of alpha subunits, alpha 7, alpha 8 and alpha 9, have been shown to form functional receptors when expressed alone and thus are presumed to form homooligomeric pentameric receptors.

An allosteric transition state model of the nAChR has been developed that involves at least a resting state, an activated state and a "desensitized" closed channel state, a process by which receptors become insensitive to the agonist. Different nAChR ligands can stabilize the conformational state of a receptor to which they preferentially bind. For example, the agonists ACh and (-)-nicotine respectively stabilize the active and desensitized states.

Changes of the activity of nicotinic receptors have been implicated in a number of diseases. Some of these, for example myasthenia gravis and autosomal dominant nocturnal front lobe epilepsy (ADNFLE) are associated with reductions in the activity of nicotinic transmission either because of a decrease in receptor number or increased desensitization.

Reductions in nicotinic receptors have also been hypothesized to mediate cognitive deficits seen in diseases such as Alzheimer's disease and schizophrenia.

The effects of nicotine from tobacco are also mediated by nicotinic receptors and since the effect of nicotine is to stabilize receptors in a desensitized state, an increased activity of nicotinic receptors may reduce the desire to smoke.

Compounds which bind nAChRs have been suggested for the treatment of a range of disorders involving reduced cholinergic function such as learning deficit, cognition deficit, attention deficit and memory loss. Modulation of alpha 7 nicotinic receptor activity is expected to be beneficial in a number of diseases including Alzheimer's disease, Lewy Body Dementia, Attention Deficit Hyperactivity Disorder, anxiety, schizophrenia, mania, bipolar disorder, Parkinson's disease, Huntington's disease, Tourette's syndrome, brain trauma and other neurological, degenerative and psychiatric disorders in which there is loss of cholinergic synapses, including jetlag, nicotine addiction, and pain.

However, treatment with nicotinic receptor agonists which act at the same site as ACh is problematic because ACh not only activates, but also blocks receptor activity through processes which include desensitization and uncompetitive blockade. Furthermore, prolonged activation appears to induce a long-lasting inactivation. Therefore, agonists of ACh can be expected to lose effectiveness upon chronic administration.

At nicotinic receptors in general, and of particular note at the alpha 7 nicotinic receptor, desensitization limits the duration of action of an applied agonist.

### Description of the invention

We have found that certain novel (pyridine-4-yl)benzylamides can increase the efficacy of agonists at nicotinic acetylcholine receptors (nAChR). Compounds having this type of action (hereinafter referred to as "positive allosteric modulators") are likely to be useful for treatment of conditions associated with reductions in nicotinic transmission In a therapeutic setting such compounds could restore normal interneuronal communication without affecting the temporal profile of activation. In addition, positive allosteric modulators are not expected to produce long-term inactivation of receptors as may occur with prolonged application of agonists.

Positive nAChR modulators of the present invention are useful for treatment and prophylaxis of psychotic disorders, intellectual impairment disorders and diseases, inflammatory diseases and conditions in which modulation of the alpha 7 nicotinic receptor is beneficial.

The present invention concerns (pyridine-4-yl)benzylamides having positive allosteric modulator properties, in particular increasing the efficacy of agonists at the alpha 7 nicotinic receptor. The invention further relates to methods for their preparation and pharmaceutical compositions comprising them. The invention also relates to the use of these derivatives for the manufacture of a medicament for the treatment and prophylaxis of psychotic disorders, intellectual impairment disorders and diseases, inflammatory diseases and conditions in which modulation of the alpha 7 nicotinic receptor is beneficial. The invention further relates to these derivatives for use in the treatment and prophylaxis of psychotic disorders, intellectual impairment disorders and diseases, inflammatory diseases and conditions in which modulation of the alpha 7 ' nicotinic receptor is beneficial.

In a first aspect, the present invention relates to a compound having the formula (I) 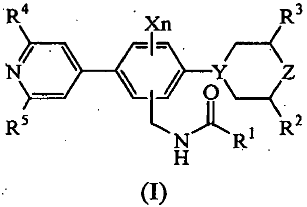 or a stereoisomer thereof, wherein
n is 0,1 or 2;
X is fluoro or chloro;
Y is N or CH;
Z is O or CH₂;
R¹ is C₁₋₈alkyl, C₁₋₈alkyl substituted with 1, 2 or 3 halogen substituents; C₃₋₆cycloalkyl; (C₃₋₆cycloalkyl)C₁₋₆alkyl; (C₁₋₆alkyloxy)C₁₋₆alkyl; (trihaloC₁₋₄alkyloxy)C₁₋₆alkyl; tetrahydrofuryl; tetrahydropyranyl; phenyl; phenyl substituted with 1, 2 or 3
substituents selected from halogen, trifluoromethyl, trifluoromethoxy, cyano, C₁₋₆alkyl, and C₁₋₄alkyloxy; or a monocyclic aromatic heterocyclic radical containing at least one heteroatom selected from N, O and S, optionally substituted with 1, 2 or where possible with 3 substituents selected from halogen, C₁₋₄alkyl, C₁₋₄alkyloxy, C₃₋₆cycloalkyl, and trifluoromethyl;
R² and R³ are independently H, C₁₋₄alkyl or trifluoromethyl;
or R² and R³ are taken together to form 1,2-ethanediyl or 1,3-propanediyl;
R⁴ and R⁵ are independently H, C₁₋₄alkyl, trifluoromethyl, C₃₋₆cycloalkyl or C₁₋₄alkyloxy;
or an acid addition salt thereof, or a solvate thereof.

In one embodiment, R¹ is C₁₋₆alkyl; C₃₋₆cycloalkyl; cyclopropyl substituted with 1, 2, 3, or 4 methyl groups; (C₃₋₆cycloalkyl)C₁₋₂alkyl; methoxymethyl; phenyl substituted with 1, 2, or 3 substitents selected from fluoro, chloro, methyl, methoxy, trifluoromethyl, trifluoromethoxy, cyano, and aminosulfonyl; or furanyl, oxazolyl, isoxazolyl, oxadiazolyl, pyrrolyl, pyrazolyl, imidazolyl, pyridinyl, pyridiminyl, pyrazinyl, pyridazinyl or benzisoxazolyl, each unsubstituted or substituted with 1, 2 or where possible 3 substituents selected from methyl, ethyl, propyl, isopropyl; butyl, isobutyl, tert-butyl, cyclopropyl, methoxy, and trifluoromethyl.

In another embodiment, R¹ is C₁₋₆alkyl; C₁₋₄alkyl substituted with 3 fluoro substituents; C₃₋₆cycloalkyl; (C₃₋₆cycloalkyl)C₁₋₂alkyl; methoxymethyl;.methoxyethyl, (2,2,2-trifluoroethoxy)methyl; tetrahydropyranyl; phenyl; or phenyl substituted with 1, 2 or 3 substituents selected from fluoro, chloro, trifluoromethyl, trifluoromethoxy, cyano, methyl, and inethoxy; or furanyl, oxazolyl, isoxazolyl, oxadiazolyl, pyrrolyl, pyrazolyl, imidazolyl, pyridinyl, pyridiminyl, pyrazinyl, pyridazinyl, thienyl, 1,2,3-thiadiazolyl, thiazolyl or benzisoxazolyl, each unsubstituted or substituted with 1, 2 or where possible 3 substituents selected from methyl, ethyl, propyl, isopropyl, n-butyl, isobutyl, sec-butyl, tert-butyl, cyclopropyl, methoxy and trifluoromethyl.

In another embodiment, R¹ is furanyl, oxazolyl, isoxazolyl, pyrazolyl, pyridinyl, pyrazinyl, thienyl, 1,2,3-thiadiazolyl, thiazolyl or benzisoxazolyl, each unsubstituted or substituted with 1, 2 or where possible 3 substituents selected from methyl, isopropyl, tert-butyl, cyclopropyl, methoxy and trifluoromethyl.

In another embodiment R² is hydrogen, methyl or trifluoromethyl.

In another embodiment R³ is hydrogen, methyl or trifluoromethyl.

In another embodiment R⁴ is hydrogen or methyl

In another embodiment R⁵ is hydrogen or methyl.

In another embodiment, R¹ is methyl, ethyl, *n*-propyl, isopropyl, *n*-butyl, isobutyl, sec-butyl, tert-butyl, cyclopropyl, cyclobutyl, cyclopentyl, (cyclopropyl)ethyl, (cyclopropyl)methyl, (cyclobutyl)methyl, (cyclohexyl)methyl, 3-methyl-isoxazol-5-yl, 3-methyl-isoxazol-4-yl, 5-methyl-isoxazol-3-yl, 2-methyl-5-trifluoromethyl-oxazol-4-yl, or 2-methyl-oxazol-4-yl.

In another embodiment, R¹ is methyl, ethyl, *n*-propyl, isopropyl, *n*-butyl, isobutyl, sec-butyl, tert-butyl, 2,2,2-trifluorethyl, 3,3,3-trifluoropropyl, 2-methoxyethyl, cyclopropyl, cyclobutyl, cyclopentyl, 1-(cyclopropyl)ethyl, (cyclopropyl)methyl, (cyclobutyl)methyl, 4-fluoro-2-methylphenyl, 3-methyl-isoxazol-5-yl, 3-methyl-isoxazol-4-yl, 5-methyl-isoxazol-3-yl, 2-methyl-5-trifluoromethyl-oxazol-4-yl, or 2-methyl-oxazol-4-yl.

In another embodiment, R¹ is isopropyl, cyclopropyl (cyclopropyl)methyl, (cyclobutyl)methyl, 3-methyl-4-isoxazolyl or 5-methyl-isoxazol-3-yl.

In another embodiment, R² and R³ are methyl or trifluoromethyl and have the cis-configuration.

In another embodiment, R² and R³ are methyl and have the trans-configuration.

In a preferred embodiment the compound is chosen from
N-[[5-[(2R,6S)-2,6-dimethyl-4-morpholinyl]-2-(2,6-dimethyl-4-pyridinyl)phenyl]methyl]-cyclopropaneacetamide,
N-[[5-[(2R,6S)-2,6-dimethyl-4-morpholinyl]-2-(2,6-dimethyl-4-pyridinyl)phenyl]methyl]-5-methyl-3-isoxazolecarboxamide,
N-[[5-[(2R,6S)-2,6-dimethyl-4-morpholinyl]-2-(2,6-dimethyl-4-pyridinyl)phenyl]methyl]-3-methyl-4-isoxazolecarboxamide,
N-[[5-[(2R,6S)-2,6-dimethyl-4-morpholinyl]-2-(2,6-dimethyl-4-pyridinyl)phenyl]methyl]-4-fluoro-2-methyl-benzamide,
N-[[5-[(2R,6S)-2,6-dimethyl-4-morpholinyl]-2-(2,6-dimethyl-4-pyridinyl)phenyl]methyl]-2-methyl-propanamide,
N-[[5-[(2R,6S)-2,6-dimethyl-4-morpholinyl]-2-(2,6-dimethyl-4-pyridinyl)phenyl]methyl]-cyclopropanecarboxamide,
N-[[5-[(2R,6S)-2,6-dimethyl-4-morpholinyl]-2-(2,6-dimethyl-4-pyridinyl)phenyl]methyl]-3,3,3-trifluoro-propanamide,
N-[[3-[(2R,6S)-2,6-dimethyl-4-morpholinyl)-6-(2,6-dimethyl-4-pyridinyl)-2-fluorophenyl]methyl]-cyclopropaneacetamide,
N-[[3-[(2R,6S)-2,6-dimethyl-4-morpholinyl]-6-(2,6-dimethyl-4-pyridinyl)-2-fluorophenyl]methyl]-2-methyl-propanamide,
N-[[3-[(2R,6S)-2,6-dimethyl-4-morpholinyl]-6-(2,6-dimethyl-4-pyridinyl)-2-fluorophenyl]methyl]-5-methyl-3-isoxazolecarboxamide,
N-[[3-[(2R,6S)-2,6-dimethyl-4-morpholinyl]-6-(2,6-dimethyl-4-pyridinyl)-2-fluorophenyl]methyl]-3-methyl-4-isoxazolecarboxamide,
N-[[3-[(2R,6S)-2,6-dimethyl-4-morpholinyl]-6-(2,6-dimethyl-4-pyridinyl)-2-fluorophenyl]methyl]-4-fluoro-2-methyl-benzamide,
N-[[5-[(2R,6S)-2,6-dimethyl-4-morpholinyl]-2-(2,6-dimethyl-4-pyridinyl)-3-fluorophenyl]methyl]-cyclopropaneacetamide,
N-[[5-[(2R,6S)-2,6-dimethyl-4-morpholinyl]-2-(2,6-dimethyl-4-pyridinyl)-3-fluorophenyl]methyl]-5-methyl-3-isoxazolecarboxamide,
N-[[S-[(2R,6S)-2,6-dimethyl-4-morpholinyl]-2-(2,6-dimethyl-4-pyridinyl)-3-fluorophenyl]methyl]-3-methyl-4-isoxazolecarboxamide,
N-[[5-[(2R,6S)-2,6-dimethyl-4-morpholinyl]-2-(2,6-dimethyl-4-pyridinyl)-3-fluorophenyl]methyl]-3,3,3-trifluoro-propanamide,
N-[[5-[(2R,6S)-2,6-dimethyl-4-morpholinyl]-2-(4-pyridinyl)phenyl]methyl]-2-methyl-propanamide
N-[[5-[(2R,6S)-2,6-dimethyl-4-morpholinyl]-2-(2,6-dimethyl-4-pyridinyl)-3-fluorophenyl]methyl]-2-methyl-propanamide,
N-[[5-[(2R,6S)-2,6-dimethyl-4-morpholinyl]-2-(4-pyridinyl)phenyl]methyl]-cyclopropaneacetamide,
N-[[5-[(2R,6S)-2,6-dimethyl-4-morph6linyl]-2-(2-methyl-4-pyridinyl)phenyl]methyl]-2-methyl-propanamide,
N-[[5-[(2R,6S)-2,6-dimethyl-4-morpholinyl]-2-(2-methyl-4-pyridinyl)phenyl]methyl]-5-methyl-3-isoxazolecarboxamide,
N-[[5-[(3R,SS)-3,5-dimethyl-1-piperidinyl]-2-(2,6-dimethyl-4-pyridinyl)phenyl]methyl]-5-methyl-3-isoxazolecarboxamide,
N-[[5-[(2R,6S)-2,6-dimethyl-4-morpholinyl]-2-(2-methyl-4-pyridinyl)phenyl]methyl]-4-fluoro-2-methyl-benzamide,
N-[[5-[(2R,6S)-2,6-dimethyl-4-morpholinyl]-2-(4-pyridinyl)phenyl]methyl]-3-methyl-4-isoxazolecarboxamide,
N-[[2-(2,6-dimethyl-4-pyridinyl)-5-(4-morpholinyl)phenyl]methyl]-cyclopropaneacetamide,
N-[[2-(2,6-dimethyl-4-pyridinyl)-5-(4-morpholinyl)phenyl]methyl]-5-methyl-3-isoxazolecarboxamide,
N-[[2-(2,6-dimethyl-4-pyridinyl)-5-(4-morpholinyl)phenyl]methyl]-3-methyl-4-isoxazolecarboxamide,
N-[[2-(2,6-dimethyl-4-pyridinyl)-5-(4-morpholinyl)phenyl]methyl]-4-fluoro-2-methyl-benzamide,
N-[[2-(2,6-dimethyl-4-pyridinyl)-5-(4-morpholinyl)phenyl]methyl]-3,3,3-trifluoro-propanamide,
N-[[2-(2,6-dimethyl-4-pyridinyl)-5-(4-morpholinyl)phenyl]methyl]-2-methyl-propanamide,
N-[[2₋(2,6-dimethyl-4-pyridinyl)-5-(4-morpholinyl)phenyl]methyl]-cyclopropanecarboxamide,
N-[[5-[(2R,6S)-2,6-dimethyl-4-morpholinyl]-2-(2-methyl-4-pyridinyl)phenyl]methyl]-cyclopropaneacetamide,
4-fluoro-2-methyl-N-[[2-(2-methyl-4-pyridinyl)-5-(4-morpholinyl)phenyl]methyl]-benzamide,
N-[[5-[(3R,5S)-3,5-bis(trifluoromethyl)-1-piperidinyl]-2-(2,6-dimethyl-4-pyridinyl)phenyl]methyl]-3-methyl-4-isoxazolecarboxamide,
N-[[5-[(3R,5S)-3,5-bis(trifluoromethyl)-1-piperidinyl]-2-(2,6-dimethyl-4-pyridinyl)phenyl]methyl]-4-fluoro-2-methyl-benzamide,
N-[[5-[(3R,5S)-3,5-bis(trifluoromethyl)-1-piperidinyl]-2-(2,6-dimethyl-4-pyridinyl)phenyl]methyl]-5-methyl-3-isoxazolecarboxamide,
N-[[5-[(3R,5S)-3,5-bis(trifluoromethyl)-1-piperidinyl]-2-(2,6-dimethyl-4-pyridinyl)phenyl]methyl]-cyclopropaneacetamide,
N-[[5-[(3R,5S)-3,5-bis(trifluoromethyt)-1-pipendinyl]-2-(2,6-dimethyl-4-pyridinyl)phenyl]methyl]-2-methyl-propanamide,
N-[[5-[(2R,6S)-2,6-diinethyl-4-moipholinyl]-2-(2,6-dimethyl-4-pyridinyl)-3-fluorophenyl]methyl]-alpha-methyl-cyclopcopaneacetamide,
N-[[5-[(2R,6S)-2,6-dimethyl-4-morpholinyl]-2-(2,6-dimethyl-4-pyridinyl)-3-fluorophenyl]methyl]-cyclopropanecarboxamide,
N-[[2-(2,6-dimethyl-4-pyridinyl)-5-[3-(trifluoromethyl)-l-piperidinyl]phenyl]methyl]-3-methyl-4-isoxazolecarboxamide,
N-[[2-(2,6-dimethyl-4-pyridinyl)-5-[3-(trifluoromethyl)-1-pipefidinyl]phenyl]methyl]-4-fluoro-2-methyl-benzamide,
N-[[2-(2,6-dimethyl-4-pyridinyl)-5-[3-(trifluoromethyl)-1-piperidinyl]phenyl]methyl]-5-methyl-3-isoxazolecarboxamide,
N-[[2-(2,6-dimethyl-4-pyridinyl)-5-[3-(trifluoromethyl)-1-piperidinyl]phenyl]methy]]-cyclopropaneacetamide,
N-[[2-(2,6-dimethyl-4-pyridinyl)-5-[3-(trifluoromethyl)-1-piperidinyl]phenyl]methyl]-2-methyl-propanamide,
N-[[2-(2,6-dimethyl-4-pyridinyl)-3-fluoro-5-[3-(trifluoromethyl)-1-piperidinyl]phenyl]methyl]-alpha-methyl-cyclopropaneacetamide,
N-[[2-(2,6-dimethyl-4-pyridinyl)-3-fluoro-5-[3-(trifluoromethyl)-1-piperidinyl]phenyl]methyl]-5-methyl-3-isoxazolecarboxamide,
N-[[2-(2,6-dimethyl-4-pyridinyl)-3-fluoro-5-[3-(trifluoromethyl)-1-piperidinyl]phenyl]methyl]-cyclopropaneacetamide,
N-[[2-(2,6-dimethyl-4-pyridinyl)-3-fluoro-5-[3-(trifluoromethyl)-1-piperidinyl]phenyl]methyl]-2-methyl-propanamide,
N-[[2-(2,6-dimethyl-4-pyridinyl)-3-fluoro-5-[3-(trifluoromethyl)-1-piperidinyl]phenyl]methyl]-cyclopropanecarboxamide,
N-[[2-(2,6-dimethyl-4-pyridinyl)-3-fluoro-5-[3-(trifluoromethyl)-1-piperidinyl]phenyl]methyl]-3-methyl-4-isoxazolecarboxamide,
N-[[5-[(2R,6S)-2,6-dimethyl-4-morpholinyl]-2-(2,6-dimethyl-4-pyridinyl)-3-fluorophenyl]methyl]-cyclobutaneacetamide,
N-[[5-[(2R,6S)-2,6-dimethyl-4-morpholiriyl]-2-(2,6-dimethyl-4-pyridinyl)-3-fluorophenyl]methyl]-4,4,4-trifluoro-butanamide,
N-[[5-[(2R,6S)-2,6-dimethyl-4-morpholinyl]-2-(2,6-diinethyl-4-pyridinyl)-3-fluorophenyl]methyl]-2-(2,2,2-trifluoroethoxy)-acetamide,
N-[[5-[(2R,6S)-2,6-dimethyl-4-morpholinyl]-2-(2,6-dimethyl-4-pyridinyl)-3-fluorophenyl]methyl]-3-methyl-butanamide,
N-[[5-[(2R,6S)-2,6-dimethyl-4-morpholinyl]-2-(2,6-dimethyl-4-pyridinyl)-3-fluorophenyl]methyl]-3,3-dimethyl-butanamide,
N-[[5-[(2R,6S)-2,6-dimethyl-4-morpholinyl]-2-(2,6-dimethyl-4-pyridinyl)-3-fluorophenyl]methyl]-cyclobutanecarboxamide,
N-[[3-[(2R,6S)-2,6-dimethyl-4-morpholinyl]-6-(2,6-dimethyl-4-pyridinyl)-2-fluorophenyl]methyl]-cyclopropanecarboxamide,
N-[[3-[(2R,6S)-2)6-dimethyl-4-morpholinyl]-6-(2,6-dimethyl-4-pyridinyl)-2-fluorophenyl]methyl]-3-methyl-butanamide,
N-[[5-[(2R,6S)-2,6-dimethyl-4-morpholinyl]-3-fluoro-2-(2-methyl-4-pyridinyl)phenyl]methyl]-cyclopropaneacetamide,
N-[[5-[(2R,6S)-2,6-dimethyl-4-morpholinyl]-3-fluoro-2-(2-methyl-4-pyridinyl)phenyl]methyl]-cyclopropanecarboxamide,
N-[[5-[(2R,6S)-2,6-dimethyl-4-morpholinyl]-3-fluoro-2-(2-methyl-4-pyridinyl)phenyl]methyl]-2-methyl-propanamide,
N-[[5-[(2R,6S)-2,6-dimethyl-4-morpholinyl]-3-fluoro-2-(2-methyl-4-pyridinyl)phenyl]methyl]-5-methyl-3-isoxazolecarboxamide,
N-[[5-[(2R,6S)-2,6-dimethyl-4-morpholinyl]-3-fluoro-2-(2-methyl-4-pyridinyl)phenyl]methyl]-3-methyl-4-isoxazolecarboxamide,
N-[[5-[(2R,6S)-2,6-dimethyl-4-morpholinyl]-3-fluoro-2-(2-methyl-4-pyridinyl)phenyl]methyl]-3-methyl-butanamide,
N-[[5-[(2R,6S)-2,6-dimethyl-4-morpholinyl]-2-(2,6-dimethyl-4-pyridinyl)-3-fluorophenyl]methyl]tetrahydro-2H-pyran-4-carboxamide,
N-[[5-[(2R,6S)-2,6-dimethyl-4-morpholinyl]-2-(2-methyl-4-pyndinyl)phenyl]methyl]-cyclopropanecarboxamide,
N-[[6-(2,6-dimethyl-4-pyridinyl)-2=fluoro-3-[(2R,6S)-tetrahydro-2,6-dimethyl-2H-pyran-4-yl]phenyl]methyl]-cyclopropaneacetamide,
N-[[6-(2,6-dimethyl-4-pyridinyl)-2-fluoro-3-[(2R)-2-(trifluoromethyl)-4-morpholinyl]phenyl]methyl]-2-methyl-propanamide,
N-[[6-(2,6-dimethyl-4-pyridinyl)-2-fluoro-3-[(2R)-2-(trifluoromethyl)-4-morpholinyl]phenyl]methyl]-5-methyl-3-isoxazolecarboxamide,
N-[[6-(2,6-dimethyl-4-pyridinyl)-2-fluoro-3-[(2R)-2-(trifluoromethyl)-4-morpholinyl]phenyl]methyl]-3-methyl-4-isoxazolecarboxamide,
N-[[6-(2,6-dimethyl-4-pyridinyl)-2-fluoro-3-[(2R)-2-(trifluoromethyl)-4-morpholinyl]phenyl]methyl]-cyclopropaneacetamide,
N-[[6-(2,6-dimethyl-4-pyridinyl)-2-fluoro-3-(tetrahydro-2,6-dimethyl-2H-pyran-4-yl)phenyl]methyl]-cyclobutaneacetamide,
N-[[6-(2,6-dimethyl-4-pyridinyl)-2-fluoro-3-(tetrahydro-2,6-dimethyl-2H-pyran-4-yl)phenyl]methyl]-2-methyl-propanamide,
N-[[6-(2,6-dimethyl-4-pyridinyl)-2-fluoro-3-(tetrahydro-2,6-dimethyl-2H-pyran-4-yl)phenyl]methyl]-5-methyl-3-isoxazolecarboxamide,
N-[[6-(2,6-dimethyl-4-pyridinyl)-2-fluoro-3-(tetrahydro-2,6-dimethyl-2H-pyran-4-yl)phenyl]methyl]-3-methyl-4-isoxazolecarboxamide,
N-[[6-(2,6-dimethyl-4-pyridinyl)-2-fluoro-3-[(2R,6S)-tetrahydro-2,6-dimethyl-2H-pyran-4-yl]phenyl]methyl]-cyclopropanecarboxamide,
N-[[6-(2,6-dimethyl-4-pyridinyl)-2-fluoro-3-[(2S)-2-(trifluoromethyl)-4-morpholinyl]phenyl]methyl]-cyclopropaneacetamide,
N-[[6-(2,6-dimethyl-4-pyridinyl)-2-fluoro-3-[(2R)-2-(trifluoromethyl)-4-morpholinyl]phenyl]methyl]-cyclobutaneacetamide,
N-[[6-(2,6-dimethyl-4-pyridinyl)-2-fluoro-3-[(2R)-2-(trifluoromethyl)-4-morpholinyl]phenyl]methyl]-3-methyl-butanamide,
N-[[2-(2,6-dimethyl-4-pyridinyl)-3-fluoro-5-[(2R)-2-(trifluoromethyl)-4-morpholinyl]phenyl]methyl]-2-methyl-propanamide,
N-[[2-(2,6-dimethyl-4-pyridinyl)-3-fluoro-5-[(2R)-2-(trifluoromethyl)-4-morpholinyl]phenyl]methyl]-5-methyl-3-isoxazolecarboxamide,
N-[[2-(2,6-dimethyl-4-pyridinyl)-3-fluoro-5-[(2R)-2-(trifluoromethyl)-4-morpholinyl]phenyl]inethyl]-3-methyl-butanamide,
N-[[6-(2,6-dimethyl-4-pyridinyl)-2-fluoro-3-[(2R,6S)-tetrahydro-2,6-dimethyl-2H-pyran-4-yl]phenyl]methyl]-3-methyl-butanamide,
N-[[6-(2,6-dimethyl-4-pyridinyl)-2-fluoro-3-[(2S)-2-(trifluoromethyl)-4-morpholinyl]phenyl]methyl]-2-methyl-propanamide,
N-[[2-(2,6-dimethyl-4-pyridinyl)-3-fluoro-5-[(2R)-2-(trifluoromethyl)-4-morpholinyl]phenyl]methyl]-cyclopropanecarboxamide,
N-[[2-(2,6-dimethyl-4-pyridinyl)-3-fluoro-5-[(2R)-2-(trifluoromethyl)-4-morpholinyl]phenyl]methyl]-3-methyl-4-isoxazolecarboxamide,
N-[[6-(2,6-dimethyl-4-pyridinyl)-2-fluoro-3-[(2R)-2-(trifluoromethyl)-4-morpholinyl]phenyl]methyl]-cyclopropanecarboxamide,
N-[[S-[(2R,6S)-2,6-dimethyl-4-morpholinyl]-2-(2-methyl-4-pyridinyl)phenyl]methyl]-3-methyl-4-isoxazolecarboxamide. 1.7HCl,
N-[[2-(2,6-dimethyl-4-pyridinyl)-3-fluoro-5-[(2R)-2-(trifluoromethyl)-4-morpholinyl]phenyl]methyl]-cyclopropaneacetamide,
N-[[6-(2,6-dimethyl-4-pyridinyl)-2-fluoro-3-[(2S)-2-(trifluoromethyl)-4-morpholinyl]phenyl]methyl]-3-methyl-4-isoxazolecarboxamide,
N-[[5-[(2R,6S)-2,6-dimethyl-4-morpholinyl]-2-(2,6-dimethyl-4-pyridinyl)-4-fluorophenyl]methyl]-cyclopropanecarboxamide,
N-[[5-[(2R,6S)-2,6-dimethyl-4-morpholinyl]-2-(2,6-dimethyl-4-pyridinyl)-4-fluorophenyl]methyl]-cyclopropaneacetamide,
N-[[5-[(2R,6S)-2,6-dimethyl-4-morpholinyl]-2-(2,6-dimethyl-4-pyridinyl)-4-fluorophenyl]methyl]-5-methyl-3-isoxazolecarboxamide,
N-[[5-[(2R,6S)-2,6-dimethyl-4-morpholinyl]-2-(2,6-dimethyl-4-pyridinyl)-4-fluorophenyl]methyl]-2-methyl-propanamide,
N-[[2-(2,6-dimethyl-4-pyridinyl)-3-fluoro-5-[(2S)-2-(trifluoromethyl)-4-morpholinyl]phenyl]methyl]-cyclobutaneacetamide,
N-[[2-(2,6-dimethyl-4-pyridinyl)-3-fluoro-5-(2-methyl-4-morpholinyl)phenyl]methyl]-cyclobutaneacetamide,
N-[[2-(2,6-dimethyl-4-pyridinyl)-3-fluoro-5-(2-methyl-4-morpholinyl)phenyl]methyl]-3-methyl-4-isoxazolecarboxamide,
N-[[2-(2,6-dimethyl-4-pyridinyl)-3-fluoro-5-[(2S)-2-(trifluoromethyl)-4-morpholinyl]phenyl]methyl]-3-methyl-4-isoxazolecarboxamide,
N-[[2-(2,6-dimethyl-4-pyridinyl)-3-fluoro-5-[(2S)-2-(trifluoromethyl)-4-morpholinyl]phenyl]methyl]-5-methyl-3-isoxazolecarboxamide,
N-[[2-(2,6-dimethyl-4-pyridinyl)-3-fluoro-5-(2-methyl-4-morpholinyl)phenyl]methyl]-5-methyl-3-isoxazolecarboxamide,
N-[[2-(2,6-dimethyl-4-pyridinyl)-3-fluoro-5-(2-methyl-4-morpholinyl)phenyl]methyl]-2-methyl-propanamide,
N-[[2-(2,6-dimethyl-4-pyridinyl)-3-f]uoro-5-[(2S)-2-(trifluoromethyl)-4-morpholinyl]phenyl]methyl]-2-methyl-propanamide,
N-[[2-(2,6-dimethyl-4-pyridinyl)-3-fluoro-5-(2-methyl-4-morpholinyl)phenyl]methyl]-cyclopropaneacetamide,
N-[[2-(2,6-dimethyl-4-pyridinyl)-3-fluoro-5-[(2S)-2-(trifluoromethyl)-4-morpholinyl]phenyl]methyl]-cyclopropaneacetamide,
N-[[2-(2,6-dimethyl-4-pyridinyl)-3-fluoro-5-(2-methyl-4-morpholinyl)phenyl]methyl]-cyclopropanecarboxamide,
N-[[2-(2,6-dimethyl-4-pyridinyl)-3-fluoro-5-[(2S)-2-(trifluoromethyl)-4-morpholinyl]phenyl]methyl]-cyclopropanecarboxamide,
N-[[2-(2,6-dimethyl-4-pyridinyl)-3-fluoro-5-(2-methyl-4-morpholinyl)phenyl]methyl]-3-methyl-butanamide,
N-[[2-(2,6-dimethyl-4-pyridinyl)-3-fluoro-5-[(2S)-2-(trifluoromethyl)-4-morpholinyl]phenyl]methyl]-3-methyl-butanamide,
N-[[3-[(2R,6S)-2,6-dimethyl-4-morpholinyl]-6-(2,6-dimethyl-4-pyridinyl)-2,5-difluorophenyl]methyl]-cyclopropanecarboxamide,
N-[[3-[(2R,6S)-2,6-dimethyl-4-morpholinyl]-6-(2,6-dimethyl-4-pyridinyl)-2,5-difluorophenyl]methyl]-5-methyl-3-isoxazolecarboxamide,
N-[[3-[(2R,6S)-2,6-dimethyl-4-morpholinyl]-6-(2,6-dimethyl-4-pyridinyl)-2,5-difluorophenyl]methyl]-cyclopropaneacetamide,
N-[[3-[(2R,6S)-2,6-dimethyl-4-morpholinyl]-6-(2,6-dimethyl-4-pyridinyl)-2-fluorophenyl]methyl]-5,5,5-trifluoro-pentanamide,
N-[[3-[(2R,6S)-2,6-dimethyl-4-morpholinyl]-6-(2,6-dimethyl-4-pyridinyl)-2-fluorophenyl]methyl]-cyclobutaneacetamide,
N-[[3-[(2R,6S)-2,6-dimethyl-4-morpholinyl]-6-(2,6-dimethyl-4-pyridinyl)-2-fluorophenyl]methyl]-cyclohexanecarboxamide,
N-[[3-[(2R,6S)-2,6-dimethyl-4-morpholinyl]-6-(2,6-dimethyl-4-pyridinyl)-2-fluorophenyl]methyl]-3-methoxy-propanamide,
N-[[3-[(2R,6S)-2,6-dirnethyl-4-morpholinyl]-6-(2,6-dimethyl-4-pyridinyl)-2-fluorophenyl]methyl]-3,3-dimethyl-butanamide,
N-[[3-[(2R,6S)-2,6-diinethyl-4-morpholinyl]-6-(2,6-dimethyl-4-pyridinyl)-2-fluorophenyl]methyl]-cyclobutanecarboxamide,
N-[[3-[(2R,6S)-2,6-dimethyl-4-morpholinyl]-6-(2,6-dimethyl-4-pyridinyl)-2-fluorophenyl]methyl]-alpha-methyl-cyclopropaneacetamide,
4,6-dichloro-N-[[3-[(2R,6S)-2,6-dimethyl-4-morpholinyl]-6-(2,6-dimethyl-4-pyridinyl)-2-fluorophenyl]methyl]-3-pyridinecarboxamide,
3-chloro-N-[[3-[(2R,6S)-2,6-dimethyl-4-morpholinyl]-6-(2,6-dimethyl-4-pyridinyl)-2-fluorophenyl]methyl]-2-fluoro-benzamide,
N-[[6-(2,6-dimethyl-4-pyridinyl)-2-fluoro-3-[(2S)-2-(trifluoromethyl)-4-morpholinyl]phenyl]methyl]-5-methyl-3-isoxazolecarboxamide,
N-[[6-(2,6-dimethyl-4-pyridinyl)-2-fluoro-3-[(2S)-2-(trifluoromethyl)-4-morpholinyl]phenyl]methyl]-3-methyl-butanamide,
N-[[6-(2,6-dimethyl-4-pyridinyl)-2-fluoro-3-[(2S)-2-(trifluoromethyl)-4-morpholinyl]phenyl]methyl]-cyclopropanecarboxamide,
N-[[6-(2,6-dimethyl-4-pyridinyl)-2-fluoro-3-[(2S)-2-(trifluoromethyl)-4-morpholinyl]phenyl]methyl]-cyclobutaneacetamide,
N-[[3-chloro-5-[(2R,6S)-2,6-dimethyl-4-morpholinyl]-2-(2,6-dimethyl-4-pyridinyl)phenyl}methyl]-2-methyl-propanamide,
N-[[3-chloro-5-[(2R,6S)-2,6-dimethyl-4-morpholinyl]-2-(2,6-dimethyl-4-pyridinyl)phenyl]methyl]-cyclopropaneacetamide,
N-[[3-chloro-5-[(2R,6S)-2,6-dimethyl-4-morpholinyl]-2-(2,6-dimethyl-4-pyridinyl)phenyl]methyl]-5-methyl-3-isoxazolecarboxamide,
N-[[2-[(2R,6S)-2,6-dimethyl-4-morpholinyl]-5-(2,6-dimethyl-4-pyridinyl)phenyl]methyl]-cyclopropaneacetamide,
N-[[2-[(2R,6S)-2,6-dimethyl-4-morpholinyl]-5-(2,6-dimethyl-4-pyridinyl)phenyl]methyl]-5-methyl-3-isoxazolecarboxamide,
N-[[2-[(2R,6S)-2,6-dimethyl-4-morpholinyl]-5-(2-6-dimethyl-4-pyridinyl)phenyl]methyl]-2-methyl-propainamide,
N-[[2-[(2R,6S)-2,6-dimethyl-4-morpholinyl]-5-(2,6-dimethyl-4-pytidinyl)phenyl]methyl]-4-fluoro-2-methyl-benzamide,
N-[[2-[(2R,6S)-2,6-dimethyl-4-morpholinyl]-5-(2,6-dimethyl-4-pyridinyl)phenyl]methyl]-3-methyl-4-isoxazolecarboxamide,
N-[[2-(2,6-dimethyl-4-pyridinyl)-5-(8-oxa-3-azabicyclo[3.2.1]oct-3-yl)phenyl]methyl]-2-methyl-propanamide,
N-[[2-(2,6-dimethyl-4-pyridinyl)-5-(8-oxa-3-azabicyclo[3.2.1]oct-3-yl)pheriyl]methyl]-cyclopropaneacetamide,
N-[[2-(2,6-dimethyl-4-pyridinyl)-5-(8-oxa-3-azabicyclo[3.2.1]oct-3-yl)phenyl]methyl]-cyclopropanecarboxamide,
2-methyl-N-[[2-(2-methyl-4-pyridinyl)-5-(4-morpholinyl)phenyl]methyl]-propanamide,
N-[[2-(2-methyl-4-pyridinyl)-5-(4-morpholinyl)phenyl]methyl]-cyclopropaneacetamide,
5-methyl-N-[[2-(2-methyl-4-pyridinyl)-5-(4-moipholinyl)phenyl]methyl]-3-isoxazolecarboxamide,
3-methyl-N-[[2-(2-methyl-4-pyridinyl)-5-(4-morpholinyl)phenyl]methyl]-4-isoxazolecarboxamide.

A particular compound is N-[[5-[(2R,6S)-2,6-dimethyl-4-morpholinyl]-2-(2,6-dimethyl-4-pyridinyl)phenyl]methyl]-2-methyl-propanamide.

All possible combinations of the above-indicated interesting embodiments are considered to be embraced within the scope of this invention.

When describing the compounds of the invention, the terms used are to be construed in accordance with the following definitions, unless a context dictates otherwise.

The term "halo" or "halogen" as a group or part of a group is generic for fluoro, chloro, bromo, iodo unless otherwise is indicated or is clear from the context.

The term "C₁₋₈alkyl" as a group or part of a group refers to a hydrocarbyl radical of Formula CₙH₂ₙ₊₁ wherein n is a number ranging from 1 to 8. C₁₋₈alkyl groups comprise from 1 to 8 carbon atoms, preferably from 1 to 6 carbon atoms, more preferably from 1 to 4 carbon atoms; still more preferably 1 to 2 carbon atoms. Alkyl groups may be linear or branched and may be substituted as indicated herein.

When a subscript is used herein following a carbon atom, the subscript refers to the number of carbon atoms that the named group may contain.

Thus, for example, C₁₋₈alkyl includes all linear, or branched alkyl groups with between 1 and 8 carbon atoms, and thus includes such as for example methyl, ethyl, n-propyl, isopropyl, butyl and its isomers (e.g. n-butyl, isobutyl and tert-butyl), pentyl, hexyl, heptyl, octyl and their isomers.

The term "C₁₋₄alkyl" as a group or part of a group refers to a hydrocarbyl radical of Formula CₙH₂ₙ₊₁ whecein n is a number ranging from 1 to 4. C₁₋₄alkyl groups comprises from 1 to 4 carbon atoms, preferably from 1 to 3 carbon atoms, more preferably 1 to 2 carbon atoms. C₁₋₄alkyl includes all linear, or branched alkyl groups with between 1 and 4 carbon atoms, and thus includes such as for example methyl, ethyl, n-propyl, isopropyl, butyl and its isomers e.g. n-butyl, isobutyl, sec-butyl and tert-butyl.

The term "C₁₋₆alkyloxy" as a group or part of a group refers to a radical having the Formula -OR^{a} wherein R^{a} is C₁₋₆alkyl. Non-limiting examples of suitable alkyloxy include methyloxy, ethyloxy, propyloxy, isopropyloxy, butyloxy, isobutyloxy, sec-butyloxy, tert-butyloxy, pentyloxy, and hexyloxy.

The term "C₁₋₄alkyloxy" as a group or part of a group refers to a radical having the Formula -OR^{b} wherein R^{b} is C₁₋₄alkyl. Non-limiting examples of suitable C₁₋₄alkyloxy include methyloxy (also methoxy), ethyloxy (also ethoxy), propyloxy, isopropyloxy, butyloxy, isobutyloxy, sec-butyloxy and tert-butyloxy.

The term "haloC₁₋₄alkyloxy" as a group or part of a group refers to a C₁₋₄alkyloxy radical wherein said C₁₋₄alkyloxy radical is further substituted with 1, 2 or 3 halo atoms. Non-limiting examples of suitable haloC₁₋₄alkyloxy radicals include trifluoromethyloxy, trifluoroethyloxy, trifluoropropyloxy, and trifluorobutyloxy.

The term "C₃₋₆cycloalkyl" alone or in combination, refers to a cyclic saturated hydrocarbon radical having from 3 to 6 carbon atoms. Non-limiting examples of suitable cycloC₃-₆alkyl includes cyclopropyl, cyclobutyl, cyclopentyl and cyclohexyl.

Hereinbefore and hereinafter, the term "compound of formula (I)" is meant to include the addition salts, the solvates and the stereoisomers thereof.

The terms "stereoisomers" or "stereochemically isomeric forms" hereinbefore or hereinafter are used interchangeably.

The invention includes all stereoisomers of the compound of Formula (I) either as a pure stereoisomer or as a mixture of two or more stereoisomers.

Enantiomers are stereoisomers that are non-superimposable mirror images of each other. A 1:1 mixture of a pair of enantiomers is a racemate or racemic mixture. Diastereomers (or diastereoisomers) are stereoisomers that are not enantiomers, i.e. they are not related as mirror images. If a compound contains a double bond, the substituents may be in the E or the Z configuration. If a compound contains a disubstituted cycloalkyl group, the substituents may be in the cis or trans configuration. Therefore, the invention includes enantiomers, diastereomers, racemates, E isomers, Z isomers, cis isomers, trans isomers and mixtures thereof.
The absolute configuration is specified according to the Cahn-Ingold-Prelog system. The configuration at an asymmetric atom is specified by either R or S. Resolved compounds whose absolute configuration is not known can be designated by (+) or (-) depending on the direction in which they rotate plane polarized light.
When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) is for instance specified as (R), this means that the compound is substantially free of the (S) isomer; when a compound of formula (I) is for instance specified as E, this means that the compound is substantially free of the Z isomer; when a compound of formula (I) is for instance specified as cis, this means that the compound is substantially free of the trans isomer.

For therapeutic use, salts of the compounds according to formula (I) are those wherein the counterion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable acid and base addition salts as mentioned hereinabove or hereinafter are meant to comprise the therapeutically active non-toxic acid and base addition salt forms which the compounds according to formula (I) are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethahesulfonic, benzenesulfonic, *p*-toluendsulfonic, cyclamic, salicylic, *p*-aminosalicylic, pamoic and the like acids. Conversely said salt forms can be converted by treatment with an appropriate base into the free base form.

The term solvates refers to hydrates and alcoholates which the compounds according to formula (I) as well as the salts thereof, may form.

The chemical names of the compounds of the present invention were generated according to the nomenclature rules agreed upon by the Chemical Abstracts Service, using Advanced Chemical Development, Inc., nomenclature software (ACD/Name product version 10.01; Build 15494, 1 Dec 2006).

Some of the compounds according to formula (I) may also exist in their tautomeric form. Such forms although not explicitly indicated in the above formula are intended to be included within the scope of the present invention.

### Preparation of the compounds

Depending on the position of acylaminomethylene group on the phenyl moiety and whether Y represents N or CH, three subgroups of Formula (Ia), (Ib) and (Ic) may be distinguished, each having its own synthetic methodology.

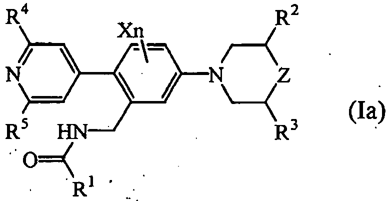

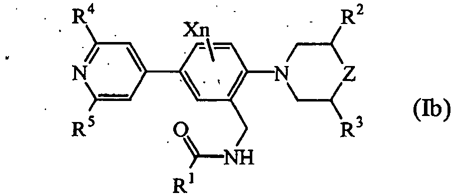

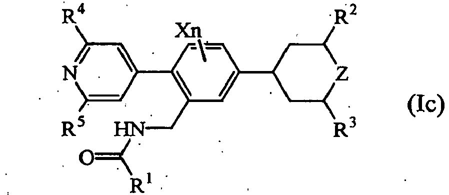

Compounds of Formula (Ia) can be prepared by reacting a compound of Formula (II), 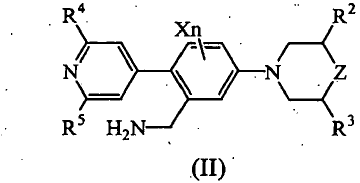 where R², R³, R⁴, R⁵, Z, and Xₙ are as defined in Formula (I), with a compound of Formula R¹-CO₂H (III) where R¹ is as defined in Formula (I), in the presence of a suitable amide coupling reagent, such as HBTU, a suitable base, such as DIPEA, in a suitable solvent, such as DCM and at a suitable temperature, such as room temperature. Alternatively, the acylation reaction of (II) may be conducted with a symmetric or asymmetric anhydride, or an acyl halide of carboxylic acid (III).

Compounds of Formula (II), can be prepared by reacting a compound of Formula (IV), 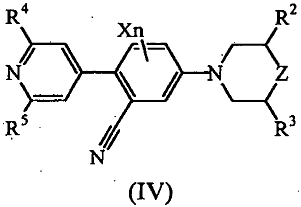 where R², R³, R⁴, R⁵, Z, and Xₙ are as defined in Formula (I), with a suitable reducing agent, such as hydrogen, in the presence of a suitable catalyst, such as Raney Nickel, in a suitable solvent, such as 7M ammonia in methanol, at a suitable temperature, such as room temperature.

Compounds of Formula (IV) can be prepared by reacting a compound of Formula (V) 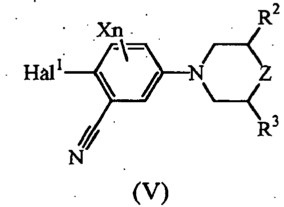 where, R², R³, Z, and Xₙ are as defined in Formula (I), and Hal¹ is a halogen atom such as iodine, bromine or chlorine with a compound of Formula (VI) 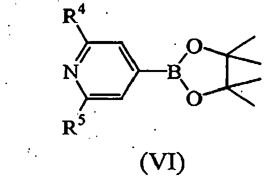 where R⁴ and R⁵ are as defined in Formula (I), in the presence of a suitable catalyst, such as Pd(PPh₃)₄, with a suitable base, such as sodium carbonate, in a suitable solvent, such as 1,4-dioxane and ethanol/water (1:1) and at a suitable temperature, such as 120°C in a sealed tube, under a suitable inert atmosphere, such as a nitrogen atmosphere.

Compounds of Formula (V) can be prepared by reacting a compound of Formula (VII) 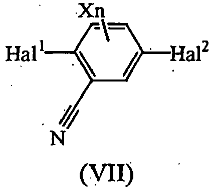 where Hal¹ and Xₙ are as defined as in Formula (V) and Hal² is a halogen atom such as fluorine, with a compound of Formula (VIII) 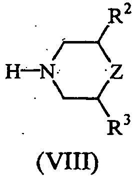 where R², R³, and Z are as defined in Formula (I), in the presence of a suitable base, such as potassium carbonate, in a suitable solvent, such as DMSO, at a suitable temperature, such as 100 °C.

Alternatively, compounds of Formula (V) can be prepared by reacting a compound of Formula (IX) 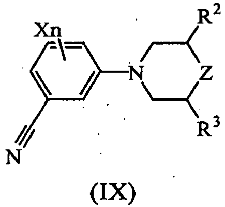 where R², R³, Z, and Xₙ are as defined in Formula (V) with a suitable base such as lithium tetramethylpiperidide and a halogen (Hal¹)₂, such as iodine, in a suitable solvent, such as THF, at a suitable temperature, such as -78°C and under a suitable inert atmosphere, such as a nitrogen atmosphere.

Compounds of Formula (IX) can be prepared by reacting a compound of Formula (X) 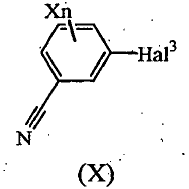 where Xₙ is as defined in Formula (IX), and Hal³ is a halogen atom, such as bromine, with a compound of Formula (VIII), in the presence of a suitable catalyst, such as Pd₂(dba)₃ or the Nolan catalyst [478980-01-7], a suitable ligand, such as Xantphos, and a suitable base, such as sodium tert-butanolate, in a suitable solvent, such as toluene or monoglyme, at a suitable temperature, such as 120 °C, in a sealed tube, and under a suitable inert atmosphere, such as a nitrogen atmosphere.

Alternatively, compounds of Formula (IV) can be prepared by reacting a compound of Formula (XI). 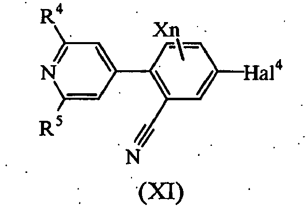 where, R⁴, R⁵, and Xₙ are as defined in Formula (I), and Hal⁴ is a halogen atom such as chlorine or bromine, with a compound of Formula (VIII), in the presence of a suitable catalyst such as Pd₂(dba)₃ or the Nolan catalyst [478980-01-7], a suitable ligands, such as Xantphos, and a suitable base, such as sodium tert-butanolate, in a suitable solvent, such as toluene or monoglyme, at a suitable temperature, such as 120 °C, in a sealed tube, and under a suitable inert atmosphere, such as a nitrogen atmosphere.

Compounds of Formula (XI) can be prepared by reacting a compound of Formula (XII) 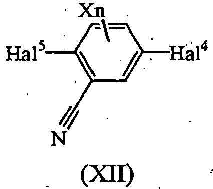 where Hal⁴ and Xₙ are as defined in Formula (XI) and Hal⁵ is a halogen atom such as bromine or iodine, with a compound of Formula (VI), in the presence of a suitable catalyst, such as Pd(PPh₃)₄, with a suitable base, such as sodium carbonate or potassium carbonate, in a suitable solvent, such as 1,4-dioxane or dimethoxyethane and water, and at a suitable temperature, such as 100°C, in a sealed tube, and under a suitable inert atmosphere, such as a nitrogen atmosphere.

Alternatively, compounds of Formula (IV) can be prepared by reacting a compound of Formula (XIII) 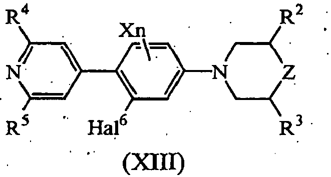 where R², R³, R⁴, R⁵, Z, and Xₙ are as defined in Formula (I), and Hal⁶ is a halogen atom such as, bromine, with a suitable cyanide salt, such as zinc cyanide, in the presence of a suitable catalyst, such as Pd(PPh₃)₄, and in the presence of a suitable ligand, such as triphenylphosphine, in a suitable solvent, such as acetonitrile, and at a suitable temperature, such as 150°C, in a sealed tube, and under a suitable inert atmosphere, such as a nitrogen atmosphere.

Compounds of Formula (XIII) can be prepared by reacting a compound of Formula (XIV) 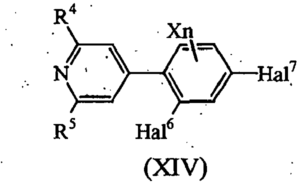 where R⁴, R⁵, Xₙ and Hal⁶ are as defined in Formula (XIII), and Hal⁷ is a halogen atom such as bromine, with a compound of Formula (VIII), in the presence of a suitable catalyst, such as Pd₂(dba)₃, a suitable ligand, such as Xantphos, and a suitable base, such as sodium tert-butanolate, in a suitable solvent, such as toluene, at a suitable temperature, such as 120 °C, in a sealed tube, arid under a suitable inert atmosphere, such as a nitrogen atmosphere.

Compounds of Formula (XIV) can be prepared by reacting a compound of Formula (XV) 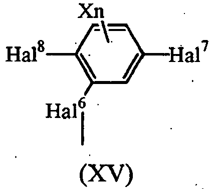 where Xₙ, Hal⁶ and Hal⁷ are as defined in Formula (XIV), and Hal⁸ is a halogen atom such as iodine, with a compound of Formula (VI), in the presence of a suitable catalyst, such as Pd(PPh₃)₄, in the presence of a suitable base, such as potassium carbonate, in a suitable solvent, such as dimethoxyethane, and at a suitable temperature, such as 100 °C, in a sealed tube, and under a suitable inert atmosphere, such as a nitrogen atmosphere.

Compounds of Formula (Ib) can be prepared by reacting a compound of Formula (XVI), 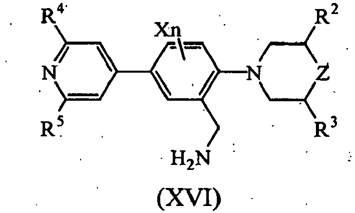 where R², R³, R⁴, R⁵, Z; and Xₙ are as defined in Formula (Ib), with a compound of Formula (III), in the presence of a suitable amide coupling reagent, such as HBTU, a suitable base, such as DIPEA, in a suitable solvent, such as DCM and at a suitable temperature, such as room temperature. Alternatively, the acylation reaction of (XVI) may be conducted with a symmetric or asymmetric anhydride, or an acyl halide of carboxylic acid (III).

Compounds of Formula (XVI), can be prepared by reacting a compound of Formula (XVII), 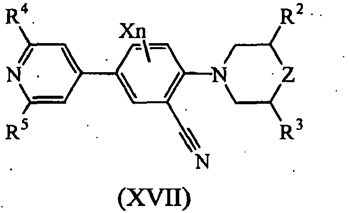 where R², R³, R⁴, R⁵, Z, and Xₙ are as defined in Formula (Ib), with a suitable reducing agent, such as hydrogen, in the presence of a suitable catalyst, such as Raney Nickel, in a suitable solvent, such as 7M ammonia in methanol, at a suitable temperature, such as room temperature.

Compounds of Formula (XVII), can be prepared by reacting a compound of Formula (XVIII), 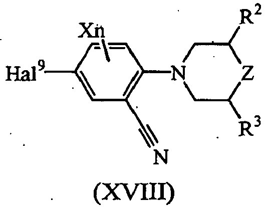 where R², R³, Z, and Xₙ are as defined in Formula (Ib), and Hal⁹ is a halogen atom such as chlorine, with a compound of Formula (VI), in the presence of a suitable catalyst, such as Pd(PPh₃)₄ or PdCl₂(dppf), with a suitable base, such as sodium carbonate, in a suitable solvent, such as a mixture of dioxane, ethanol and water, or acetonitrile, at a suitable temperature, such as 130°C, in a sealed tube, and under a suitable inert atmosphere, such as a nitrogen atmosphere.

Compounds of Formula (XVIII), can be prepared by reacting a compound of Formula (XIX), 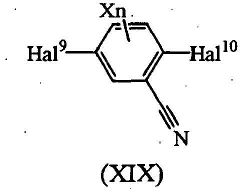 where Hal⁹ and Xₙ are as defined in Formula (XVIII) and Hal¹⁰ is a halogen atom such as, bromine, with a compound of Formula (VIII), in the presence of a suitable catalyst, such as Pd₂(dba)₃, in the presence of a suitable ligand, such as Xantphos, with a suitable base, such as cesium carbonate, in a suitable solvent, such as dioxane, at a suitable temperature, such as 145 °C, in a sealed tube, and under a suitable inert atmosphere, such as a nitrogen atmosphere.

Compounds of Formula (Ic) can be prepared by reacting a compound of Formula (XX), 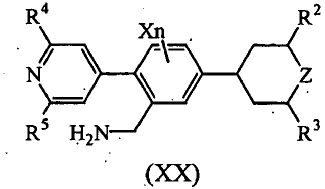 where R², R³, R⁴, R⁵, Z, and Xₙ are as defined in Formula (I), with a compound of Formula (III)

R¹-CO₂H (III)

where R¹ is as defined in Formula (I), in the presence of a suitable amide coupling reagent, such as HBTU, a suitable base, such as DIPEA, in a suitable solvent, such as DCM and at a suitable temperature, such as room temperature. Alternatively, the acylation reaction of (XX) may be conducted with a symmetric or asymmetric anhydride, or an acyl halide of carboxylic acid (III).

Compounds of Formula (XX) can be prepared by reacting a compound of Formula (XXI), 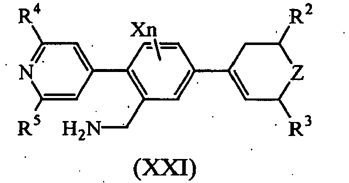 where R², R³, R⁴, R⁵, Z, and Xₙ are as defined in Formula (I), with a suitable reducing agent, such as hydrogen, in the presence of a suitable catalyst, such as platinum on charcoal, in a suitable solvent, such as THF, at a suitable temperature, such as 50 °C.

Compounds of Formula (XXI) can be prepared by reacting a compound of Formula (XXII), 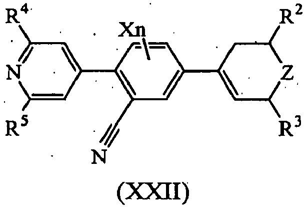 where R², R³, R⁴, R⁵, Z, and Xₙ are as defined in Formula (I), with a suitable reducing agent, such as hydrogen, in the presence of a suitable catalyst, such as Raney Nickel, in a suitable solvent, such as 7M ammonia in methanol, at a suitable temperature, such as room temperature.

Compounds of Formula (XXII) can be prepared by reacting a compound of Formula (XXIII), 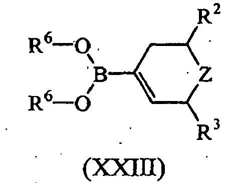 where R², R³, and Z, are as defined in Formula (I), and R⁶ is C₁₋₆ alkyl or both R⁶ together form C₂₋₈alkanediyl, with a compound of Formula (XI), in the presence of a suitable catalyst such as Pd(PPh₃)₄, with a suitable base, such as potassium carbonate, in a suitable solvent, such as dimethoxyethane, at a suitable temperature, such as 100°C, under a suitable inert atmosphere, such as nitrogen atmosphere.

Compounds of Formula (XXIII) can be prepared by reacting a compound of Formula (XXIV), 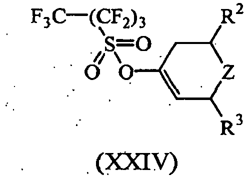 where R², R³, and Z, are as defined in Formula (I), with a suitable boron derivative, such as bis(pinacolato)diboron, in the presence of a suitable catalyst, such as PdCl₂(dppf), in the presence of a suitable ligand, such as dppf, with a suitable base, such as potassium acetate, in a suitable solvent, such as dimethoxyethane, at a suitable temperature, such as 80 °C, under a suitable inert atmosphere, such as nitrogen atmosphere.

Compounds of Formula (XXIV) can be prepared by reacting a compound of Formula (XXV),

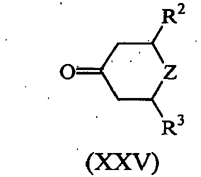

Where R², R³, and Z, are as defined in Formula (I), with a suitable base, such as LDA, and with a suitable sulfonylating agent, such as perfluorobutanesulfonyl fluoride, in a suitable solvent, such as THF, at a suitable temperature, such as -78 °C, under a suitable inert atmosphere, such as a nitrogen atmosphere.

### Pharmacology

The compounds of the present invention were found to be positive allosteric modulators of the alpha 7 nicotinic receptor. The alpha 7 nicotinic receptor (alpha 7 nAChR) belongs to the superfamily of cys-loop, ionotropic ligand-gated ion channels which includes the 5-HT₃, GABA_{A} and glycine receptor families. It is activated by acetylcholine and its breakdown product choline and a major feature of the alpha 7 nAChR is its rapid desensitisation in the persistent presence of agonist. It is the second most abundant nicotinic receptor subtype in the brain and is an important regulator of release of many neurotransmitters. It has a discrete distribution in several brain structures with relevance to attentional and cognitive processes, such as the hippocampus and pre-frontal cortex and has been implicated in a variety of psychiatric and neurological disorders in humans. It is also implicated in the cholinergic inflammatory pathway.

Genetic evidence for its association with schizophrenia is seen in the form of strong linkage between a schizophrenia marker (sensory gating deficit) and the alpha 7 locus on 15q13-14 and polymorphisms in the core promoter region of the alpha 7 gene.

Pathological evidence points to a loss of alpha 7 immunoreactivity and α-bungarotoxin (Btx)-binding in the hippocampus, frontal and cingulate cortex of schizophrenic brains, in Parkinson's and Alzheimer's disease, and in the paraventricular nucleus and nucleus reuniens in autism.

Pharmacological evidence such as the marked smoking habits of schizophrenics compared to normals has been interpreted as an attempt by the patients to self-medicate to make up for a deficit in alpha 7 nicotinergic transmission. Transient normalization of defects in sensory gating (pre-pulse inhibition, PPI) in both animal models and man upon nicotine administration and temporary restoration of normal sensory gating in schizophrenics when forebrain cholinergic activity is low (e.g. stage 2 sleep) have both been interpreted to be the result of transient activation of the alpha 7 nicotinic receptor followed by desensitization.

Thus there is good reason to suppose that activating the alpha 7 nAChR will have therapeutically beneficial effects for a number of CNS (psychiatric and neurological) disorders.

As already mentioned the alpha 7 nAChR rapidly desensitizes in the persistent presence of the natural transmitter acetylcholine as well as exogenous ligands such as nicotine. In the desensitized state the receptor remains ligand-bound but functionally inactive. This is not so much a problem for natural transmitters such as acetylcholine and choline since these are substrates for very powerful breakdown (acetylcholinesterase) and clearance (choline transporter) mechanisms. These transmitter breakdown/clearance mechanisms are likely to maintain the balance between activatible and desensitized alpha 7 nAChRs in a physiologically useful range. However, synthetic agonists, which are not substrates for the natural breakdown and clearance mechanisms are perceived to have a potential liability both for over-stimulation and also to push the alpha 7 nAChR population equilibrium towards a persistently desensitized state, which is undesirable in disorders in which deficiencies in alpha 7 nAChR expression or function play a role. Agonists by their nature must target the ACh binding pocket which is highly conserved across the different nicotinic receptor subtypes leading to the potential for adverse reactions by non-specific activation of other nicotinic receptor subtypes. Therefore, to avoid these potential liabilities an alternative therapeutic strategy to alpha 7 agonism is to enhance receptor responsiveness to the natural agonists with a positive allosteric modulator (PAM). A PAM is defined as an agent which binds to a site distinct from the agonist binding site, and therefore is not expected to have agonist or desensitization properties, but enhances the responsiveness of the alpha 7 nAChR to the natural transmitter. The value of this strategy is that for a given amount of transmitter the magnitude of the alpha 7 nAChR response is increased in the presence of the PAM relative to the level of transmission possible in its absence. Additionally, PAMs can also increase the potency of the natural transmitter. So for disorders in which there is a deficit in alpha 7 nAChR protein, the PAM-induced increase in alpha 7 nicotinergic transmission can be beneficial. As a PAM relies on the presence of the natural transmitter the potential for over-stimulation is limited by the breakdown/clearance mechanisms for the natural transmitter.

The compounds of the present invention are classified as type 1-4, based on qualitative kinetic properties, as determined by whole-cell voltage-clamp recordings. This classification is based on the effect of an alpha 7 PAM compound, as described hereinbefore, on the signal elicited by an agonist application. In particular, said agonist is choline at a concentration of 1mM. In a preferred experimental setting, said alpha 7 PAM compound and choline are simultaneously applied to the cell, as described hereinafter. Desensitization is defined as the closure of the receptor upon activation during the application of the agonist in whole-cell voltage-clamp electrophysiology measurements seen as the reduction of the outward current after initial activation by the agonist.

The definition of the PAM types 1-4 is described hereinafter:
- Type 0: compounds minimally change the effect size of the current elicited by 1 mM choline.
- Type 1: compounds enhance the effect size of the current elicited by 1 mM choline but minimally alter the kinetics of the receptor. In particular, the rate and the extent of desensitization and of deactivation of the receptor elicited by the agonist is not affected. The compound-modulated response to 1 mM choline, therefore, is close to a linear scaling of the 1 mM choline response in absence of the alpha 7 PAM compound:
- Type 2: compounds enhance the effect size of the current elicited by 1 mM choline while reducing the rate and/or the extent of desensitization. Deactivation of the receptor is generally unaffected.
- Type 3: compounds enhance the effect size of the current elicited by 1 mM choline. When tested at higher concentrations up to 10 µM they completely inhibit desensitization, in particular a 1 mM choline application of 250 milliseconds. Deactivation of the receptor may be slowed down.
- Type 4: compounds allow for an initial desensitization of the receptor followed by a re-opening of the receptor during agonist application. At low-potency concentrations of the alpha 7 PAM compound, the agonist-induced activation, which is followed by desensitization, can still be separated from the compound-induced re-opening as an initial inward current-maximum. At higher potency concentrations of the alpha 7 PAM compound, the re-opening occurs faster than the closure due to desensitization so that the initial current-maximum disappears.

A compound was considered to have interesting PAM-like activity when the potentiation of the peak current was at least 200% compared to the control choline response (= 100%). Such compounds are classified as belonging to a particular PAM type in the Experimental Part. Compounds not meeting the condition are not classified as belonging to a particular PAM-type.

A number of compounds according to the invention may prove active in the auditory evoked potential test. The DBA/2 inbred mouse strain used in this test shows sensory processing deficits similar to schizophrenia patients which are also correlated with reduced nicotinic alpha 7 receptors in the hippocampus. The DBA/2 mouse has proven to be a useful model of schizophrenia-like sensory processing deficits. Human studies of nicotine effects on sensory processing predicted the results in the DBA/2 mouse and studies with the selective alpha 7 agonist GTS-21 in DBA/2 mice, predicted the effects in humans. This model of sensory gating ability therefore has high translational relevance.

It is accordingly an object of the present invention to provide methods of treatment that include administering either a positive allosteric modulator as the only active substance, thus modulating the activity of endogenous nicotinic receptor agonists such as acetylcholine or choline, or administering a positive allosteric modulator together with a nicotinic receptor agonist. In a particular form of this aspect of the invention, the method of treatment comprises treatment with a positive allosteric modulator of the alpha 7 nicotinic receptor as described herein and an alpha 7 nicotinic receptor agonist or partial agonist Examples of suitable compounds with alpha 7 nicotinic receptor agonistic activity include
- 1,4-Diazabicyclo[3.2.2]nonane-4-carboxylic acid, 4-bromophenyl ester, monohydrochloride (SSR180711A) ;
- (-)-spiro[-1-azabicyclo[2.2.2.]octane-3,5'-oxazolidine]-2'-one;
- 3-[(2,4-Dimethoxy)Benzylidene]-Anabaseine Dihydrochloride (GTS-21);
- [*N*-[(3R)-1-Azabicyclo[2.2.2]oct-3-yl]-4-chlorobenzamide Hydrochloride] PNU-282987;
- nicotine;
- varenicline;
- MEM3454;
- AZD-0328;
- MEM63908;
- (+)-*N*-(1-azabicyclo[2.2.2]oct-3-yl)benzo[b]furan-2-carboxamide;
- A-582941;
- AR-R17779;
- TC-1698;
- PHA-709829;
- tropisetron;
- WAY-317538;
- EVP-6124; and
- TC-5619.

In particular, examples of suitable compounds with α7 nicotinic receptor agonistic activity include 1,4-Diazabicyclo[3.2.2]nonane-4-carboxylic acid, 4-bromophenyl ester, monohydrochloride (SSR180711A) ;
(-)-spiro[1-azabicyclo[2.2.2.]octane-3,5'-oxazolidine]-2'-one;
3-[(2,4-Dimethoxy)Benzylidene]-Anabaseine Dihydrochloride (GTS-21);
[*N*-[(3R)-1-Azabicyclo[2.2.2]oct-3-yl]-4-chlorobenzamide Hydrochloride] PNU-282987; nicotine; varenicline; MEM3454; AZD-0328; and MEM63908.

Positive nAChR modulators of the present invention are useful for treatment or prophylaxis of psychotic disorders, intellectual impairment disorders or diseases or conditions in which modulation of alpha 7 nicotinic receptor activity is beneficial. A particular aspect of the method of the invention is a method of treatment for learning deficit, cognition deficit, attention deficit or memory loss, modulation of alpha 7 nicotinic receptor activity is expected to be beneficial in a number of diseases including Alzheimer's disease, Lewy Body Dementia, Attention Deficit Hyperactivity Disorder, anxiety, schizophrenia, mania, manic depression, Parkinson's disease, Huntington's disease, Tourette's syndrome, brain trauma or other neurological, degenerative or psychiatric disorders in which there is loss of cholinergic synapses, including jetlag, nicotine addiction, pain.

The compounds may also find therapeutical use as anti-inflammatory medicines because the nicotinic acetylcholine receptor alpha 7 subunit is essential for inhibiting cytokine synthesis by the cholinergic inflammatory pathway. Examples of indications which may be treated by the compounds are endotoxaemia, endotoxic shock, sepsis, rheumatoid arthritis, asthma, multiple sclerosis, psoriasis, urticaria, inflammatory bowel disease, inflammatory bile disease, Crohn's disease, ulcerative colitis, postoperative ileus, pancreatitis, heart failure, acute lung injury and allograft rejection.

The compounds of the invention may find therapeutical use in the following indications as cognition in schizophrenia, cognition in Alzheimer's disease, mild cognitive impairment, Parkinson's disease, attention deficit hyperactivity disorder, ulcerative colitis, pancreatitis, arthritis, sepsis, postoperative ileus and acute lung injury.

In view of the above described pharmacological properties, the compounds according to formula (I) or any subgroup thereof, their pharmaceutically acceptable addition salts, solvates and stereochemically isomeric forms, may be used as a medicine. In particular, the present compounds can be used for the manufacture of a medicament for treatment or prophylaxis of psychotic disorders, intellectual impairment disorders or diseases or conditions in which modulation of the alpha 7 nicotinic receptor is beneficial.

In an embodiment the present invention relates to the compounds according to formula (I) for use in the treatment or prophylaxis, in particular treatment of psychotic disorders, intellectual impairment disorders or diseases or conditions in which modulation of the α7 nicotinic receptor is beneficial.

In an embodiment the present invention relates to the compounds according to formula (I) for use in the treatment or prophylaxis, in particular treatment, of psychotic disorders, intellectual impairment disorders, or inflammatory diseases.

In an embodiment the present invention relates to the compounds according to formula (I) for treating or preventing, in particular treating, said diseases or conditions.

In view of the utility of the compounds according to formula (I), there is provided a method of treating or preventing warm-blooded animals, including humans, suffering from diseases in which modulation of the alpha 7 nicotinic receptor is beneficial, such as schizophrenia, mania, and manic depression, anxiety, Alzheimer's disease, learning deficit, cognition deficit, attention deficit, memory loss, Lewy Body Dementia, Attention Deficit Hyperactivity Disorder, Parkinson's disease, Huntington's disease, Tourette's syndrome, brain trauma, jetlag, nicotine addiction and pain. Said methods comprise the administration, i.e. the systemic or topical administration, preferably oral administration, of an effective amount of a compound according to formula (I), a stereochemically isomeric form thereof, a pharmaceutically acceptable addition salt, or a solvate thereof, to warm-blooded animals, including humans.

One skilled in the art will recognize that a therapeutically effective amount of the PAM's of the present invention is the amount sufficient to modulate the activity of the alpha 7 nicotinic receptor and that this amount varies *inter alia,* depending on the type of disease, the concentration of the compound in the therapeutic formulation, and the condition of the patient. Generally, an amount of PAM to be administered as a therapeutic agent for treating diseases in which modulation of the alpha 7 nicotinic receptor is beneficial, such as schizophrenia, mania, and manic depression, anxiety, Alzheimer's disease, learning deficit, cognition deficit, attention deficit, memory loss, Lewy Body Dementia, Attention Deficit Hyperactivity Disorder, Parkinson's disease, Huntington's disease, Tourette's syndrome, brain trauma, jetlag, nicotine addiction and pain will be determined on a case by case by an attending physician.

Generally, a suitable dose is one that results in a concentration of the PAM at the treatment site in the range of 0.5 nM to 200 µM, and more usually 5 nM to 50 µM. To obtain these treatment concentrations, a patient in need of treatment likely will be administered between 0.01 mg/kg to 2.50 mg/kg body weight, in particular from 0.1 mg/kg to 0.50 mg/kg body weight. The amount of a compound according to the present invention, also referred to here as the active ingredient, which is required to achieve a therapeutically effect will be, of course vary on case-by-case basis, vary with the particular compound, the route of administration, the age and condition of the recipient, and the particular disorder or disease being treated. A method of treatment may also include administering the active ingredient on a regimen of between one and four intakes per day. In these methods of treatment the compounds according to the invention are preferably formulated prior to admission. As described herein below, suitable pharmaceutical formulations are prepared by known procedures using well known and readily available ingredients.

### Pharmaceutical compositions

The present invention also provides compositions for preventing or treating diseases in which modulation of the alpha 7 nicotinic receptor is beneficial, such as schizophrenia, mania, and manic depression, anxiety, Alzheimer's disease, learning deficit, cognition deficit, attention deficit, memory loss, Lewy Body Dementia, Attention Deficit Hyperactivity Disorder, Parkinson's disease, Huntington's disease, Tourette's syndrome, brain trauma, jetlag, nicotine addiction and pain. Said compositions comprising a therapeutically effective amount of a compound according to formula (I) and a pharmaceutically acceptable carrier or diluent.

While it is possible for the active ingredient to be administered alone, it is preferable to present it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of this invention may be prepared by any methods well known in the art of pharmacy, for example, using methods such as those described in Gennaro et al. Remington's Pharmaceutical Sciences (18th ed., Mack Publishing Company, 1990, see especially Part 8 : Pharmaceutical preparations and their Manufacture). A therapeutically effective amount of the particular compound, in base form or addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The exact dosage and frequency of administration depends on the particular compound of formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

Depending on the mode of administration, the pharmaceutical composition will comprise from 0.05 to 99 % by weight, preferably from 0.1 to 70 % by weight, more preferably from 0.1 to 50 % by weight of the active ingredient, and, from 1 to 99.95 % by weight, preferably from 30 to 99.9 % by weight, more preferably from 50 to 99.9 % by weight of a pharmaceutically acceptable carrier, all percentages being based on the total weight of the composition.

The amount of a compound of Formula (I) that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg of the active compound. A preferred unit dose is between 1 mg to about 500 mg. A more preferred unit dose is between 1 mg to about 300mg. Even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treading physician will know how and when to start, interrupt, adjust, or terminate therapy in conjunction with individual patient response.

The present compounds can be used for systemic administration such as oral, percutaneous or parenteral administration; or topical administration such as via inhalation, a nose spray, eye drops or via a cream, gel, shampoo or the like. The compounds are preferably orally administered. The exact dosage and frequency of administration depends on the particular compound according to formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The compounds according to formula (I) may also be used in combination with other conventional α7 nicotinic receptor agonists, such as for example 1,4-Diazabicyclo-[3.2.2]nonane-4-carboxylic acid, 4-bromophenyl ester, monohydrochloride (SSR180711A); (-)-spiro[1-azabicyclo[2.2.2.]octane-3,5'-oxazolidine]-2'-one; 3-[(2,4-Dimethoxy)Benzylidene]-Anabaseine Dihydrochloride (GTS-21); [*N*-[(3R)-1-Azabicyclo[2.2.2]oct-3-yl]-4-chlorobenzamide Hydrochloride] PNU-282987; nicotine; varenicline, MEM3454; AZD-0328; MEM63908; (+)-N-(1-azabicyclo[2.2.2]oct-3-yl)benzo[b]furan-2-carboxamide; A-582941; AR-R17779; TC-1698; PHA-709829; tropisetron; WAY-317538; EVP-6124; and TC-5619.

Thus, the present invention also relates to the combination of a compound according to formula (I) and a alpha 7 nicotinic receptor agonist. Said combination may be used as a medicine. The present invention also relates to a product comprising (a) a compound according to formula (I), and (b) an alpha 7 nicotinic receptor agonist, as a combined preparation for simultaneous, separate or sequential use in the treatment of diseases wherein modulation of the alpha 7 nicotinic receptor is beneficial. The different drugs may be combined in a single preparation together with pharmaceutically acceptable carriers.

### EXPERIMENTAL PART

Several methods for preparing the compounds of this invention are illustrated in the following Examples. Unless otherwise noted, all starting materials were obtained from commercial suppliers and used without further purification.

Hereinafter or hereinbefore, "min" means minutes; "MeOH" means methanol; "EtOH" means ethanol; "Et₂O" means diethyl ether; "TFA" means trifluoroacetic acid "NH₄OAc" means ammonium acetate, "HBTU," means O-(benzotriazol-1-yl)-N,N,N',N'-tetramethyluronium hexafluorophosphate ; "DIPEA" means diisopropylethylamine; "LDA" means lithium diisopropylamine; "DCM" means dichloromethane; "VCD" means vibrational circular dichroism.

Microwave assisted reactions were performed in a single-mode reactor: Initiator^{™} Sixty EXP microwave reactor (Biotage AB), or in a multimode reactor: MicroSYNTH Labstation (Milestone, Inc.).

The following examples are intended to illustrate the present invention.

### A. Preparation of the Intermediates

### 5-chloro-2-(2,6-dimethyl-4-pyridinyl)-benzonitrile (Intermediate 1)

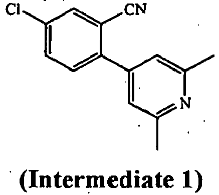

A mixture of 2-bromo-5-chlorobenzonitrile (10.03 g,.46.33 mmol), 2,6-dimethylpyridine-4-boronic acid, pinacol ester ([325142-95-8], 16.20 g, 69.50 mmol), tetrakis(triphenylphosphine)palladium (3.21 g, 2.78 mmol), 1,4-dioxane (50 mL), and sodium carbonate (14.73 g, 139.00 mmol) in water (50 mL) was stirred and heated under nitrogen atmosphere at 100 °C for 2 h. The reaction was diluted with CH₂Cl₂ and water and the layers were separated. The organic layer was dried with MgSO₄, filtered and evaporated. The residue was purified by column chromatography on silica gel (eluent gradient from 100% DCM to 98/2 DCM/MeOH). The desired fractions were collected and evaporated, yielding 8.73 g (77 %) of Intermediate 1, after drying overnight under vacuum at 50 °C.

### 5-[(2R,6S)-2,6-dimethyl-4-morpholinyl]-2-(2,6-dimethyl-4-pyridinyl)-benzonitrile (Intermediate 2)

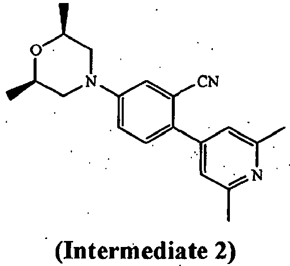

A mixture of Intermediate 1 (2.64 g, 10.88 mmol), cis-2,6-dimethylmorpholine (1.35 mL, 10.88 mmol), sodium tert-butoxide (1.57 g, 16.33 mmol) and dry toluene (15 mL) was introduced in a pressure tube and was degassed with nitrogen for 5 min. Xantphos [161265-03-8] (378 mg, 0.65 mmol) and Pd₂(dba)₃ (199 mg, 0.22 mmol) were added and the mixture was further degassed for 5 min. The tube was sealed and the reaction mixture was stirred at 120 °C for 1.5 h. The reaction mixture was diluted with CH₂Cl₂ and washed with water. The aqueous layer was extracted twice with CH₂Cl₂. The combined organic layers were washed with water, dried on MgSO₄, filtered and evaporated. The residue was purified by column chromatography on silica gel (eluent gradient from 100% DCM to 99/1 DCM/MeOH), yielding 2.69 g of Intermediate 2 (77 %) as a white solid after drying in a vacuum oven at 50°C overnight.

### 5-[(2R,6S)-2,6-dimethyl-4-morpholinyl]-2-(2,6-dimethyl-4-pyridinyl)-benzenemethanamine (Intermediate 3)

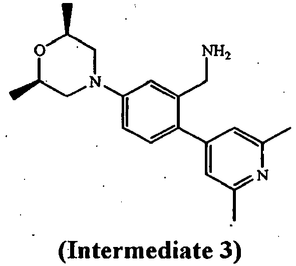

Intermediate 2 (3.26 g, 10.14 mmol) was added to a suspension of Raney Nickel (1 g) in a 7 N ammonia solution in MeOH (200 mL). The reaction mixture was stirred at 14 °C under hydrogen atmosphere until 2 equivalents of hydrogen were absorbed. The catalyst was removed by filtration over diatomaceous earth. The solvent was evaporated, yielding Intermediate 3 quantitatively. Intermediate 3 was used without further purification.

### 3,5-bis(trifluoromethyl)piperidine (Intermediate 4)

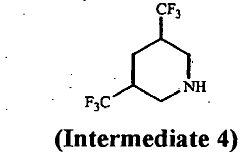

3,5-bis(trifluoromethyl)pyridine [20857-47-0] (10 g, 46.49 mmol) and 6 N HCl in isopropanol (7.75 mL, 46.49 mmol) were added to a suspension of Pt on charcoal 5% (1 g) in EtOH (150 mL). ). The reaction mixture was stirred at 25 °C under hydrogen atmosphere until 3 equivalents of hydrogen were absorbed. The catalyst was removed by filtration over diatomaceous earth. The solvent was evaporated, yielding Intermediate 4 quantitatively as a 50:50 mixture of cis and trans isomers. Intermediate 4 was used without further purification.

**Table 1**

| The following intermediates were prepared according to the procedures used for Intermediate 3 (starting from Intermediate 1, via Intermediate 2): | | |
|---|---|---|
| Intermediate | Structure | Starting from |
| 5 | 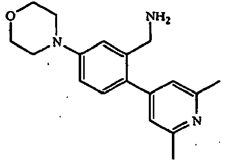 | Intermediate 1 and morpholine |
| 6 | 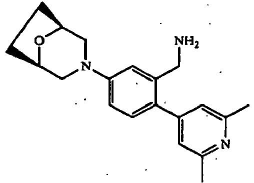 | Intermediate 1 and 8-Oxa-3-azabicyclo[3.2.1]octane hydrochloride [54745-74-3] |
| 7 | 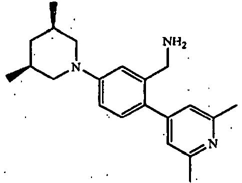 | Intermediate 1 and cis-3,5-dimethylpiperidine |
| 8 | 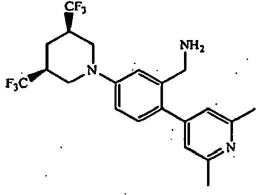 | Intermediate 1 and 4, with separation of the cis and trans isomers |
| 9 | 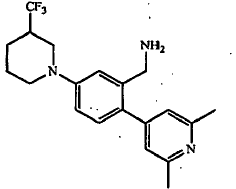 | Intermediate 1 and 3-(trifluoromethyl)-piperidine |

**Table 2**

| The following intermediates were prepared according to the procedures used for Intermediate 3 (starting from 2-bromo-5-chlorobenzonitrile) : | | |
|---|---|---|
| Intermediate | Structure | Starting from |
| 10 | 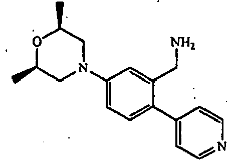 | 2-Bromo-5-chlorobenzonitrile and pyridine-4-boronic acid ([1692-15-5]) instead of 2,6-dimethylpyridine-4-boronic acid, pinacol ester |
| 11 | 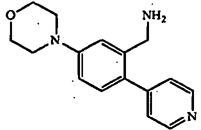 | 2-Bromo-5-chlorobenzonitrile and pyridine-4-boronic acid ([1692-15-5]) instead of 2,6-dimethylpyridine-4-boronic acid, pinacol ester, and with morpholine instead of cis-2,6-dimethylmorpholine |
| 12 | 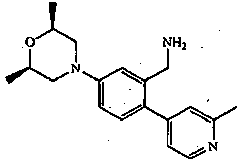 | 2-Bromo-5-chlorobenzonitrile and 2-methylpyridine-4-boronic acid, pinacol ester ([660867-80-1]) instead of 2,6-dimethylpyridine-4-boronic acid, pinacol ester |

### 2-bromo-5-(4-morpholinyl)-benzonitrile (Intermediate 10)

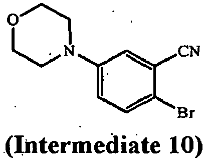

A mixture of 2-bromo-5-fluorobenzonitrile (10 g, 50 mmol), morpholine (4.37 mL, 50 mmol), and potassium carbonate (6.91 g, 50 mmol), in DMSO (100 mL) was stirred at 100 °C for 48 h. The reaction mixture was poured into water (200 mL) and was stirred for 1 h. The precipitate was filtered off and purified by column chromatography on silica gel (eluent gradient from 50/50 to 100/0 DCM/heptane). The desired fractions were collected and evaporated, yielding 4.1 g (30 %) of Intermediate 10, after drying overnight under vacuum at 50 °C.

### 2-(2-methyl-4-pyridinyl)-5-(4-morpholinyl)-benzonitrile (Intermediate 11)

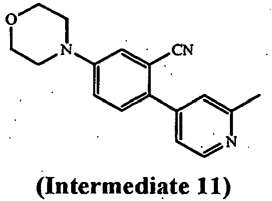

A mixture of Intermediate 10 (4.1 g, 15.35 mmol), 2-methylpyridine-4-boronic acid, pinacol ester ([660867-80-1], 3.70 g, 16.88 mmol), tetrakis(triphenylphosphine) palladium (0.88 g, 0.77 mmol), 1,4-dioxane (50 mL), and sodium carbonate (3.58 g, 33.67 mmol) dissolved in water (20 mL) and EtOH (30 mL) was degassed and the vessel was closed. The reaction mixture was stirred and heated under a nitrogen atmosphere at 120 °C for 2 h. The solvent was evaporated. The residue was taken up in DCM and washed with water. The organic layer was dried with MgSO₄ and evaporated. The residue was purified by column chromatography on silica gel (eluent gradient from 100% DCM to 99/1 DCM/MeOH). The desired fractions were collected and evaporated, yielding 3.7 g (86 %) of Intermediate 11 after drying overnight under vacuum at 50 °C.

### 2-(2-methyl-4-pyridinyl)-5-(4-morpholinyl)-benzenemethanamine (Intermediate 12)

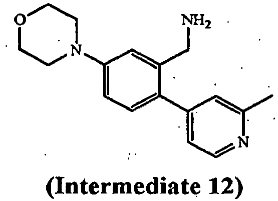

Intermediate 12 was prepared according to the procedure used for Intermediate 3, starting from Intermediate 11 instead of Intermediate 2.

### 3-[(2R,6S)-2,6-dimethyl-4-morpholinyl]-5-fluoro-benzonitrile (Intermediate 13)

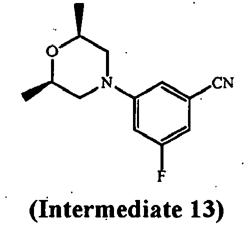

A mixture of 3-bromo-5-fluorobenzonitrile (55.56 g, 277.80 mmol), cis-2,6-dimethylmorpholine (34.40 mL, 277.80 mmol), sodium tert-butoxide (40.05 g, 416.70 mmol) and dry toluene (300 mL) was introduced in a pressure tube and was degassed with nitrogen for 5 min. Xantphos [161265-03-8] (9.64 g, 16.67 mmol) and Pd₂(dba)₃ (5.09 g, 5.55 mmol) were added and the mixture was further degassed for 5 min. The tube was sealed and the reaction mixture was stirred at 120 °C for 2 h. The reaction mixture was diluted with CH₂Cl₂ and washed with water. The aqueous layer was extracted with CH₂Cl₂. The combined organic layers were washed with water, dried on MgSO₄, filtered and evaporated. The residue was purified by column chromatography on silica gel (eluent gradient from 100% DCM to 99/1 DCM/MeOH). The desired fractions were evaporated. The residue was triturated in Et₂O and was filtered, yielding 33 g of Intermediate 13 (51 %) as a white solid after drying in a vacuum oven at 50°C overnight,

### 5-[(2R,6S)-2,6-dimethyl-4-morpholinyl]-3-fluoro-2-iodo-benzonitrile (Intermediate 14)

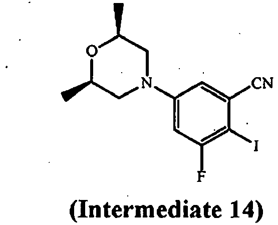

A solution of 2,2,6,6-tetramethylpiperidine (18.82 mL, 110.58 mmol) in THF (1 L) was cooled to 0 °C. N-Butyllithium (2.5 M in hexanes, 44.23 mL, 110.58 mmol) was added dropwise while stirring at 0 °C. The reaction mixture was then stirred for 15 min at 0 °C. The resulting solution was cooled to -78 °C and a solution of Intermediate 13 (29.44 g, 100.53 mmol) dissolved in THF (200 mL) was added dropwise over 20 min. The dark yellow solution was stirred at -78 °C for 30 min before a solution of iodine (30.62 g, 120.64 mmol) dissolved in THF (250 ml) was added dropwise over 15 min. The mixture was stirred for 30 min at -78 °C before being allowed to warm up to room temperature. Stirring was continued for 1 h. The reaction was quenched with aqueous NH₄Cl. The mixture was diluted with water and Et₂O and the layers were separated. The aqueous layer was extracted with Et₂O. The combined organic layer was washed again with aqueous Na₂S₂O₃ and was dried on MgSO₄, filtered and evaporated. The residue was triturated in warm DCM and was filtered, yielding 31.28 g of Intermediate 14 (86 %) as a white solid after drying in a vacuum oven at 50°C overnight.

### 5-[(2R,6S)-2,6-dimethyl-4-morpholinyl]-2-(2,6-dimethyl-4-pyridinyl)-3-fluoro-benzonitrile (Intermediate 15)

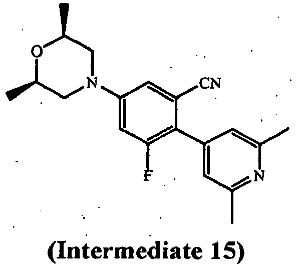

A mixture of Intermediate 14 (12.09 g, 33.57 mmol), 2,6-dimethylpyridine-4-boronic acid, pinacol ester ([325142-95-8], 11.74 g, 50.35 mmol), tetrakis(triphenylphosphine)palladium (2.33 g, 2.01 mmol), 1,4-dioxane (100 mL), and sodium carbonate 1 M (100 mL, 100 mmol) was degassed with nitrogen. The reaction mixture was stirred and heated under a nitrogen atmosphere at 140 °C for 12 h. The reaction mixture was diluted with DCM and water and the layers were separated. The aqueous layer was extracted with DCM. The combined organic layer was dried with MgSO₄, filtered and evaporated. The residue was purified by column chromatography on silica gel (eluent gradient from 100% DCM to 99/1 DCM/MeOH). The desired fractions were collected and evaporated, yielding 8.37 g (73 %) of Intermediate 15, after drying overnight under vacuum at 50 °C.

### 5-[(2R,6S)-2,6-dimethyl-4-morpholinyl]-2-(2,6-dimethyl-4-pyridinyl)-3-fluoro-benzenemethanamine (Intermediate 13)

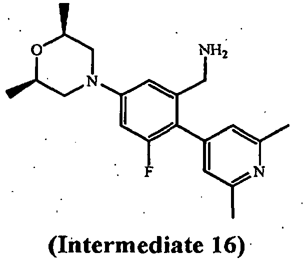

Intermediate 16 was prepared according to the procedure used for Intermediate 3, starting from Intermediate 15 instead of Intermediate 2.

### 5-[(2R,6S)-2,6-dimethyl-4-morpholinyl]-3-fluoro-2-(2-methyl-4-pyridinyl)-benzenemethanamine (Intermediate 17)

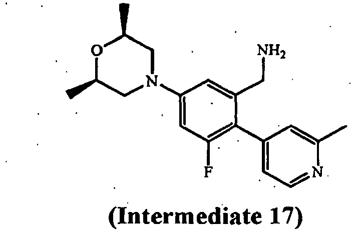

Intermediate 17 was prepared according to the procedures used for Intermediate 13, starting from Intermediate 14, and using 2-methylpyridine-4-boronic acid, pinacol ester ([660867-80-1] instead of 2,6-dimethylpyridine-4-boronic acid, pinacol ester.

### 2-bromo-N-(phenylmethyl)-N-(3,3,3-trifluoro-2-hydroxypropyl)-acetamide (Intermediate 18)

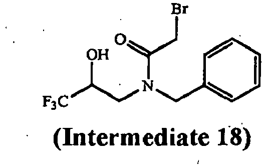

A solution of 1,1,1-Trifluro-3-benzylaminopropan-2-ol ([178218-36-5], 11.64 g, 53.12 mmol) and triethylamine (7.38 mL, 53.12 mmol) in DCM (150 mL) was cooled to 0 °C under nitrogen atmosphere. Bromoacetyl chloride (5.41 mL, 58.43 mmol) dissolved in DCM (50 mL) was added dropwise and the reaction mixture was stirred for 30 min. The reaction mixture was diluted with DCM, washed with HCl 1N followed by aqueous sodium hydrogenocarbonate and brine. The organic layer was dried with MgSO₄, filtered and evaporated, yielding 18.07 g of Intermediate 18 (quantitative) that was used without further purification.

### 4-(phenylmethyl)-6-(trifluoromethyl)-3-morpholinone (Intermediate 19)

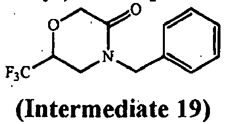

Intermediate 18 (123 g, 361.61 mmol) dissolved in dry THF (500 mL) was added dropwise to a suspension of sodium hydride (60 % dispersion in mineral oil, 17.35 g, 433.94 mmol) in dry THF (500 mL) cooled to -5 °C under nitrogen atmosphere. After addition, the reaction mixture was warmed to room temperature and stirred for 30 min. The reaction was quenched by careful addition of MeOH at 0 °C. The reaction mixture was then poured into HCl 1 N (100 mL) and the aqueous layer was extracted with EtOAc. The organic layer was washed with aqueous hydrogenocarbonate, and with brine before drying with MgSO₄, filtration and evaporation, yielding 90 g of Intermediate 19 (96 %) as an oil that was used without further purification.

### 4-(phenylmethyl)-2-(trifluoromethyl)-, (2S)-morpholine (Intermediate 20) and 4-(phenylmethyl)-2-(trifluoromethyl)-, (2R)-morpholine (Intermediate 21)

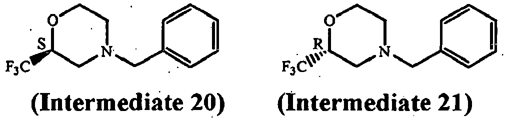

Borane/THF complex solution 1 M ([14044-65-6], 868 mL, 868.96 mmol) was added dropwise to a solution of Intermediate 19 (90 g, 347.18 mmol) dissolved in dry THF (850 mL) at room temperature under a nitrogen atmosphere. The mixture was stirred for 2.5 h at 70 °C (until no gas evolution occurred anymore). MeOH (70.32 mL, 1735.92 mmol) was then added dropwise, followed by HCl (3 M in water, 578 mL, 1735.92 mmol) and the mixture was stirred at 70 °C for another hour. The reaction mixture was then quenched with potassium carbonate (239.91 g, 1735.92 mmol) and dissolved in water (250 mL). The reaction mixture was extracted with diethyl ether. The organic layer was washed with brine, dried with MgSO₄ filtered and evaporated. The resulting oil was purified by column chromatography on silica gel (eluent: DCM), yielding a clear oil that was then separated into its pure enantiomers by column chromatography on Chiralcel OJ 1000Å 20µm (Daicel) (eluent: heptane/iPrOH 99/1), yielding 27 g of Intermediate 21 (32 %) and 25 g of Intermediate 20 (29 %), both as clear oils. The absolute stereochemistry of these two enantiomers was determined by VCD.

### 2-(trifluoromethyl)-(2S)-morpholine hydrochloride (Intermediate 22)

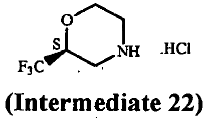

Intermediate 20 (25 g, 101.94 mmol) was added to a suspension of Pd on charcoal 10 % (2 g) in MeOH (150 mL). The reaction mixture was stirred at 25 °C under a hydrogen atmosphere until 1 equivalent of hydrogen were absorbed. The catalyst was removed by filtration over diatomaceous earth. The filtrate was treated with aqueous HCl 5.5 M (18.5 mL, 101.94 mmol). The solvent was evaporated, yielding 18.46 g of Intermediate 22 (95 %) as a white solid.

### 2-(trifluoromethyl)-(2R)-morpholine hydrochloride (Intermediate 23)

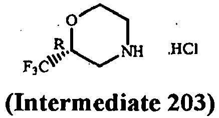

Intermediate 21 (27 g, 110.09 mmol) was added to a suspension of Pd on charcoal 10 % (2 g) in MeOH (150 mL). The reaction mixture was stirred at 25 °C under a hydrogen atmosphere until 1 equivalent of hydrogen were absorbed. The catalyst was removed by filtration over diatomaceous earth. The filtrate was treated with aqueous HCl 5.5 M(20.0 mL, 110.09 mmol). The solvent was evaporated, yielding 16.4 g of Intermediate 23 (78 %) as a white solid.

### 3-fluoro-5-[(2S)-2-(trifluoromethyl)-4-morpholinyl]-benzonitrile (Intermediate 24)

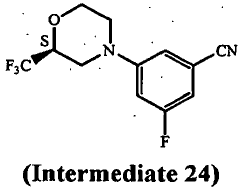

A mixture of Intermediate 22 (4.94 g; 25 mmol) and sodium tert-butoxide (6 g, 62.5 mmol) in monoglyme (50 mL) was degassed for 15 min with nitrogen. The Nolan catalyst ([478980-01-7], 718 mg, 1.25 mmol) was added, followed by 3-bromo-5-fluorobenzonitrile (5 g, 25 mmol). The reaction mixture was stirred at 50 °C for 48 h. The reaction mixture was poured into ice/water. The mixture was neutralized with 1N HCl and was extracted twice with EtOAc. The combined organic layer was washed with brine, dried on MgSO₄, filtered and evaporated. The residue was purified by column chromatography on silica gel (eluent 50/50 DCM/heptane). The desired fractions were evaporated and the residue was crystallized in iPr₂O/hepta 1/10, yielding 3 g of Intermediate 24 (44 %) after drying in a vacuum oven at 50°C overnight.

### 2-iodo-3-fluoro-5-[(2S)-2-(trifluoromethyl)-4-morpholinyl]-benzonitrile (Intermediate 25)

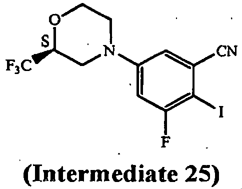

Intermediate 25 was prepared according to the procedure used for Intermediate 14, starting from Intermediate 24 instead of Intermediate 13.

### 2-(2,6-dimethyl-4-pyridinyl)-3-fluoro-5-[(2S)-2-(trifluoromethyl)-4-morpholinyl]-benzonitrile (Intermediate 26)

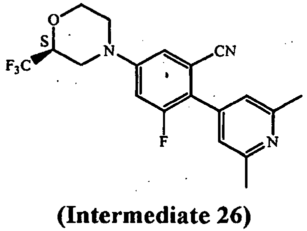

A mixture of Intermediate 25 (4.03 g, 10.07 mmol), 2,6-dimethylpyridine-4-boronic acid, pinacol ester ([325142-95-8], 2.82 g, 12.09 mmol), tetrakis(triphenylphosphine)-palladium (582 mg, 0.50 mmol), dimethoxyethane (100 mL), and potassium carbonate 2 M (10.07 mL, 20.14 mmol) was degassed with nitrogen. The reaction mixture was stirred in a closed vessel and heated under nitrogen atmosphere at 145 °C for 3 h. After evaporation of the solvent, the reaction mixture was diluted with DCM and water and the layers were separated. The aqueous layer was extracted with DCM. The combined organic layer was washed with brine, dried with MgSO₄, filtered and evaporated. The residue was purified by column chromatography on silica gel (eluent gradient from 10/90 to 50/50 EtOAc/heptane). The desired fractions were collected and evaporated, yielding 2.05 g (54 %) of Intermediate 26, as a white solid after drying overnight under vacuum at 50 °C.

### 2-(2,6-dimethyl-4-pyridinyl)-3-fluoro-5-[(2S)-2-(trifluoromethyl)-4-morpholinyl]-benzenemethanamine (Intermediate 27) and 2-(2,6-dimethyl-4-pyridinyl)-3-fluoro-5-[2-(methyl)-4-morpholinyl]-benzenemethanamine (Intermediate 28)

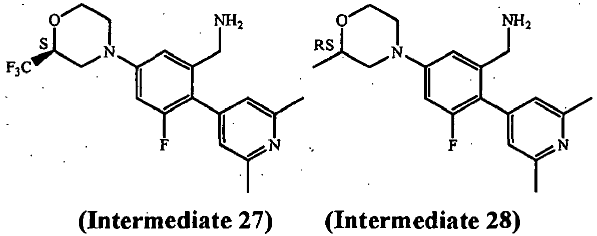

Intermediates 27 and 28 were prepared according to the procedure used for Intermediate 3, starting from Intermediate 26 instead of Intermediate 2. Racemic intermediate 28 was formed as a side-product in this step (ratio 27:28 = 64:36). The two intermediates were used as a mixture for the synthesis of the final compounds 64 to 70 and 112 to 118, which were separated by chromatography.

### 2-(2,6-dimethyl-4-pyridinyl)-3-fluoro-5-[(2R)-2-(trifluoromethyl)-4-morpholinyl]-benzenemethanamine (Intermediate 29)

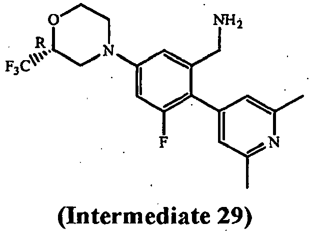

Intermediate 29 was prepared according to the procedures used for Intermediate 27, using Intermediate 23 instead of Intermediate 22.Racemic intermediate 28 was also obtained as a side product in this step (ratio 29:28 = 88:12). The two intermediates were used as a mixture for the synthesis of the final compounds 56 to 59 and 71 to 73 and 112 to 118, which were separated by chromatography.

### 3-fluoro-5-[3-(trifluoromethyl)-1-piperidinyl]-benzonitrile (Intermediate 30)

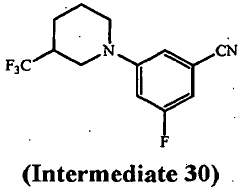

A mixture of 3,5-difluorobenzonitrile (1.38 g, 9.9 mmol), 3-trifluoromethylpiperidine (1.82 g, 11.88 mmol) and diisopropylethylamine (2.56, 14.85 mmol) in acetonitrile (20 mL) was stirred in a pressure tube at 150°C for 72 h.The reaction mixture was evaporated, taken up in water/DCM, and basified with K₂CO₃. The organic layer was separated and the water layer was extracted once more with DCM. The combined organic layers were dried on MgSO₄, filtered and evaporated. The residue was purified by column chromatography on silica gel (eluent: gradient heptane/DCM 30/70 to 70/30) as eluent. The desired fractions were evaporated, yielding 2.5 g of Intermediate 30 (93 %) after drying in a vacuum oven at 50°C overnight.

### 2-(2,6-dimethyl-4-pyridinyl)-3-fluoro-5-[3-(trifluoromethyl)-1-piperidinyl]-benzenemethanamine (Intermediate 31)

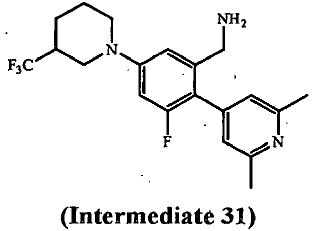

Intermediate 31 was prepared according to the procedures used for Intermediate 27, starting from Intermediate 30 instead of Intermediate 24.

### 3-bromo-6-(2,6-dimethyl-4-pyridinyl)-2-fluoro-benzonitrile (Intermediate 32)

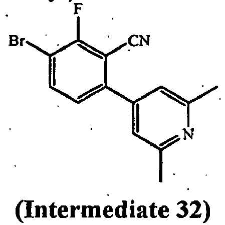

3-Bromo-2-fluoro-6-iodobenzonitrile (10 g, 30.68 mmol), 2,6-dimethylpyridine-4-boronic acid, pinacol ester ([325142-95-8], 7.51 g, 32.22 mmol), and dimethoxyethane (400 mL) were charged in a pressure tube and the mixture was degassed with nitrogen Potassium carbonate 2 M (46 mL, 92.05 mmol) and tetrakis(triphenylphosphine)-palladium (1.77 g, 1.53 mmol) were added while degassing with nitrogen. The reaction mixture was stirred and heated under nitrogen atmosphere at 100 °C for 17 h. The solvent was evaporated and the residue was diluted with DCM and water and the layers were separated. The aqueous layer was extracted twice with DCM. The combined organic layer was dried with MgSO₄, filtered and evaporated. The residue was purified by column chromatography on silica gel (eluent: DCM). The desired fractions were collected and evaporated, yielding 5.96 g (63 %) of Intermediate 32 as an off-white solid after drying overnight under vacuum at 50 °C.

### 3-[(2R,6S)-2,6-dimethyl-4-morpholinyl]-6-(2,6-dimethyl-4-pyridinyl)-2-fluoro-benzonitrile (Intermediate 33)

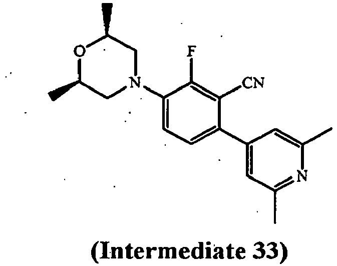

A mixture of Xantphos [161265-03-8] (265 mg, 0.46 mmol) and Pd₂(dba)₃ (191 mg, 0.21 mmol) in 1,4-dioxane (45 mL) was degassed for 5 min. Intermediate 110 (2.54 g, 8.33 mmol), cis-2,6-dimethylmorpholine (1.24 mL), 9.99 mmol) and cesium carbonate (5.43 g, 16.66 mmol) were added and the mixture was further degassed with nitrogen for 5 min. The tube was sealed and the reaction mixture was stirred at 140 °C for 1.5 h in a microwave oven. The reaction mixture was poured into water. The precipitate was filtered, rinsed with water and dried under vacuum. The resulting solid was purified by column chromatography on silica gel (eluent gradient from 100% DCM to 98/2 DCM/MeOH). The desired fractions were evaporated, yielding 2.4 g of Intermediate 33 (85 %) as a yellow solid after drying in a vacuum oven at 50°C overnight.

### 3-[(2R,6S)-2,6-dimethyl-4-morpholinyl]-6-(2,6-dimethyl-4-pyridinyl)-2-fluoro-benzenemethanamine (Intermediate 34)

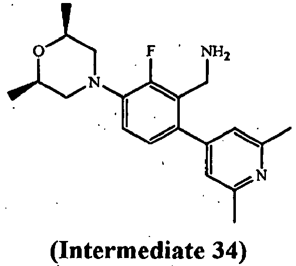

Intermediate 34 was prepared according to the procedure used for Intermediate 3, starting from Intermediate 33 instead of Intermediate 2.

### 6-(2,6-dimethyl-4-pyridinyl)-2-fluoro-3-[(2S)-2-(trifluoromethyl)-4-morpholinyl]-benzonitrile (Intermediate 35)

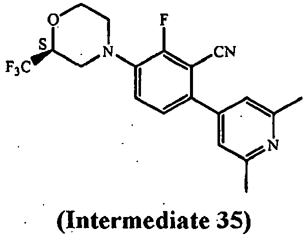

A mixture of Xantphos [161265-03-8] (171 mg, 0.29 mmol) and Pd₂(dba)₃ (135 mg, 0.15 mmol) in dry toluene (30 mL) was degassed for 5 min. Intermediate 22 (1.13 g, 5.90 mmol), Intermediate 32 (1.5 g, 4.91 mmol) and sodium tert-butoxide (1.42 g, 14.75 mmol) were added and the mixture was further degassed with nitrogen for 5 min. The tube was sealed and the reaction mixture was stirred at 140 °C for 1 h. The solvent was evaporated and the residue was taken up in DCM and water. The layers were separated and the aqueous layer was extracted twice with DCM. The combined organic layer was washed with brine, dried with MgSO₄ filtered and evaporated. The residue was purified by column chromatography on silica gel (eluent gradient from 100% DCM to 97/3 DCM/MeOH). The desired fractions were collected and evaporated, yielding 1.2 g (64 %) of Intermediate 35 as an orange solid after drying overnight under vacuum at 50 °C.

### 6-(2,6-dimethyl-4-pyridinyl)-2-fluoro-3-[(2S)-2-(trifluoromethyl)-4-morpholinyl]-benzenemethanamine (Intermediate 36)

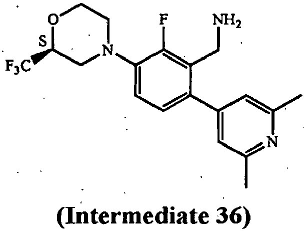

Intermediate 36 was prepared according to the procedure used for Intermediate 3, starting from Intermediate 35 instead of Intermediate 2.

### 6-(2,6-dimethyl-4-pyridinyl)-2-fluoro-3-[(2R)-2-(trifluoromethyl)-4-morpholinyl]-benzenemethanamine (Intermediate 37)

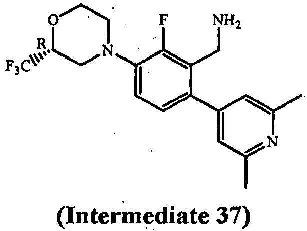

Intermediate 37 was prepared according to the procedure used for Intermediate 36, starting from Intermediate 23 instead of Intermediate 22.

### Nonafluorobutane-1-sulfonic acid (2R,6S)-2,6-dimethyl-3,6-dihydro-2H-pyran-4-yl ester (Intermediate 38)

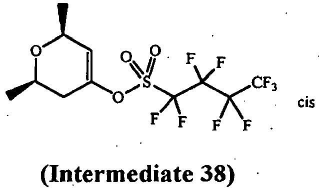

LDA (2 M in THF/heptane/ethylbenzene, 93.62 mL, 187.25 mmol) was cooled to -78 °C under a nitrogen atmosphere. A solution of (2R,6S)-2,6-dimethyltetrahydro-4-pyranone ([14505-80-7], 20 g, 156.04 mmol) in THF (400 mL) was added dropwise while stirring at -78 °C. After addition, the reaction mixture was allowed to warm to 0 °C and was stirred for 1 h. The solution was cooled to -78 °C again and nonafluoro-1-butanesulfonylfluoride ([375-72-4], 36.43 mL, 202.85 mmol) was then added dropwise. The reaction mixture was allowed to warm to room temperature and was further stirred for 12 h. The reaction mixture was quenched with saturated aqueous NaHCO₃ (300 mL) and the mixture was extracted twice with EtOAc (2 x 300 mL). The combined organic layer was washed with brine, dried with MgSO₄, filtered, and evaporated. The crude mixture was purified by column chromatography on silica gel (eluent: gradient EtOAc/heptane 0/100 to 5/95). The desired fractions were collected and evaporated, yielding 48.5 g (76 %) of Intermediate 38 as a clear oil.

### 3,6-dihydro-(2R,6S)-2,6-dimethyl-4-(4,4,5,5-tetramethyl-1,3,2-dioxaborolan-2-yl)-2H-pyran (Intermediate 39)

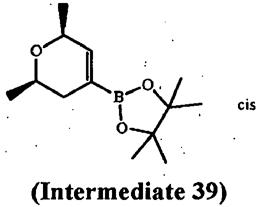

A pressure tube was charged with Intermediate 38 (48.5 g, 118.22 mmol), bis(pinacolato)diboron ([73183-34-3], 36.02 g, 141.86 mmol), 1.1'-bisdiphenylphosphino)ferrocene ([12150-46-8], 1.96 g, 3.55 mmol), potassium acetate (11.60 g, 118.22 mmol) and dimethoxyethane (944 mL). The mixture was degassed with nitrogen before the addition of [1,1'-bis(diphenylphosphino)ferrocene]-dichloropalladium(II) ([72287-26-4], 1.90 g, 2.60 mmol). The reaction mixture was stirred at 80 °C for 3 h. After cooling, the reaction mixture was filtered on diatomaceous earth and the filtrate was evaporated. The residue was purified by column chromatography on silica gel (eluent: gradient EtOAc/heptane 0/100 to 5/95). The desired fractions were collected and evaporated, yielding 22.2 g (79 %) of Intermediate 39 as a slightly yellow oil.

### 3-(3,6-dihydro-(2R,6S)-2,6-dimethyl-2H-pyran-4-yl)-6-(2,6-dimethyl-4-pyridinyl)-2-fluoro-benzonitrile (Intermediate 40)

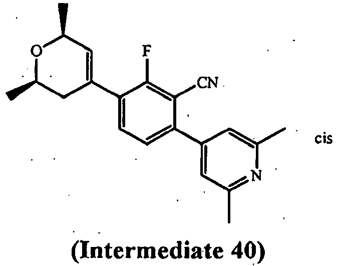

Intermediate 32 (1.70 g, 5.58 mmol), Intermediate 39 (1.46 g, 6.14 mmol), potassium carbonate 2 M (5.59 mL, 11.17 mmol) and tetrakis(triphenylphosphine)palladium (323 mg, 0.28 mmol) and dimethoxyethane (117 mL) were charged in a pressure tube and the mixture was degassed with nitrogen. The reaction mixture was stirred and heated under nitrogen atmosphere at 100 °C for 17 h. After cooling to room temperature, water was added to the reaction mixture until precipitation of the product. The solid was recrystallised from this mixture by adding a small amount of acetonitrile to dissolve the product in the warm mixture. The crystallised solid was filtered, yielding 1.53 g (81 %) of Intermediate 40 as an off-white solid after drying overnight under vacuum at 50 °C.

### 3-(3,6-dihydro-(2R,6S)-2,6-dimethyl-2H-pyran-4-yl)-6-(2,6-dimethyl-4-pyridinyl)-2-fluoro-benzenemethanamine (Intermediate 41)

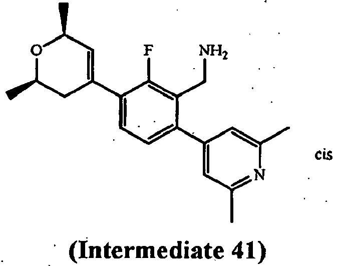

Intermediate 41 was prepared according to the procedure used for Intermediate 3, starting from Intermediate 40 instead of Intermediate 2.

### 6-(2,6-dimethyl-4-pyridinyl)-2-fluoro-3-(tetrahydro-(2R,6S)-2,6-dimethyl-2H-pyran-4-yl)-benzenemethanamine (Intermediate 42)

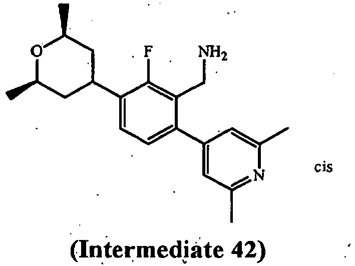

Intermediate 41 (200 mg, 0.58 mmol) was added to a suspension of Pt on charcoal 5 % (50 mg) in THF (40 mL) under nitrogen atmosphere. The reaction mixture was stirred at 50 °C under hydrogen atmosphere until 1 equivalent of hydrogen was absorbed. The catalyst was removed by filtration over diatomaceous earth. The solvent was evaporated, yielding Intermediate 42 quantitatively as a clear oil, which was used without further purification.

### 4-(4-bromo-2-chloro-5-fluorophenyl)-2,6-dimethyl-pyridine (Intermediate 43)

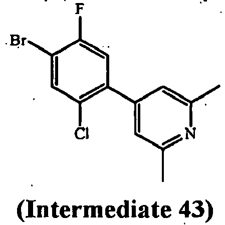

1-bromo-5-chloro-2-fluoro-4-iodo-benzene ([1000572-73-5 ], 9.7 g, 28.93 mmol), 2,6-dimethylpyridine-4-boronic acid, pinacol ester ([325142-95-8], 8.09 g, 34.71 mmol), potassium carbonate 2 M (28.93 mL, 57.85 mmol) and tetrakis(triphenylphosphine)-palladium (2 g, 1.74 mmol) and dimethoxyethane (150 mL) were charged in a pressure tube and the mixture was degassed with nitrogen. The reaction mixture was stirred and heated under nitrogen atmosphere at 100 °C for 18 h. The solvent was evaporated. The residue was taken up in water and extracted with DCM. The organic layer was dried on MgSO₄, filtered and evaporated. The residue was purified by column chromatography on silica gel (eluent: gradient DCM/MeOH 100/0 to 97/3). The desired fractions were collected and evaporated, yielding 5.8 g (64 %) of Intermediate 43.

### 4-[5-chloro-4-(2,6-dimethyl-4-pyridinyl)-2-fluorophenyl]-2,6-dimethyl-, (2R,6S)-morpholine (Intermediate 44)

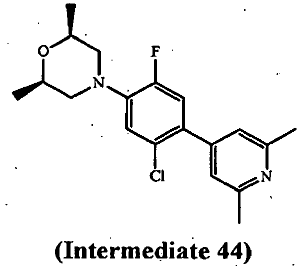

A pressure tube was charged with a mixture of Intermediate 43 (5.6 g, 17.8 mmol), cis-2,6-dimethylmorpholine (2.05 g, 17.8 mmol) and sodium tert-butoxide (2.56 g, 26.7 mmol) in toluene (150 mL). The mixture was degassed for 5 min before Xantphos [161265-03-8] (618 mg, 1.07 mmol) and Pd₂(dba)₃ (326 mg, 0.35 mmol) were added. The mixture was further degassed for 15 min. The tube was sealed and the reaction mixture was stirred at 120 °C for 1 h. The solvent was evaporated. Water was added to the residue and it was extracted twice with DCM. The combined organic layer was dried on MgSO₄, filtered and evaporated. The residue was purified by column chromatography on silica gel (eluent gradient from 100% DCM to 98/2 DCM/MeOH). The desired fractions were evaporated, yielding 2.4 g of Intermediate 44 (37 %) after drying in a vacuum oven at 50°C overnight.

### 5-[(2R,6S)-2,6-dimethyl-4-morpholinyl]-2-(2,6-dimethyl-4-pyridinyl)-4-fluoro-benzonitrile (Intermediate 45)

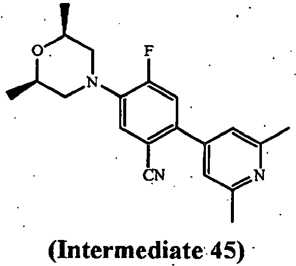

A pressure tube was charged with a mixture of Intermediate 44 (1 g, 2.86 mmol), zinc cyanide (202 mg, 1.72 mmol), triphenylphosphine (75 ing, 0.28 mmol) and tetrakis(triphenylphosphine)palladium (165 mg, 0.14 mmol) in acetonitrile (2 mL). The mixture was stirred at 170 °C in a microwave oven for 18 h. The solvent was evaporated and the residue was purified by preparative HPLC on RP Vydac® Denali® C18 - 10µm, 250g, 5cm, mobile phase (0.5% NH₄Ac solution in water + 10% CH₃CN, CH₃CN). The desired fractions were collected and concentrated. The precipitate was filtered off and washed with water, yielding 850 mg of Intermediate 45 (87 %).

### 5-[(2R,6S)-2,6-dimethyl-4-morpholinyl]-2-(2,6-dimethyl-4-pyridinyl)-4-fluoro-benzenemethanamine (Intermediate 46)

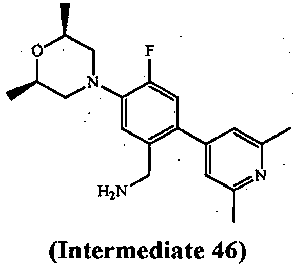

Intermediate 46 was prepared according to the procedure used for Intermediate 3, starting from Intermediate 45 instead of Intermediate 2.

### 4-(2,4-dibromo-3,6-difluorophenyl)-2,6-dimethyl-pyridine (Intermediate 47)

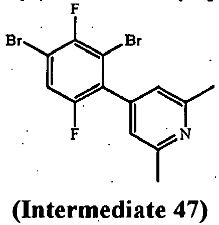

1,3-Dibromo-2,5-difluoro-4-iodo-benzene ([1000577-89-8], 3.6 g, 9.05 mmol), 2,6-dimethylpyridine-4-boronic acid, pinacol ester ([325142-95-8], 2.11 g, 9.05 mmol), potassium carbonate 2 M (9.05 mL, 18.1 mmol) and tetraki s(triphenylphosphine)-palladium (627 mg, 0.54 mmol) and dimethoxyethane (57 mL) were charged in a pressure tube and the mixture was degassed with nitrogen. The reaction mixture was stirred and heated under nitrogen atmosphere at 130 °C for 18 h. The solvent was evaporated. The residue was taken up in water and extracted with DCM. The organic layer was dried on MgSO₄, filtered and evaporated. The residue was purified by column chromatography on silica gel (eluent: DCM). The desired fractions were collected and evaporated, yielding 1.6 g (47%) of Intermediate 47.

### 4-[3-bromo-4-(2,6-dimethyl-4-pyridinyl)-2,5-difluorophenyl]-2,6-dimethyl-, (2R,6S)-morpholine (Intermediate 48)

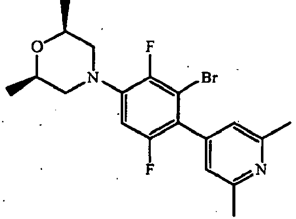

### (Intermediate 48)

A pressure tube was charged with a mixture of Intermediate 47 (800 mg, 2.12 mmol), cis-2,6-dimethylmorpholine (203 mg, 1.77 mmol) and sodium tert-butoxide (255 mg, 2.65 mmol) in toluene (150 mL). The mixture was degassed for 5 min before Xantphos [161265-03-8] (61 mg, 0.10 mmol) and Pd₂(dba)₃ (32 mg, 0.03 mmol) were added. The mixture was further degassed for 15 min. The tube was sealed and the reaction mixture was stirred at 100 °C for 16 h. The solvent was evaporated. Water was added to the residue and it was extracted twice with DCM. The combined organic layer was dried on MgSO₄, filtered and evaporated. The residue was purified by column chromatography on silica gel (eluent: DCM). The desired fractions were evaporated. The solid was recrystallised from heptane, yielding 229 mg of Intermediate 48 (31 %) after drying in a vacuum oven at 50°C overnight.

### 3-[(2R,6S)-2,6-dimethyl-4-morpholinyl)-6-(2,6-dimethyl-4-pyridinyl)-2,5-difluoro-benzonitrile (Intermediate 49)

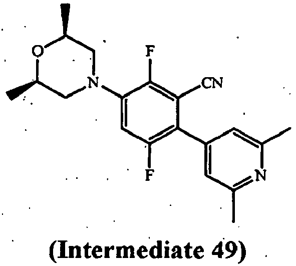

A pressure tube was charged with a mixture of Intermediate 48 (280 mg, 0.68 mmol), zinc cyanide (48 mg, 0.41 mmol), triphenylphosphine (18 mg, 0.07 mmol) and tetrakis(triphenylphosphine)palladium (79 mg, 0.07 mmol) in acetonitrile (15 mL). The mixture was stirred at 150 °C in a microwave oven for 18 h. The solvent was evaporated and the residue taken up in water and DCM. The organic layer was separated, dried with MgSO₄, filtered and evaporated. The residue was purified by column chromatography on silica gel (eluent: DCM). The desired fractions were collected and concentrated, yielding Intermediate 49 quantitatively.

### 3-[(2R,6S)-2,6-dimethyl-4-morpholinyl]-6-(2,6-dimethyl-4-pyridinyl)-2,5-difluoro-benzenemethanamine (Intermediate 50)

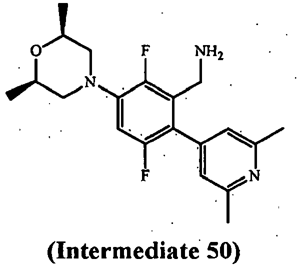

Intermediate 50 was prepared according to the procedure used for Intermediate 3, starting from Intermediate 49 instead of Intermediate 2.

### 5-bromo-3-chloro-2-(2,6-dimethyl-4-pyridinyl)-benzonitrile (Intermediate 51)

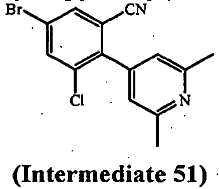

5-bromo-3-chloro-2-iodobenzonitrile ([1000577-40-1], 6.9 g, 20.15 mmol), 2,6-dimethylpyridine-4-boronic acid, pinacol ester ([325142-95-8], 5.64 g, 24.18 mmol), potassium carbonate 2 M (20.15 mL, 40.31 mmol), tetrakis(triphenylphosphine) palladium (1.40 g, 1.21 mmol) and dimethoxyethane (150 mL) were charged in a pressure tube and the mixture was degassed with nitrogen. The reaction mixture was stirred and heated under nitrogen atmosphere at 120 °C for 18 h. The solvent was evaporated. The residue was taken up in water and extracted with DCM. The organic layer was dried on MgSO₄, filtered and evaporated. The residue was purified by column chromatography on silica gel (eluent: DCM). The desired fractions were collected and evaporated, yielding 2.3 g (35 %) of Intermediate 51.

### 3-chloro-5-[(2R,6S)-2,6-dimethyl-4-morpholinyl]-2-(2,6-dimethyl-4-pyridinyl)-benzonitrile (Intermediate 52)

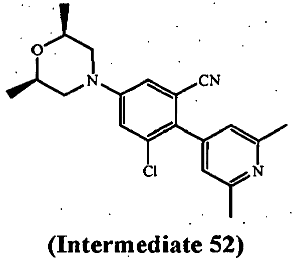

A pressure tube was charged with a mixture of Intermediate 51 (2.2 g, 6.84 mmol), cis-2,6-dimethylmorpholine (656 mg, 5.70 mmol) and sodium tert-butoxide (822 mg, 8.55 mmol) in toluene (100 mL). The mixture was degassed for 5 min before Xantphos [161265-03-8] (198 mg, 0.34 mmol) and Pd₂(dba)₃ (104 mg, 0.11 mmol) were added. The mixture was further degassed for 15 min. The tube was sealed and the reaction mixture was stirred at 100 °C for 5 h. The solvent was evaporated. Water was added to the residue and it was extracted with DCM. The combined organic layer was dried on MgSO₄, filtered and evaporated. The residue was purified by column chromatography on silica gel (eluent: DCM). The desired fractions were evaporated, yielding 600 mg of Intermediate 52 (29 %) after drying in a vacuum oven at 50°C overnight.

### 3-chloro-5-[(2R,6S)-2,6-dimethyl-4-morpholinyl]-2-(2,6-dimethyl-4-pyriditiyl)-benzenemethanamine (Intermediate 53)

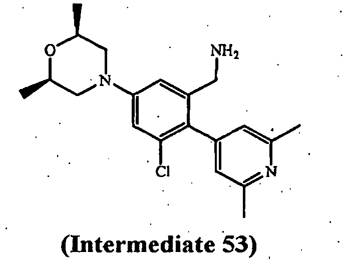

Intermediate 53 was prepared according to the procedure used for Intermediate 3, starting from Intermediate 52 instead of Intermediate 2.

### 5-chloro-2-[(2R,6S)-2,6-dimethyl-4-morpholinyl]-benzonitrile (Intermediate 54)

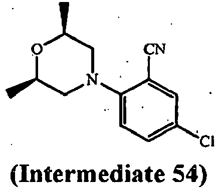

A mixture of Xantphos [161265-03-8] (294 mg, 0.51 mmol) and Pd₂(dba)₃ (211 mg, 0.23 mmol) in dry 1,4-dioxane (75 mL) was degassed for 5 min. Cis-2,6-dimethylmorpholine (1.5 mL, 12.13 mmol), 2-bromo-5-chlorobenzonitrile (2.5 g, 11.55 mmol) and cesium carbonate (7.52 g, 23.10 mmol) were added and the mixture was further degassed with nitrogen for 5 min. The tube was sealed and the reaction mixture was stirred at 145 °C for 5 h. The solvent was evaporated and the residue was purified by column chromatography on silica gel (eluent gradient DCM/heptanes 0/100 to 100/0). The desired fractions were collected and evaporated, yielding 1.39 g (39 %) of Intermediate 54 as a yellow oil.

### 2-[(2R,6S)-2,6-dimethyl-4-morpholinyl]-5-(2,6-dimethyl-4-pyridinyl)-benzonitrile (Intermediate 55)

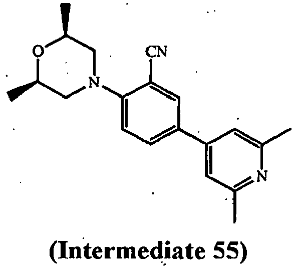

Intermediate 54 (550 mg, 2.19 mmol), 2,6-dimethylpyridine-4-boronic acid, pinacol ester ([325142-95-8], 767 mg, 3.29 mmol), sodium carbonate (697 mg, 6.58 mmol) and tetrakis(triphenylphosphine)palladium (152 mg, 0.13 mmol), water (5 mL), EtOH (5 mL), and 1,4-dioxane (5 mL) were charged in a pressure tube and the mixture was degassed with nitrogen. The reaction mixture was stirred and heated under nitrogen atmosphere at 130 °C for 4 h. The solvent was evaporated. The residue was taken up in water and extracted with DCM. The organic layer was dried on MgSO₄, filtered and evaporated. The residue was purified by column chromatography on silica gel (eluent: gradient DCM/MeOH 100/0 to 99/1). The desired fractions were collected and evaporated. The solid was recrystallised from EtOAc, yielding 485 mg (70 %) of Intermediate 55, after drying in a vacuum oven at 50°C overnight.

### 2-[(2R,6S)-2,6-dimethyl-4-morpholinyl]-5-(2,6-dimethyl-4-pyridinyl)-benzenemethanamine (Intermediate 56)

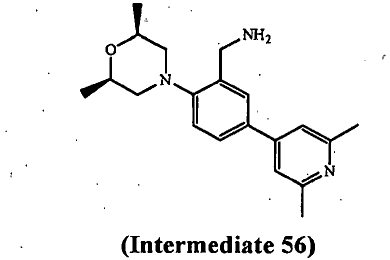

Intermediate 56 was prepared according to the procedure used for Intermediate 3, starting from Intermediate 55 instead of Intermediate 2.

### B. Preparation of the Final compounds

### B1) Example 1

### N-[[5-[(2R,6S)-2,6-dimethyl-4-morpholinyl]-2-(2,6-dimethyl-4-pyridinyl)phenyl]methyl]-cyclopropaneacetamide (Compound 1)

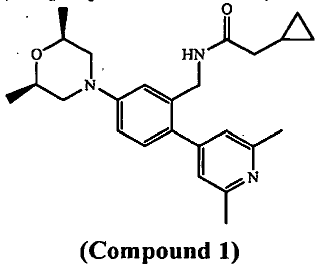

HBTU (1.00 g, 2.64 mmol) was added at room temperature to a mixture of Intermediate 3 (851 mg, 2.40 mmol), cyclopropylacetic acid (265 mg, 2.64 mmol), and DIPEA (0.91 mL, 5.29 mmol) in 20 ml dichloromethane. The mixture was stirred overnight at room temperature. The reaction mixture was washed with water and the organic layer was separated, dried with MgSO₄ and concentrated under reduced pressure. The residue was purified on a silica column with an eluent gradient from 100% DCM to 98/2 DCM/MeOH. The desired pure fractions were collected and evaporated. The product was triturated in hot iPr₂O/MeOH (70/30), cooled, filtered and dried in vacuum at 50 °C, yielding 485 mg (49 %) of Compound 1 as a yellow solid.

### B2) Example 2

### N-[[5-[(2R,6S)-2,6-dimethyl-4-morpholinyl]-2-(2,6-dimethyl-4-pyridinyl)phenyl]methyl]-2-methyl-propanamide (Compound 2)

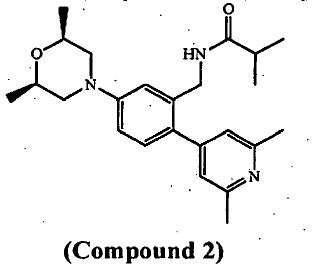

Isobutyryl chloride (1.09 g, 10.26 mmol) was added to a solution of Intermediate 3 (3.3 g, 9.33 mmol) and triethylamine (2.58 mL, 18.66 mmol) in DCM (200 mL). The reaction mixture was stirred for 1 h at room temperature. The reaction was washed with water and aqueous sodium carbonate. The aqueous layer was extracted with DCM. The combined organic layer was dried with MgSO₄, filtered and evaporated. The residue was purified by column chromatography on silica gel with an eluent gradient from 100% DCM to 95/5 DCM/MeOH. The desired pure fractions were collected and evaporated. The resulting solid was recrystallised from acetonitrile, yielding 3.4 g (92 %) of Compound 2 as a white powder.

The compounds of Tables 3 to 5 were prepared according to either Example 1 or 2.

**Table 3**

| | | | | | | | | | | | |
|---|---|---|---|---|---|---|---|---|---|---|---|
| 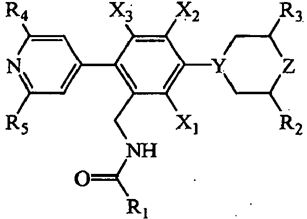 | | | | | | | | | | | |

| Co. No. | R₁ | R₂ | R₃ | R₄ | R₅ | X₁ | X₂ | X₃ | Y | Z | Stereo-chemistry |
|---|---|---|---|---|---|---|---|---|---|---|---|
| 1 | 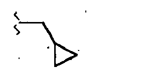 | CH₃ | CH₃ | CH₃ | CH₃ | H | H | H | N | O | cis |
| 2 | 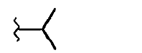 | CH₃ | CH₃ | CH₃ | CH₃ | H | H | H | N | O | cis |
| 3 | 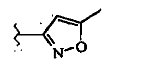 | CH₃ | CH₃ | CH₃ | CH₃ | H | H | H | N | O | cis |
| 4 | 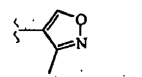 | CH₃ | CH₃ | CH₃ | CH₃ | H | H | H | N | O | cis |
| 5 | 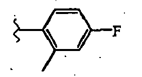 | CH₃ | CH₃ | CH₃ | CH₃ | H | H | H | N | O | cis |
| 6 | 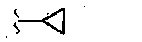 | CH₃ | CH₃ | CH₃ | CH₃ | H | H | H | N | O | cis |
| 7 | 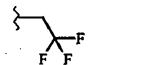 | CH₃ | CH₃ | CH₃ | CH₃ | H | H | H | N | O | cis |
| 8 | 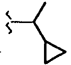 | CH₃ | CH₃ | CH₃ | C | H | H | H | N | O | cis |
| 9 | 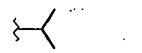 | CH₃ | CH₃ | CH₃ | H | H | H | H | N | O | cis |
| 10 | 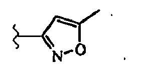 | CH₃ | CH₃ | CH₃ | H | H | H | H | N | O | cis |
| 11 | 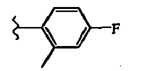 | CH₃ | CH₃ | CH₃ | H | H | H | H | N | O | cis |
| 12 | 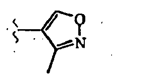 | CH₃ | CH₃ | CH₃ | H | H | H | H | N | O | cis |
| 13 | 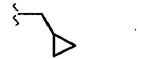 | CH₃ | CH₃ | CH₃ | H | H | H | H | N | O | cis |
| 14 | 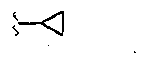 | CH₃ | CH₃ | CH₃ | H | H | H | H | N | O | cis |
| 15 | 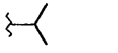 | CH₃ | CH₃ | H | H | H | H | H | N | O | cis |
| 16 | 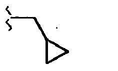 | CH₃ | CH₃ | H | H | H | H | H | N | O | cis |
| 17 | 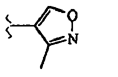 | CH₃ | CH₃ | H | H | H | H | H | N | O | cis |
| 18 | 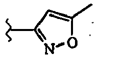 | CH₃ | CH₃ | CH₃ | CH₃ | H | H | H | N | CH₂ | cis |
| 19 | 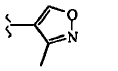 | CF₃ | CF₃ | CH₃ | CH₃ | H | H | H | N | CH₂ | cis |
| 20 | 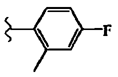 | CF₃ | CF₃ | CH₃ | CH₃ | H | H | H | N | CH₂ | cis |
| 21 | 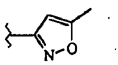 | CF₃ | CF₃ | CH₃ | CH₃ | H | H | H | N | CH₂ | cis |
| 22 | 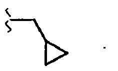 | CF₃ | CF₃ | CH₃ | CH₃ | H | H | H | N | CH₂ | cis |
| 23 | 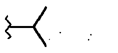 | CF₃ | CF₃ | CH₃ | CH₃ | H | H | H | N | CH₂ | cis |
| 24 | 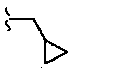 | H | H | CH₃ | CH₃ | H | H | H | N | O | |
| 25 | 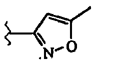 | H | H | CH₃ | CH₃ | H | H | H | N | O | |
| 26 | 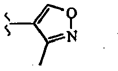 | H | H | CH₃ | CH₃ | H | H | H | N | O | |
| 27 | 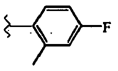 | H | H | CH₃ | CH₃ | H | H | H | N | O | |
| 28 | 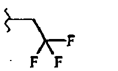 | H | H | CH₃ | CH₃ | H | H | H | N | O | |
| 29 | 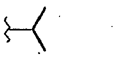 | H | H | CH₃ | CH₃ | H | H | H | N | O | |
| 30 | 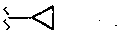 | H | H | CH₃ | CH₃ | H | H | H | N | O | |
| 31 | 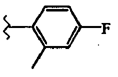 | H | H | CH₃ | H | H | H | H | N | O | |
| 32 | 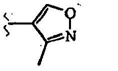 | CF₃ | H | CH₃ | CH₃ | H | H | H | N | CH₂ | |
| 33 | 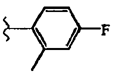 | CF₃ | H | CH₃ | CH₃ | H | H | H | N | CH₂ | |
| 34 | 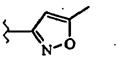 | CF₃ | H | CH₃ | CH₃ | H | H | H | N | CH₂ | |
| 35 | 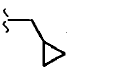 | CF₃ | H | CH₃ | CH₃ | H | H | H | N | CH₂ | |
| 36 | 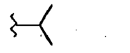 | CF₃ | H | CH₃ | CH₃ | H | H | H | N | CH₂ | |
| 37 | 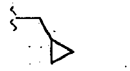 | CH₃ | CH₃ | CH₃ | CH₃ | F | H | H | N | O | cis |
| 38 | 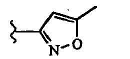 | CH₃ | CH₃ | CH₃ | CH₃ | F | H | H | N | O | cis |
| 39 | 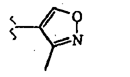 | CH₃ | CH₃ | CH₃ | CH₃ | F | H | H | N | O | cis |
| 40 | 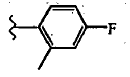 | CH₃ | CH₃ | CH₃ | CH₃ | F | H | H | N | O | cis |
| 41 | 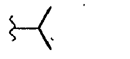 | CH₃ | CH₃ | CH₃ | CH₃ | F | H | H | N | O | cis |
| 42 | 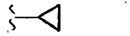 | CH₃ | CH₃ | CH₃ | CH₃ | F | H | H | N | O | cis |
| 43 | 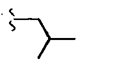 | CH₃ | CH₃ | CH₃ | CH₃ | F | H | H | N | O | cis |
| 44 | 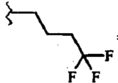 | CH₃ | CH₃ | CH₃ | CH₃ | F | H | H | N | O | cis |
| 45 | 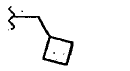 | CH₃ | CH₃ | CH₃ | CH₃ | F | H | H | N | O | cis |
| 46 | 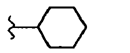 | CH₃ | CH₃ | CH₃ | CH₃ | F | H | H | N | O | cis |
| 47 | 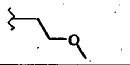 | CH₃ | CH₃ | CH₃ | CH₃ | F | H | H | N | O | cis |
| 48 | 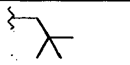 | CH₃ | CH₃ | CH₃ | CH₃ | F | H | H | N | O | cis |
| 49 | 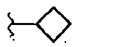 | CH₃ | CH₃ | CH₃ | CH₃ | F | H | H | N | O | cis |
| 50 | 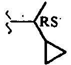 | CH₃ | CH₃ | CH₃ | CH₃ | F | H | H | N | O | cis |
| 51 | 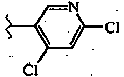 | CH₃ | CH₃ | CH₃ | CH₃ | F | H | H | N | O | cis |
| 52 | 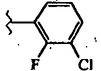 | CH₃ | CH₃ | CH₃ | CH₃ | F | H | H | N | O | cis |
| 53 | 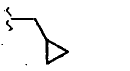 | CH₃ | CH₃ | CH₃ | CH₃ | F | H | H | CH | O | cis |
| 54 | 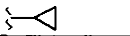 | CH₃ | CH₃ | CH₃ | CH₃ | F | H | H | CH | O | cis |
| 55 | 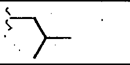 | CH₃ | CH₃ | CH₃ | CH₃ | F | H | H | CH | O | cis |
| 56 | 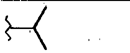 | CF₃ | H | CH₃ | CH₃ | F | H | H | N | O | R-enantiomer |
| 57 | 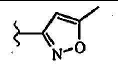 | CF₃ | H | CH₃ | CH₃ | F | H | H | N | O | R-enantiomer |
| 58 | 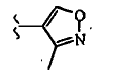 | CF₃ | H | CH₃ | CH₃ | F | H | H | N | O | R-enantiomer |
| 59 | 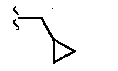 | CF₃ | H | CH₃ | CH₃ | F | H | H | N | O | R-enantiomer |
| 60 | 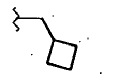 | CH₃ | CH₃ | CH₃ | CH₃ | F | H | H | CH | O | cis |
| 61 | 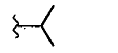 | CH₃ | CH₃ | CH₃ | CH₃ | F | H | H | CH | O | cis |
| 62 | 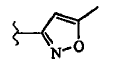 | CH₃ | CH₃ | CH₃ | CH₃ | F | H | H | CH | O | cis |
| 63 | 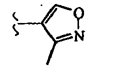 | CH₃ | CH₃ | CH₃ | CH₃ | F | H | H | CH | O | cis |
| 64 | 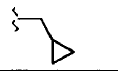 | CF₃ | H | CH₃ | CH₃ | F | H | H | N | O | S-enantiomer |
| 65 | 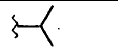 | CF₃ | H | CH₃ | CH₃ | F | H | H | N | O | S-enantiomer |
| 66 | 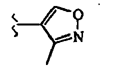 | CF₃ | H | CH₃ | CH₃ | F | H | H | N | O | S-enantiomer |
| 67 | 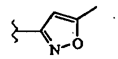 | CF₃ | H | CH₃ | CH₃ | F | H | H | N | O | S-enantiomer |
| 68 | 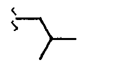 | CF₃ | H | CH₃ | CH₃ | F | H | H | N | O | S-enantiomer |
| 69 | 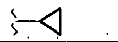 | CF₃ | H | CH₃ | CH₃ | F | H | H | N | O | S-enantiomer |
| 70 | 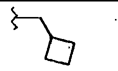 | CF₃ | H | CH₃ | CH₃ | F | H | H | N | O | S-enantiomer |
| 71 | 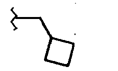 | CF₃ | H | CH₃ | CH₃ | F | H | H | N | O | R-enantiomer |
| 72 | 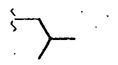 | CF₃ | H | CH₃ | CH₃ | F | H | H | N | O | R-enantiomer |
| 73 | 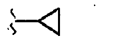 | CF₃ | H | CH₃ | CH₃ | F | H | H | N | O | R-enantiomer |
| 74 | 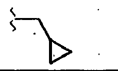 | CH₃ | CH₃ | CH₃ | CH₃ | H | H | F | N | O | cis |
| 75 | 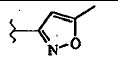 | CH₃ | CH₃ | CH₃ | CH₃ | H | H | F | N | O | cis |
| 76 | 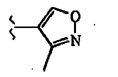 | CH₃ | CH₃ | CH₃ | CH₃ | H | H | F | N | O | cis |
| 77 | 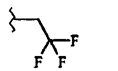 | CH₃ | CH₃ | CH₃ | CH₃ | H | H | F | N | O | cis |
| 78 | 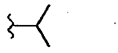 | CH₃ | CH₃ | CH₃ | CH₃ | H | H | F | N | O | cis |
| 79 | 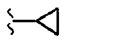 | CH₃ | CH₃ | CH₃ | CH₃ | H | H | F | N | O | cis |
| 80 | 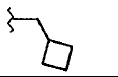 | CH₃ | CH₃ | CH₃ | CH₃ | H | H | F | N | O | cis |
| 81 | 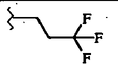 | CH₃ | CH₃ | CH₃ | CH₃ | H | H | F | N | O | cis |
| . 82 | 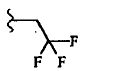 | CH₃ | CH₃ | CH₃ | CH₃ | H | H | F | N | O | cis |
| 83 | 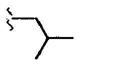 | CH₃ | CH₃ | CH₃ | CH₃ | H | H | F | N | O | cis |
| 84 | 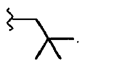 | CH₃ | CH₃ | CH₃ | CH₃ | H | H | F | N | O | cis |
| 85 | 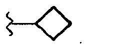 | CH₃ | CH₃ | CH₃ | CH₃ | H | H | F | N | O | cis |
| 86 | 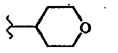 | CH₃ | CH₃ | CH₃ | CH₃ | H | H | F | N | O | cis |
| 87 | 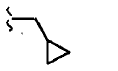 | CH₃ | CH₃ | H | CH₃ | H | H | F | N | O | cis |
| 88 | 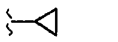 | CH₃ | CH₃ | H | CH₃ | H | H | F | N | O | cis |
| 89 | 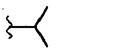 | CH₃ | CH₃ | H | CH₃ | H | H | F | N | O | cis |
| 90 | 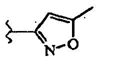 | CH₃ | CH₃ | H | CH₃ | H | H | F | N | O | cis |
| 91 | 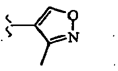 | CH₃ | CH₃ | H | CH₃ | H | H | F | N | O | cis |
| 92 | 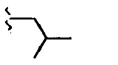 | CH₃ | CH₃ | H | CH₃ | H | H | F | N | O | cis |
| 93 | 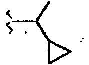 | CF₃ | H | CH₃ | CH₃ | H | H | F | N | CH₂ | |
| 94 | 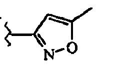 | CF₃ | H | CH₃ | CH₃ | H | H | F | N | CH₂ | |
| 95 | 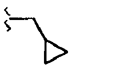 | CF₃ | H | CH₃ | CH₃ | H | H | F | N | CH₂ | |
| 96 | 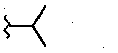 | CF₃ | H | CH₃ | CH₃ | H | H | F | N | CH₂ | |
| 97 | 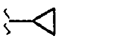 | CF₃ | H | CH₃ | CH₃ | H | H | F | N | CH₂ | |
| 98 | 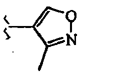 | CF₃ | H | CH₃ | CH₃ | H | H | F | N | CH₂ | |
| 99 | 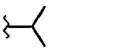 | CF₃ | H | CH₃ | CH₃ | H | H | F | N | O | R-enantiomer |
| 100 | 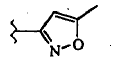 | CF₃ | H | CH₃ | CH₃ | H | H | F | N | O | R-enantiomer |
| 101 | 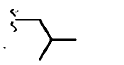 | CF₃ | H | CH₃ | CH₃ | H | H | F | N | O | R-enantiomer |
| 102 | 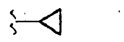 | CF₃ | H | CH₃ | CH₃ | H | H | F | N | O | R-enantiomer |
| 103 | 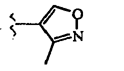 | CF₃ | H | CH₃ | CH₃ | H | H | F | N | O | R-enantiomer |
| 104 | 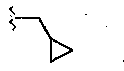 | CF₃ | H | CH₃ | CH₃ | H | H | F | N | O | R-enantiomer |
| 105 | 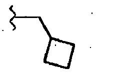 | CF₃ | H | CH₃ | CH₃ | H | H | F | N | O | S-enantiomer |
| 106 | 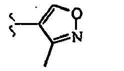 | CF₃ | H | CH₃ | CH₃ | H | H | F | N | O | S-enantiomer |
| 107 | 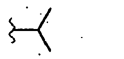 | CF₃ | H | CH₃ | CH₃ | H | H | F | N | O | S-enantiomer |
| 108 | 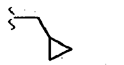 | CF₃ | H | CH₃ | CH₃ | H | H | F | N | O | S-enantiomer |
| 109 | 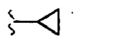 | CF₃ | H | CH₃ | CH₃ | H | H | F | N | O | S-enantiomer |
| 110 | 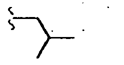 | CF₃ | H | CH₃ | CH₃ | H | H | F | N | O | S-enantiomer |
| 111 | 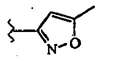 | CF₃ | H | CH₃ | CH₃ | H | H | F | N | O | S-enantiomer |
| 112 | 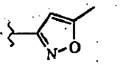 | CH₃ | H | CH₃ | CH₃ | H | H | F | N | O | |
| 113 | 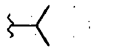 | CH₃ | H | CH₃ | CH₃ | H | H | F | N | O | |
| 114 | 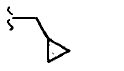 | CH₃ | H | CH₃ | CH₃ | H | H | F | N | O | |
| 115 | 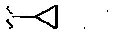 | CH₃ | H | CH₃ | CH₃ | H | H | F | N | O | |
| 116 | 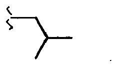 | CH₃ | H | CH₃ | CH₃ | H | H | F | N | O | |
| 117 | 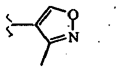 | CH₃ | H | CH₃ | CH₃ | H | H | F | N | O | |
| 118 | 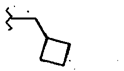 | CH₃ | H | CH₃ | CH₃ | H | H | F | N | O | |
| 119 | 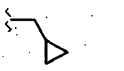 | CH₃ | CH₃ | CH₃ | CH₃ | H | H | Cl | N | O | cis |
| 120 | 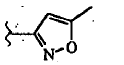 | CH₃ | CH₃ | CH₃ | CH₃ | H | H | Cl | N | O | cis |
| 121 | 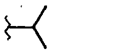 | CH₃ | CH₃ | CH₃ | CH₃ | H | H | Cl | N | O | cis |
| 122 | 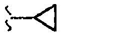 | CH₃ | CH₃ | CH₃ | CH₃ | F | H | F | N | O | cis |
| 123 | 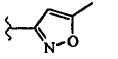 | CH₃ | CH₃ | CH₃ | CH₃ | F | H | F | N | O | cis |
| 124 | 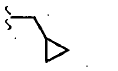 | CH₃ | CH₃ | CH₃ | CH₃ | F | H | F | N | O | cis |
| 125 | 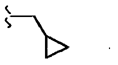 | CH₃ | CH₃ | CH₃ | CH₃ | H | F | H | N | O | cis |
| 126 | 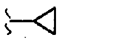 | CH₃ | CH₃ | CH₃ | CH₃ | H | F | H | N | O | cis |
| 127 | 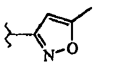 | CH₃ | CH₃ | CH₃ | CH₃ | H | F | H | N | O | cis |
| 128 | 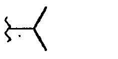 | CH₃ | CH₃ | CH₃ | CH₃ | H | F | H | N | O | cis |
| 137 | 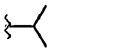 | H | H | H | H | H | H | H | N | O | |
| 138 | 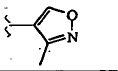 | H | H | H | H | H | H | H | N | O | |
| 139 | 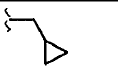 | H | H | H | H | H | H | H | N | O | |
| 140 | 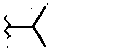 | H | H | CH₃ | H | H | H | H | N | O | |
| 141 | 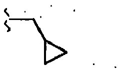 | H | H | CH₃ | H | H | H | H | N | O | |
| 142 | 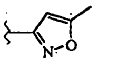 | H | H | CH₃ | H | H | H | H | N | O | |
| 143 | 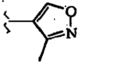 | H | H | CH₃ | H | H | H | H | N | O | |

**Table 4**

| | | |
|---|---|---|
| | | 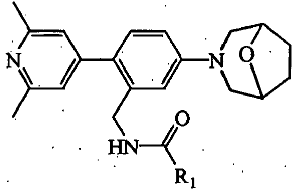 |

| Co.No. | R₁ | |
|---|---|---|
| 129 | 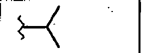 | |
| 130 | 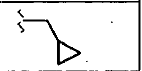 | |
| 131 | 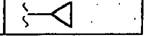 | |

**Table 5**

| | | |
|---|---|---|
| | | 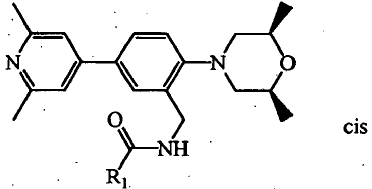 |

| Co.No. | R₁ | |
|---|---|---|
| 132 | 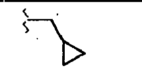 | |
| 133 | 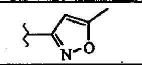 | |
| 134 | 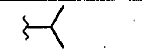 | |
| 135 | 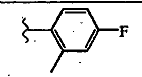 | |
| 136 | 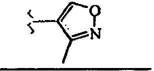 | |

### Analytical Part

### LCMS (Liquid Chromatography/Mass spectrometry)

### LCMS General procedure A

The LC measurement was performed using an Acquity UPLC (Waters) system comprising a binary pump, a sample organizer, a column heater (set at 55 °C), a diode-array detector (DAD) and a column as specified in the respective methods below. Flow from the column was split to a MS spectrometer. The MS detector was configured with an electrospray ionization source. Mass spectra were acquired by scanning from 100 to 1000 in 0.18 seconds using a dwell time of 0.02 seconds. The capillary needle voltage was 3.5 kV and the source temperature was maintained at 140 °C. Nitrogen was used as the nebulizer gas. Data acquisition was performed with a Waters-Micromass MassLynx-Openlynx data system.

### LCMS General procedure B

The HPLC measurement was performed using an Alliance HT 2790 (Waters) system comprising a quaternary pump with degasser, an autosampler, a column oven (set at 40 °C, unless otherwise indicated), a diode-array detector (DAD) and a column as specified in the respective methods below. Flow from the column was split to a MS spectrometer. The MS detector was configured with an electrospray ionization source. Mass spectra were acquired by scanning from 100 to 1000 in 1 second using a dwell time of 0.1 second. The capillary needle voltage was 3 kV and the source temperature was maintained at 140 °C. Nitrogen was used as the nebulizer gas. Data acquisition was performed with a Waters-Micromass MassLynx-Openlynx data system.

### LCMS Method 1

In addition to general procedure A: Reversed phase UPLC (Ultra Performance Liquid Chromatography) was carried out on a bridged ethylsiloxane/silica hybrid (BEH) C18 column (1.7 µm, 2.1 x 50 mm; Waters Acquity) with a flow rate of 0.8 ml/min. Two mobile phases (mobile phase A: 0.1 % formic acid in H₂O/methanol 95/5; mobile phase B: methanol) were used to run a gradient condition from 95 % A and 5 % B to 5 % A and 95 % B in 1.3 minutes and hold for 0.2 minutes. An injection volume of 0.5 µl was used.

Cone voltage was 10 V for positive ionization mode and 20 V for negative ionization mode.

### LCMSMethod 2

In addition to general procedure A: Reversed phase UPLC (Ultra Performance Liquid Chromatography) was carried out on a bridged ethylsiloxane/silica hybrid (BEH) C18 column (1.7 µm, 2.1 x 50 mm; Waters Acquity) with a flow rate of 0.8 ml/min. Two mobile phases (25 mM ammonium acetate in H₂O/acetonitrile 95/5; mobile phase B: acetonitrile) were used to run a gradient condition from 95 % A and 5 % B to 5 % A and 95 % B in 1.3 minutes and hold for 0.3 minutes. An injection volume of 0.5 µl was used.

Cone voltage was 30 V for positive ionization mode and 30 V for negative ionization mode.

### LCMS Method 3

In addition to general procedure B: Reversed phase HPLC was carried out on an Xterra MS C18 column (3.5 µm, 4.6 x 100 mm) with a flow rate of 1.6 ml/min. Three mobile phases (mobile phase A: 95% 25 mM ammoniumacetate + 5 % acetonitrile; mobile phase B: acetonitrile; mobile phase C: methanol) were employed to run a gradient condition from 100 % A to 1 % A, 49 % B and 50 % C in 6.5 minutes, to 1 % A and 99 % B in 1 minute and hold these conditions for 1 minute and reequilibrate with 100 % A for 1.5 minutes. An injection volume of 10 µl was used. Cone voltage was 10 V for positive ionization mode and 20 V for negative ionization mode.

### LCMS Method 4

In addition to the general procedure B: Reversed phase HPLC was carried out on an Xterra MS C18 column (3.5 µm, 4.6 x 100 mm) with a flow rate of 1.6 ml/min. Three mobile phases (mobile phase A: 95% 25 mM ammoniumacetate + 5 % acetonitrile; mobile phase B: acetonitrile; mobile phase C: methanol) were employed to run a gradient condition from 100 % A to 1 % A, 49 % B and 50 % C in 6.5 minutes, to 1 % A, 99 % B in 0.5 minute and keep these conditions for 1 minute. An injection volume of 10 µl was used.

Cone voltage was 10 V for positive ionization mode and 20 V for negative ionization mode.

### Melting Points

For a number of compounds, melting points were determined with a DSC823e from Mettler-Toledo. Melting points were measured with a temperature gradient of 30°C/minute. Values are peak values.

The results of the analytical measurements are shown in table 6.

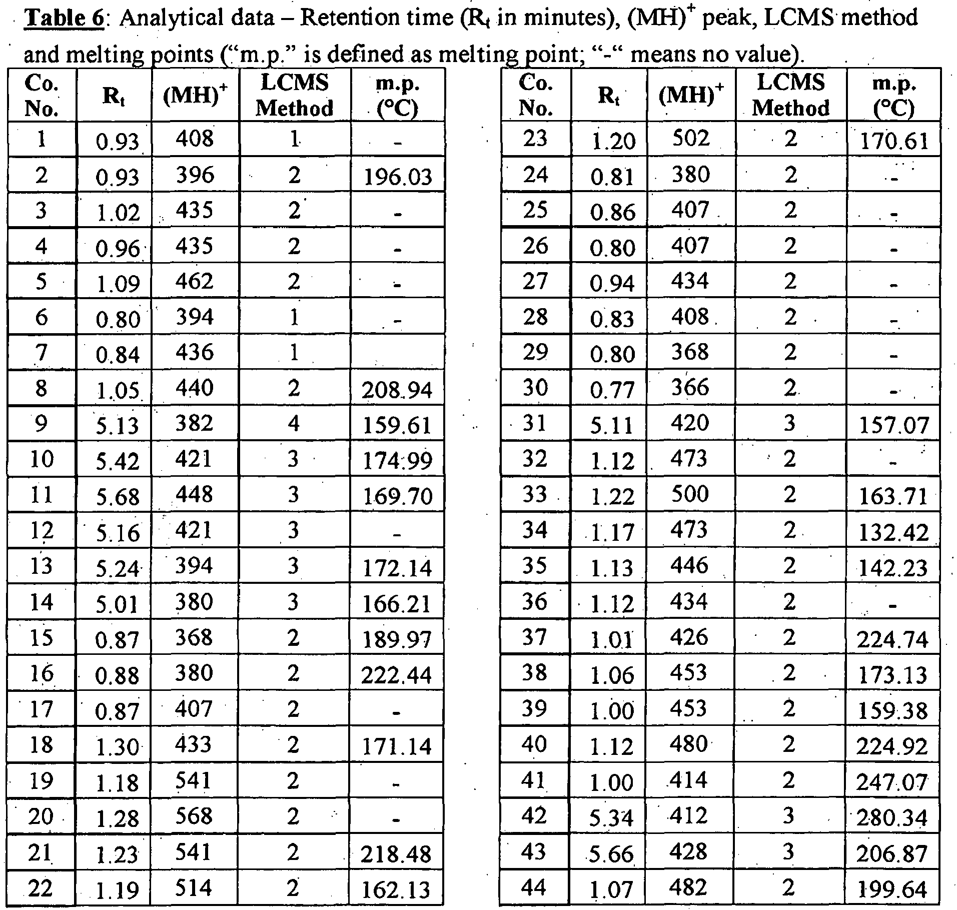

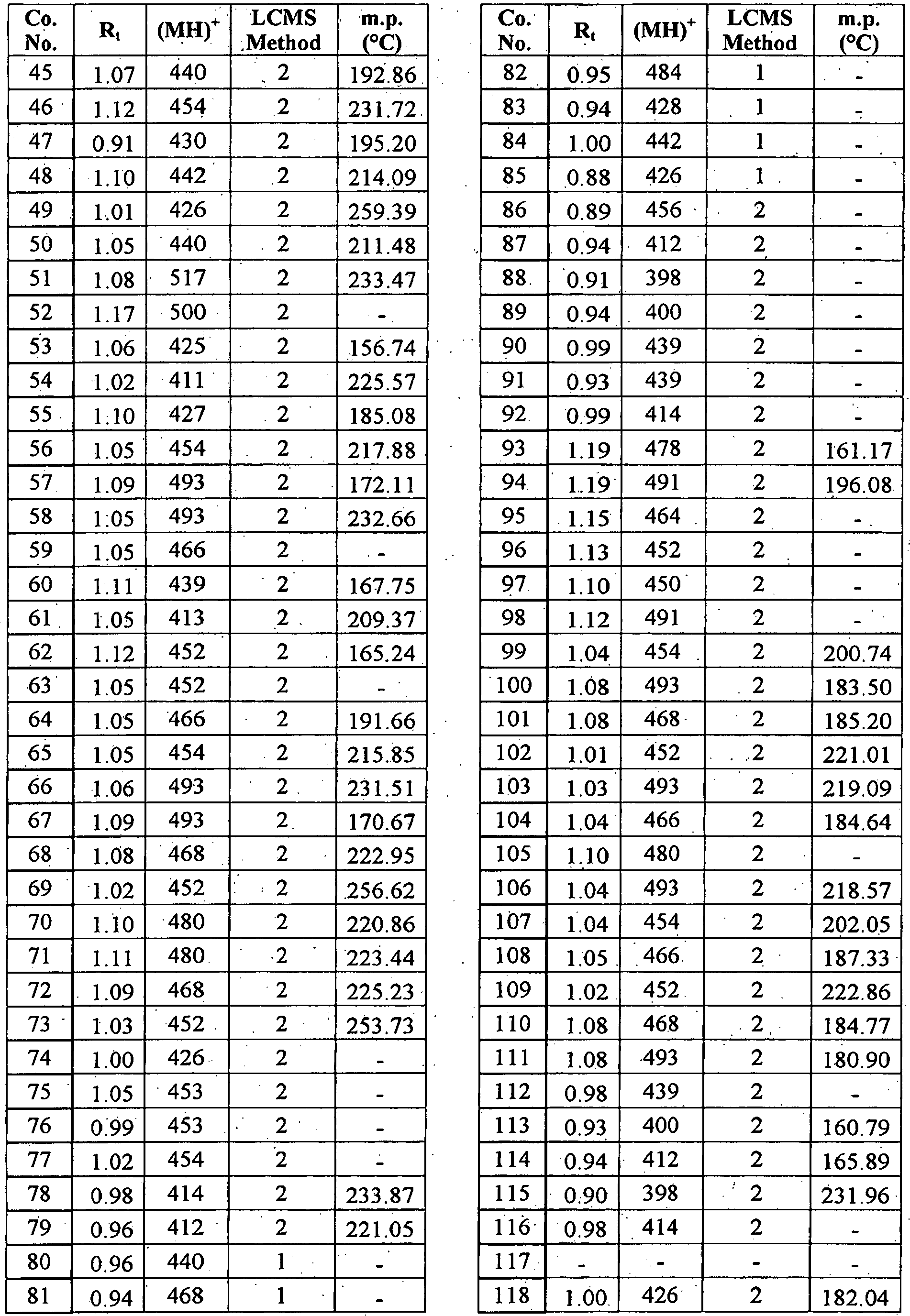

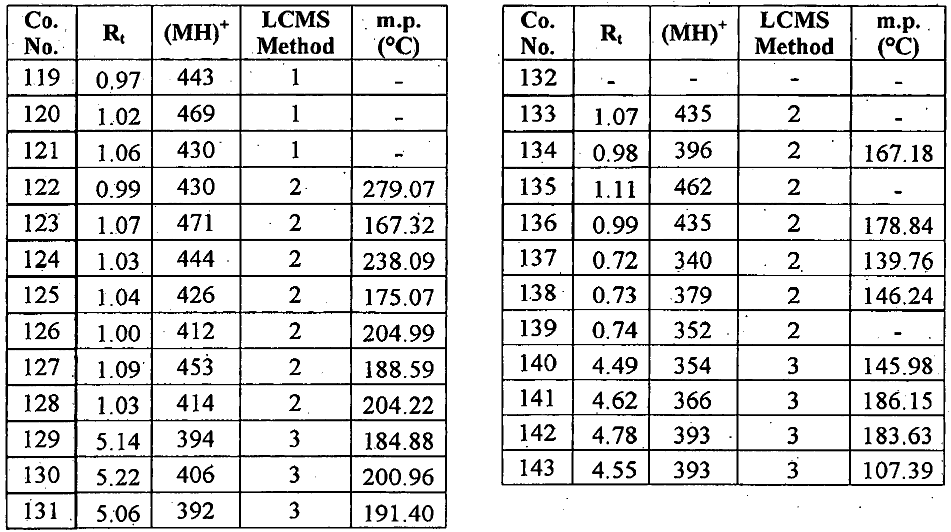

### Optical Rotation (OR)

The optical rotation was measured using a Perkin Elmer 341 polarimeter. [α]_{D}²⁰ indicates the optical rotation measured with light at the wavelength (λ) of 589 nm, at a temperature of 20 °C, in MeOH. The cell pathlength is 1 dm. Behind the actual value the concentration which was used to measure the optical rotation is mentioned.

### NMR (nuclear magnetic resonance)

For a number of compounds, ¹H NMR spectra were recorded on a Bruker DPX-360, on a Bruker DPX-400 or on a Bruker Avance 600 spectrometer with standard pulse sequences, operating at 360 MHz, 400 MHz and 600 MHz respectively, using CHLOROFORM-*d* (deuterated chloroform, CDCl₃) or DMSO-*d*₆ (deuterated DMSO, dimethyl-d6 sulfoxide) as solvents. Chemical shifts (δ) are reported in parts per million (ppm) relative to tetramethylsilane (TMS), which was used as internal standard.

Compound 1 : ¹H NMR (360 MHz, CHLOROFORM-d) δ ppm 0.12 (m, J=4.8, 4:8, 4.8 Hz, 2 H) 0.48 - 0.58 (m, 2 H) 0.73 - 0.90 (m, 1 H) 1.28 (d, J=6.6 Hz, 6 H) 2.11 (d, J=7.3 Hz, 2 H) 2.41 - 2.51 (m, 2 H) 2.56 (s, 6 H) 3.51 (m, J=12.4 Hz, 2 H) 3.74 - 3.87 (m, 2 H) 4.46 (d, J=5.5 Hz, 2 H) 5.93 (t, J=5.3 Hz, 1 H) 6.88 (dd, J=8.4, 2.6 Hz, 1 H) 6.92 (s, 2 H) 6.95 (d, J=2.2 Hz, 1 H) 7.14 (d, J=8.8 Hz, 1 H)

Compound 2 : ¹H NMR (360 MHz, CHLOROFORM-d) δ ppm 1.10 (d, J=7.0 Hz, 6 H) 1.28 (d, J=6.6 Hz, 6 H) 2.29 (spt, J=6.9 Hz, 1 H) 2.45 (dd, J=11.9, 10.8 Hz, 2 H) 2:55 (s, 6 H) 3.50 (dd, J=12.6, 2.0 Hz, 2 H) 3.71 - 3.89 (m, 2 H) 4.41 (d, J=5.5 Hz, 2 H) 5.45 (t, J=4.9 Hz, 1 H) 6.81 - 6.94 (m, 4 H) 7.14 (d, J=8.4 Hz, 1 H)

Compound 9 : ¹H NMR (360 MHz, DMSO-d6) δ ppm 0.98 (d, J=6.6 Hz, 6 H) 1.16 (d, J=6.2 Hz, 6 H) 2.22 - 2.33 (m, 2 H) 2.39 (spt, J=7.0 Hz, 1 H) 2.48 (s, 3 H) 3.56 - 3.64 (m, 2 H) 3.64 - 3.76 (m, 2 H) 4.18 (d, J=5.5 Hz, 2 H) 6.87 - 6.99 (m, 2 H) 7.09 - 7.17 (m, 2 H) 7.20 (s, 1 H) 8.16 (t, J=5.5 Hz, 1 H) 8.42 (d, J=5.1 Hz, 1 H)

Compound 37 : ¹H NMR (360 MHz, CHLOROFORM-d) δ ppm 0.13 (m, J=4.9, 4.9, 4.9 Hz, 2 H) 0.50 - 0.61 (m, 2 H) 0.76 - 0.97 (m, 1 H) 1.25 (d, J=6.2 Hz, 6 H) 2.09 (d, J=7.0 Hz, 2 H) 2.50 (t, J=11.0 Hz, 2 H) 2.57 (s, 6 H) 3.31 (d, J=11.0 Hz, 2 H) 3.81 - 3.97 (m, 2 H) 4.48 (d, J=4.0 Hz, 2 H) 6.04 (t, J=4.0 Hz, 1 H) 6.88 - 7.03 (m, 4 H)

Compound 59 : ¹H NMR (360 MHz, DMSO-d6) δ ppm 0.02 - 0.14 (m, 2 H) 0.33 - 0:47 (m, 2 H) 0.83 - 1.03 (m, 1 H) 1.97 (d, J=7.0 Hz, 2 H) 2.44 (s, 6 H) 2.82 - 3.02 (m, 2 H) 3.29 (d, J=12.1 Hz, 1 H) 3.47 (d, J=11.3 Hz, 1 H) 3.84 (td, J=11.1, 2.0 Hz, 1 H) 4.02 - 4.20 (m, 3 H) 4.33 - 4:50 (m, 1 H) 7.01 (s, 2 H) 7.09 (d, J=8.4 Hz, 1 H) 7.17 (t, J=8.6 Hz, 1 H) 8.01 (t, J=4.4 Hz, 1 H)

Compound 74 : ¹H NMR (360 MHz, CHLOROFORM-d) δ ppm 0.08 - 0.18 (m, 2 H) 0.47 - 0.61 (m, 2 H) 0.72 - 0.91 (m, 1 H) 1.28 (d, J=6.2 Hz, 6 H) 2.10 (d, J=7.3 Hz, 2 H) 2.47 (t, J=11.2 Hz, 2 H) 2.56 (s, 6 H) 3.41 - 3.54 (m, 2 H) 3.67 - 3.87 (m, 2 H) 4.32 (d, J=5.9 Hz, 2 H) 5.90 (t, J=5.7 Hz, 1 H) 6.58 (dd, J=12.8, 2.2 Hz, 1 H) 6.75 (d, J=2.6 Hz, 1 H) 6.90 (s, 2 H)

Compound 75 : ¹H NMR (360 MHz, CHLOROFORM-d) δ ppm 1.27 (d, J=6.2 Hz, 6 H) 2.46 (m, J=11.3, 11.3 Hz, 5 H) 2.54 (s, 6 H) 3.47 (dd, J=12.1, 1.8 Hz, 2 H) 3.70 - 3.85 (m, 2 H) 4.40 (d; J=5.9 Hz, 2 H) 6.36 - 6.43 (m, 1 H) 6.59 (dd, J=12.6, 2.4 Hz, 1 H) 6.72 - 6.83 (m, 2 H) 6.89 (s, 2 H)

Compound 78 : ¹H NMR (360 MHz, CHLOROFORM-d) δ ppm 1.10 (d, J=7.0 Hz, 6 H) 1.27 (d, J=6.2 Hz, 6 H) 2.28 (spt, J=7.1 Hz, 1 H) 2.39 - 2.51 (m, 2 H) 2.56 (s, 6 H) 3.47 (dd, J=12.1, 1.8 Hz, 2 H) 3.69 - 3.86 (m, 2 H) 4.27 (d, J=5.9 Hz, 2 H) 5.43 (t, J=4.6 Hz, 1 H) 6.57 (dd, J=12.6, 2.4 Hz, 1 H) 6.70 (d, J=2.2 Hz, 1 H) 6.88 (s, 2 H)

Compound 104 : ¹H NMR (360 MHz, DMSO-d6) δ ppm 0.08 - 0.16 (m, 2 H) 0.35 - 0.47 (m, 2 H) 0.86 - 1.00 (m, 1 H) 1.98 (d, J=7.0 Hz, 2 H) 2.45 (s, 6 H) 2.73 - 2.91 (m, 2 H) 3.65 (d, J=12.4 Hz, 1 H) 3.71 - 3.85 (m, 2 H) 4.04 (d, J=5.9 Hz, 2 H) 4.07 - 4.15 (m, 1 H) 4.29 - 4.45 (m, 1 H) 6.84 (d, J=1.8 Hz, 1 H) 6.93 (dd, J=13.2, 2.2 Hz, 1 H) 6.97 (s, 2 H) 8.13 (t, J=5.7 Hz, 1 H)

### D. Pharmacological examples

### Example D.1 : Ca²⁺ flux imaging (FDSS) (protocol B)

### Materials

a) Assay buffer
   Hanks buffered saline solution (HBSS, Invitrogen, Belgium), supplemented with 10 mM HEPES (Invitrogen, Belgium), CaCl₂ to a final concentration of 5 mM, 0.1 % Bovine serum albumin (Sigma-Aldrich NV, Belgium).
b) Calcium-sensitive dye - Fluo-4AM
   Fluo-4AM (Molecular Probes, USA) was dissolved in DMSO containing 10% Pluronic acid (Molecular Probes, USA) to give a stock solution which was diluted in assay buffer supplemented with 5 mM probenicid (Sigma, Aldrich NV, Belgium) to give a final concentration of 2 µM
c) 384-well plates
   Black-sided, transparent bottomed 384 well plates coated with poly-D-lysine, PDL pre-coated (Coming, Incorporated, USA)
d) Calcium flux measurement
   A Functional drug screening system (FDSS, Hamamatsu) was used to measure intracellular free-calcium flux signals.

### Method

Monolayers of human alpha 7-wt nAChR-expressing cells were grown in black-sided, transparent bottomed 384 well plates coated with PDL for 24 hours prior to loading with a fluorescent calcium indicator, fluo-4AM for up to 120 minutes.

PAM activity was detected in real time by applying the compounds to be tested to the loaded cells along with an alpha 7 nicotinic receptor agonist during constant monitoring of cellular fluorescence in a FDSS. Compounds giving peak fluorescent responses greater than the response due to agonist alone, were consideted to be alpha 7 nAChR PAMs. The alpha 7 nicotinic receptor agonist was choline, applied at a sub-maximal concentration of 100 µM. In a further setting of the present invention the compounds were applied prior to the alpha 7 nicotinic receptor agonist, in a particular 10 minutes prior to the agonist.

A control response to choline was calculated on each plate from the difference in peak in fluorescence in wells receiving either choline or assay buffer alone. Compounds of the present invention were tested at a concentration range from 0.01 µM to 30 µM. Compounds were considered to have an interesting activity when they potentiated the choline signal at least with 200 % when tested at a concentration of 30 µM (the efficacy of 100 µM choline was defined as 100% in the absence of a PAM). An EC₅₀ (or pEC₅₀) was determined as a concentration relating to half the maximal effect, when a clear sigmoidal curve with top plateau was obtained. The EC₅₀ (or pEC₅₀) was defined as lower than maximal concentration in case the compound activity did not reach a top plateau at maximal concentration (indicated in table 7 as "< 5")

The compounds also have a potentiating effect on the response to choline when measured by whole-cell patch clamp electrophysiology in GH4C1 cells stably over-expressing the human wild-type alpha 7 receptor.

### Example D.2 : Patch-clamp current recording

Patch-clamp recording from mammalian cells has provided a powerful means of assessing the function of membrane-bound proteins thought to be subunits of ligand-gated ion channels. Activation of such proteins by endogenous or exogenous ligands cause opening of a pore associated with the receptor through which ions flow down their electrochemical gradient. In the case of the human alpha 7-wt nAChR-expressing GH4C1 recombinant cell line the preferential permeability to calcium of this receptor means that calcium flows into the cell upon activation by ACh, choline and other nicotinic ligands giving rise to a calcium current. Since this receptor rapidly desensitizes in the presence of agonist it is important that an application system is used which is capable of very rapid switching of solutions (< 100 ms) to prevent partial or full desensitisation of receptor responses coincident with the time of agonist application. Consequently, a second convenient technique to assess the enhancement of nicotinic efficacy is a patch-clamp recording from human alpha 7-wt nAChR-expressing GH4C1 cells coupled with a rapid-application system.

### Materials

a) Assay buffers
   The external recording solution consisted of 152 mM NaCl, 5 mM KCI, 1 mM MgCl₂, 1 mM Calcium, 10 mM HEPES ; pH 7.3. The internal recording solution consisted of 140 mM CsCl, 10 mM HEPES, 10 mM EGTA, 1 mM MgCl₂, pH 7.3.
b) Patch-clamp recording was carried out using a Patch -clamp amplifier (Multiclamp 700A, Axon Instruments, CA, USA). Human alpha 7-wt nAChR-expressing GH4Cl cells were patch-clamped in the whole cell configuration (Hamill et al, 1981) with a borosilicate glass electrode of 1.5-3 MΩ tip resistance when filled with the internal recording solution. Recordings were made on cells with membrane resistance >500 MΩ and more preferably 1GΩ and series resistance <15 MΩ with at least 60% series resistance compensation. Membrane potential was clamped at -70 mV.
c) Agonists
   ACh, choline,were purchased from Sigma-Aldrich NV, Belgium.
d) Compound application
A 16-channel Dynflow DF-16 microfluidics system (Cellectricon, Sweden) for rapid switching of solutions (switching resolution time <100 ms) was used to apply control, agonist and PAM compounds to human alpha 7-wt nAChR-expressing GH4C1 cells.

### Method

Human alpha 7-wt nAChR-expressing GH4C1 cells were plated in external recording solution in the Dynaflow perfusion chamber and were allowed to settle for up to 20 minutes. Individual cells were whole-cell patched and gently lifted off the chamber bottom with the patch pipette into a continuously-flowing perfusion stream (12 µl/min) of external recording solution. PAM activity was detected in real time by pre-applying the compounds to the loaded cells followed by an alpha 7 nicotinic receptor agonist during constant monitoring of cellular membrane current. Compounds giving current responses greater than the response due to agonist alone, were considered to be alpha 7 nAChR PAM's. The alpha 7 nicotinic receptor was activated by a non-selective nicotinic agonist, choline applied at a sub-maximal concentration of 1 mM. In a further setting of the present invention the compounds were applied prior to the alpha 7 nicotinic receptor agonist, 30 seconds prior to the agonist or 5 seconds prior to the agonist. A control response was calculated from the area under the curve of the current elicited in each cell to the application of submaximal choline for 250 ms. Area under the curve is the integration of net current over time and is a common representation of the total ion flux through the channel. Increases in agonist efficacy elicited by a positive allosteric modulator were calculated as percent potentiation of "area under curve" (AUC) of the agonist response. Potentiation greater than control AUC caused by compounds of the invention indicates that they are expected to have useful therapeutic activity. EC₅₀ values (potency), maximal effect (% efficacy), and Hill slopes were estimated by fitting the data to the logistic equation using GraphPad Prism (GraphPad Software, Inc., San Diego, CA).

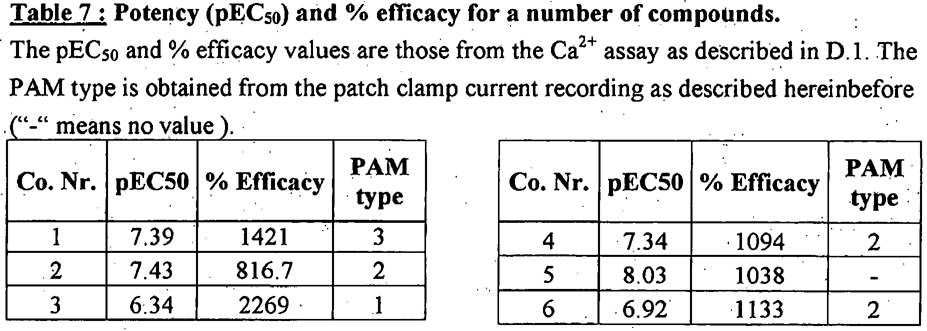

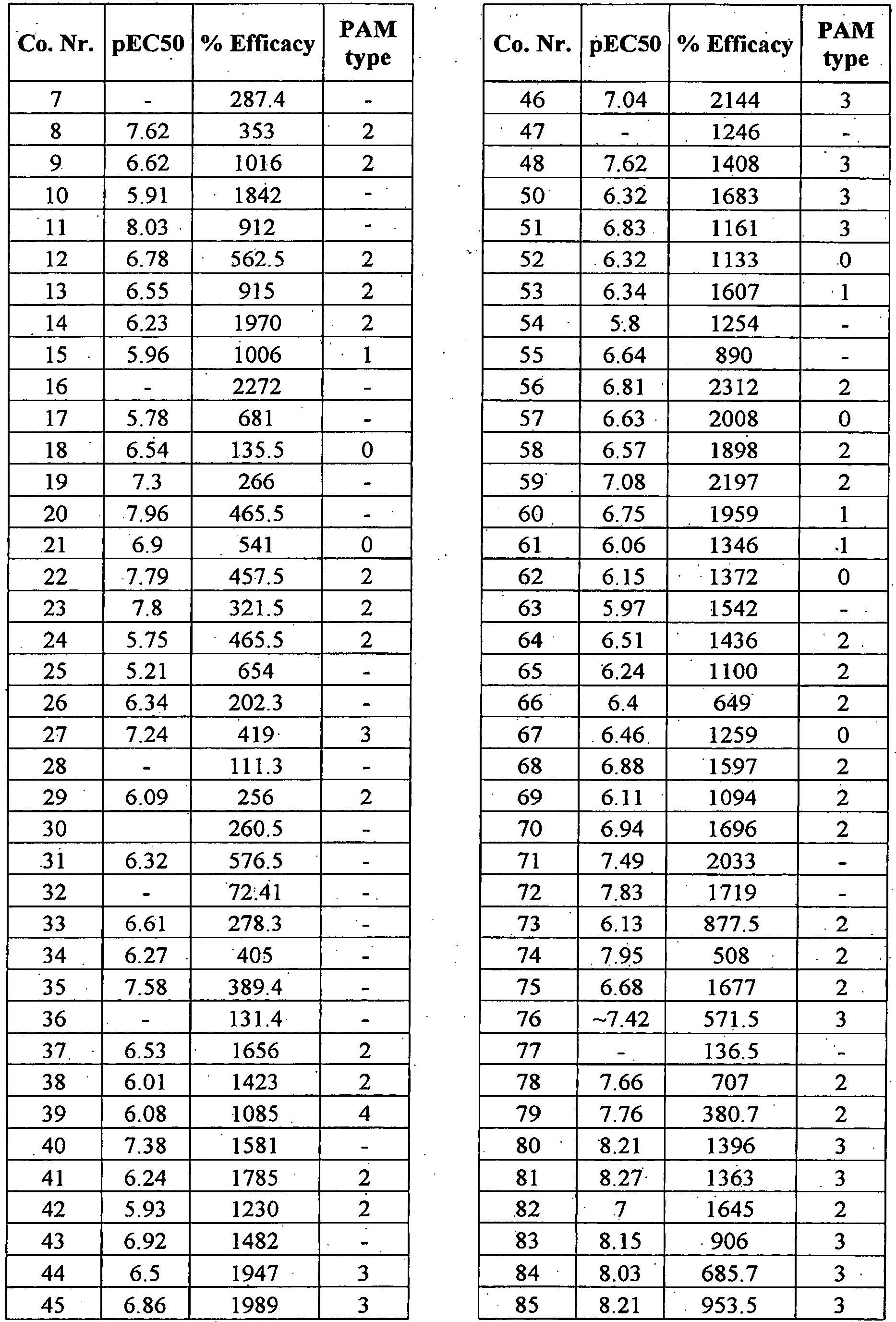

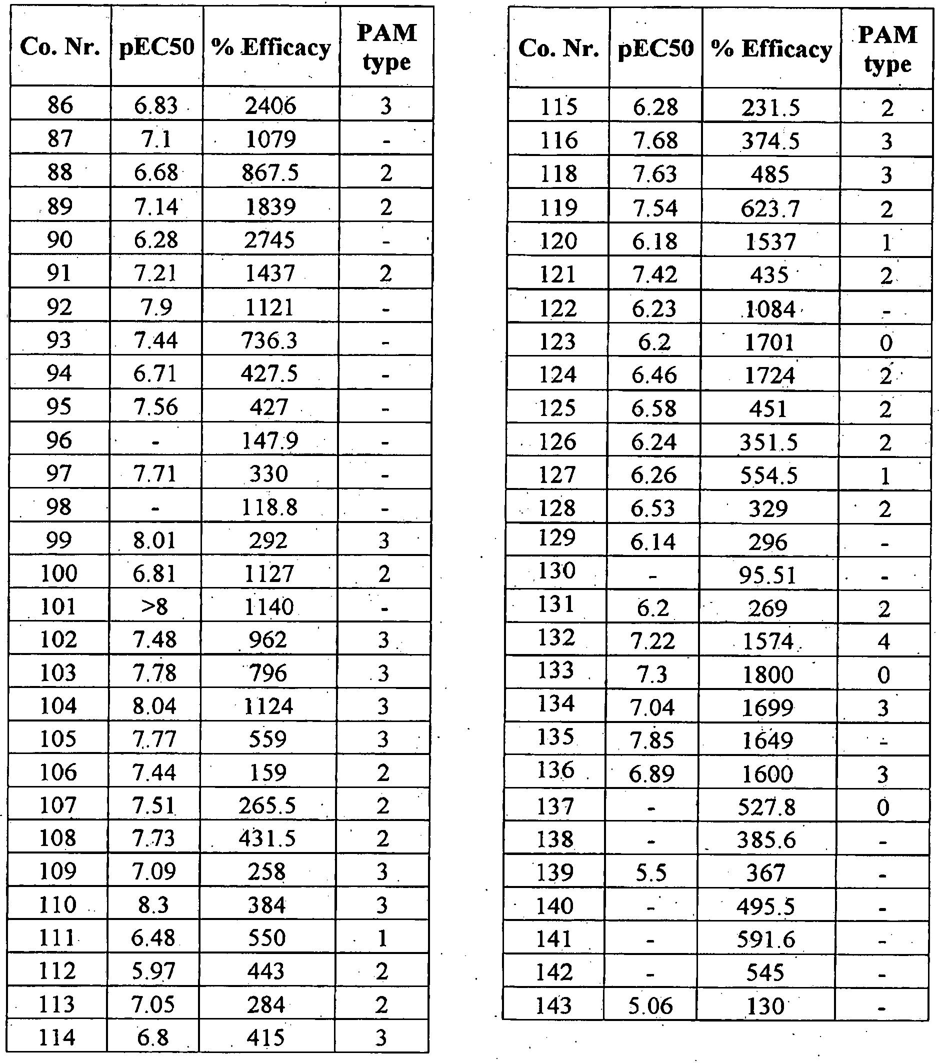

## Claims

1. A compound having the formula (I) 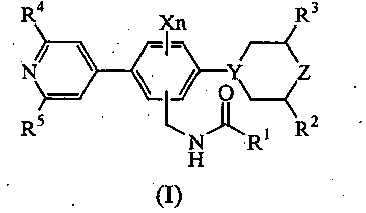 or a stereoisomer thereof, wherein
n is 0, 1 or 2;
X is fluoro or chloro;
Y is N or CH;
2 is O or CH₂;
R¹ is C₁₋₈alkyl; C₁₋₈alkyl substituted with 1, 2 or 3 halogen substituents, C₃₋₆cyclo-alkyl; (C₃₋₆cycloalkyl)C₁₋₆alkyl; (C₁₋₆alkyloxy)C₁₋₆alkyl; (trihaloC₁₋₄alkyloxy)-C₁₋₆alkyl; tetrahydrofuryl; tetrahydropyranyl; phenyl; or phenyl substituted with 1, 2 or 3 substituents selected from halogen, trifluoromethyl, trifluoromethoxy, cyano, C₁₋₆alkyl, and C₁₋₄alkyloxy; or a monocyclic aromatic heterocyclic radical containing at least one heteroatom selected from N, O and S, optionally substituted with 1, 2 or where possible with 3 substituents selected from halogen, C₁₋₄alkyl, C₁₋₄alkyloxy, C₃₋₆cycloalkyl, and trifluoromethyl;
R² and R³ are independently H, C₁₋₄alkyl or trifluoromethyl;
or R² and R³ are taken together to form 1,2-ethanediyl or 1,3-propanediyl;
R⁴ and R⁵ are independently H, C₁₋₄alkyl, trifluoromethyl, C₃₋₆cycloalkyl or C₁₋₄alkyloxy;
or an acid addition salt thereof, or a solvate thereof.

2. The compound according to claim 1 wherein
R¹ is C₁₋₆alkyl; C₁₋₄alkyl substituted with 3 fluoro substituents; C₃₋₆cycloalkyl; (C₃₋₆cycloalkyl)C₁₋₂alkyl; methoxymethyl; methoxyethyl; tetrahydropyranyl; phenyl; phenyl substituted with 1, 2 or 3 substituents selected from fluoro, chloro, trifluoromethyl, trifluoromethoxy, cyano, methyl, and methoxy;
or furanyl, oxazolyl, isoxazolyl, oxadiazolyl, pyrrolyl, pyrazolyl, imidazolyl, pyridinyl, pyridiminyl, pyrazinyl, pyridazinyl, thienyl, 1,2,3-thiadiazolyl, thiazolyl or benzisoxazolyl, each unsubstituted or substituted with 1, 2 or where possible 3 substituents selected from methyl, ethyl, propyl, isopropyl, butyl, isobutyl, tert-butyl, cyclopropyl, methoxy or trifluoromethyl.

3. The compound according to claim 1 wherein R² is hydrogen, methyl or trifluoromethyl.

4. The compound according to claim 1 wherein R³ is hydrogen, methyl or trifluoromethyl.

5. The compound according to claim 1 wherein R⁴ is hydrogen or methyl.

6. The compound according to claim 1 wherein R⁵ is hydrogen or methyl.

7. The compound according to claim 1 wherein R¹ is methyl, ethyl, *n*-propyl, isopropyl, *n*-butyl, isobutyl, sec-butyl, tert-butyl, 2,2,2-trifluorethyl, 3,3,3-trifluoropropyl, 2-methoxyethyl, cyclopropyl, cyclobutyl, cyclopentyl, 1-(cyclopropyl)ethyl, (cyclopropyl)methyl, (cyclobutyl)methyl, 4-fluoro-2-methylphenyl, 3-methyl-isoxazol-5-yl, 3-methyl-isoxazol-4-yl, 5-methyl-isoxazol-3-yl, 2-methyl-5-trifluoromethyl-oxazol-4-yl, 2-methyl-oxazol-4-yl.

8. The compound according to claim 1 wherein R² and R³ are methyl or trifluoromethyl and have the cis-configuration.

9. The compound according to claim 1 wherein R⁴ and R⁵ are methyl.

10. The compound according to claim 1 wherein the compound is N-[[5-[(2R,6S)-2,6-dimethyl-4-morpholinyl]-2-(2,6-dimethyl-4-pyridinyl)phenyl]methyl]-2-methyl-propanamide.

11. A pharmaceutical composition comprising as active ingredient a compound as defined in any one of claims 1 to 10.

12. A product comprising
(a) a compound of formula (I) as defined in claim 1, and
(b) a α7 nicotinic receptor agonist selected from
1,4-Diazabicyclo[3.2.2]nonane-4-carboxylic acid, 4-bromophenyl ester, monohydrochloride (SSR180711A);
(-)-spiro[1-azabicyclo[2.2.2]octane-3,5'-oxazolidine]-2'-one;
(+)-*N-*(1-azabicyclo[2.2.2]oct-3-yl)benzo[b]furan-2-carboxamide;
3-[(2,4-Dimethoxy)Benzylidene]-Anabaseine Dihydrochloride (GTS-21); [*N*-[(3R)-1-Azabicyclo[2.2.2]oct-3-yl]-4-chlorobenzamide Hydrochloride] PNU-282987; nicotine; varenicline; A-582941; AR-R17779; TC-1698;
PHA-709829; tropisetron; WAY-317538; MEM3454; EVP-6124; TC-5619;
MEM-63908; and AZD-0328, as a combined preparation for simultaneous, separate or sequential use in preventing or treating of psychotic disorders, intellectual impairment disorders, or inflammatory diseases.

13. A compound as defined in any one of claims 1 to 10 for use as a medicine.

14. A compound as defined in any one of claims 1 to 10 for use in treating Alzheimer's disease, Lewy Body Dementia, Attention Deficit Hyperactivity Disorder, anxiety, schizophrenia, mania, manic depression, Parkinson's disease, Huntington's disease, Tourette's syndrome, brain trauma, jetlag, nicotine addiction, pain; endotoxaemia, endotoxic shock, sepsis, rheumatoid arthritis, asthma, multiple sclerosis, psoriasis, urticaria, inflammatory bowel disease, inflammatory bile disease, Crohn's disease, ulcerative colitis, post-operative ileus, pancreatitis, heart failure, acute lung injury or allograft rejection; cognition in schizophrenia, cognition in Alzheimer's disease, mild cognitive impairment or arthritis.

15. A process of preparing a pharmaceutical composition as defined in claim 11 comprising the step of intimately mixing a pharmaceutically acceptable carrier with a therapeutically effective amount of a compound as defined in any one of claims 1 to 10.

## Patentansprüche

1. Verbindung der Formel (I) 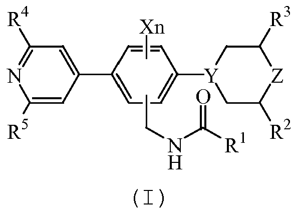 oder ein Stereoisomer davon, wobei
n für 0, 1 oder 2 steht;
X für Fluor oder Chlor steht;
Y für N oder CH steht;
Z für 0 oder CH₂ steht;
R¹ für C₁₋₈-Alkyl; durch 1, 2 oder 3 Halogensubstituenten substituiertes C₁₋₈-Alkyl; C₃₋₆-Cycloalkyl; (C₃₋₆-Cycloalkyl) -C₁₋₆-alkyl; (C₁₋₆-Alkyloxy) -C₁₋₆-alkyl; (Trihalogen-C₁₋₄-alkyloxy)-C₁₋₆-alkyl; Tetrahydrofuryl; Tetrahydropyranyl; Phenyl; oder durch 1, 2 oder 3 aus Halogen, Trifluormethyl, Trifluormethoxy, Cyano, C₁₋₆-Alkyl und C₁₋₄-Alkyloxy ausgewählte Substituenten substituiertes Phenyl; oder einen monocyclischen aromatischen heterocyclischen Rest mit mindestens einem aus N, O und S ausgewählten Heteroatom, gegebenenfalls substituiert durch 1, 2 oder, falls möglich, 3 aus Halogen, C₁₋₄-Alkyl, C₁₋₄-Alkyloxy, C₃₋₆-Cycloalkyl und Trifluormethyl ausgewählte Substituenten, steht;
R² und R³ unabhängig voneinander für H, C₁₋₄-Alkyl oder Trifluormethyl stehen;
oder R² und R³ zusammen 1,2-Ethandiyl oder 1,3-Propandiyl bilden;
R⁴ und R⁵ unabhängig voneinander für H, C₁₋₄-Alkyl, Trifluormethyl, C₃₋₆-Cycloalkyl oder C₁₋₄-Alkyloxy stehen;
oder ein Säureadditionssalz davon, oder ein Solvat davon.

2. Verbindung nach Anspruch 1, wobei
R¹ für C₁₋₆-Alkyl; durch 3 Fluorsubstituenten substituiertes C₁₋₄-Alkyl; C₃₋₆-Cycloalkyl; (C₃₋₆-Cycloalkyl)-C₁₋₂-alkyl; Methoxymethyl; Methoxyethyl; Tetrahydropyranyl; Phenyl; durch 1, 2 oder 3 aus Fluor, Chlor, Trifluormethyl, Trifluormethoxy, Cyano, Methyl und Methoxy ausgewählte Substituenten substituiertes Phenyl;
oder Furanyl, Oxazolyl, Isoxazolyl, Oxadiazolyl, Pyrrolyl, Pyrazolyl, Imidazolyl, Pyridinyl, Pyrimidinyl, Pyrazinyl, Pyridazinyl, Thienyl, 1,2,3-Thiadiazolyl, Thiazolyl oder Benzisoxazolyl, jeweils unsubstituiert oder durch 1, 2 oder, wo möglich, 3 aus Methyl, Ethyl, Propyl, Isopropyl, Butyl, Isobutyl, tert.-Butyl, Cyclopropyl, Methoxy und Trifluormethyl ausgewählte Substituenten substituiert, steht.

3. Verbindung nach Anspruch 1, wobei R² für Wasserstoff, Methyl oder Trifluormethyl steht.

4. Verbindung nach Anspruch 1, wobei R³ für Wasserstoff, Methyl oder Trifluormethyl steht.

5. Verbindung nach Anspruch 1, wobei R⁴ für Wasserstoff oder Methyl steht.

6. Verbindung nach Anspruch 1, wobei R⁵ für Wasserstoff oder Methyl steht.

7. Verbindung nach Anspruch 1, wobei R¹ für Methyl, Ethyl, *n*-Propyl, Isopropyl, *n*-Butyl, Isobutyl, sec.-Butyl, tert.-Butyl, 2,2,2-Trifluorethyl, 3,3,3-Trifluorpropyl, 2-Methoxyethyl, Cyclopropyl, Cyclobutyl, Cyclopentyl, 1-(Cyclopropyl)ethyl, (Cyclopropyl)methyl, (Cyclobutyl)methyl, 4-Fluor-2-methylphenyl, 3-Methylisoxazol-5-yl, 3-Methyl-isoxazol-4-yl, 5-Methylisoxazol-3-yl, 2-Methyl-5-trifluormethyloxazol-4-yl, 2-Methyloxazol-4-yl steht.

8. Verbindung nach Anspruch 1, wobei R² und R³ für Methyl oder Trifluormethyl stehen und die cis-Konfiguration ausweisen.

9. Verbindung nach Anspruch 1, wobei R⁴ und R⁵ für Methyl stehen.

10. Verbindung nach Anspruch 1, wobei es sich bei der Verbindung um N-[[5-[(2R,6S)-2,6-Dimethyl-4-morpholinyl]-2-(2,6-dimethyl-4-pyridinyl)phenyl]methyl]-2-methylpropanamid handelt.

11. Pharmazeutische Zusammensetzung, umfassend als Wirkstoff eine wie in einem der Ansprüche 1 bis 10 definierte Verbindung.

12. Produkt, umfassend
(a) eine wie in Anspruch 1 definierte Verbindung der Formel (I) und
(b) einen nicotinischen α7-Rezeptoragonisten, ausgewählt aus
1,4-Diazabicyclo[3.2.2]nonan-4-carbonsäure-4-bromphenylestermonohydrochlorid (SSR180711A);
(-)-spiro[1-Azabicyclo[2.2.2]octan-3,5'-oxazolidin)-2'-on;
(+)-*N*-(1-Azabicyclo[2.2.2]oct-3-yl)benzo[b]furan-2-carbonsäureamid; 3-[(2,4-Dimethoxy)benzyliden]anabaseindihydrochlorid (GTS-21);
[*N*-[(3R)-1-Azabicyclo[2.2.2]oct-3-yl]-4-chlorbenzamidhydrochlorid] PNU-282987; Nicotin; Vareniclin; A-582941; AR-R17779; TC-1698; PHA-709829; Tropisetron; WAY-317538; MEM3454; EVP-6124; TC-5619; MEM-63908 und AZD-0328, als Kombinationspräparat zur gleichzeitigen, separaten oder aufeinanderfolgenden Anwendung bei der Prävention oder Behandlung psychotischer Erkrankungen, Erkrankungen mit intellektuellen Störungen oder entzündlichen Krankheiten.

13. Verbindung, definiert wie in einem der Ansprüche 1 bis 10, zur Verwendung als Medizin.

14. Verbindung, definiert wie in einem der Ansprüche 1 bis 10, zur Verwendung bei der Behandlung von Alzheimer-Krankheit, Lewis-Körperchen-Demenz, Aufmerksamkeitsdefizit-Hyperaktivitätssyndrom, Angst, Schizophrenie, Manie, manischer Depression, Parkinson-Krankheit, Chorea Huntington, Tourette-Syndrom, Hirntrauma, Jetlag, Nikotinabhängigkeit, Schmerzen, Endotoxämie, endotoxischem Schock, Sepsis, rheumatoider Arthritis, Asthma, multipler Sklerose, Psoriasis, Nesselsucht, entzündlicher Darmkrankheit, entzündlicher Gallenkrankheit, Morbus Crohn, Colitis ulcerosa, postoperativem Ileus, Pankreatitis, Herzinsuffizienz, akuter Lungenverletzung oder Allograftabstoßung, Kognition bei Schizophrenie, Kognition bei Alzheimer-Krankheit, milder kognitiver Störung oder Arthritis.

15. Verfahren zur Herstellung einer wie in Anspruch 11 definierten pharmazeutischen Zusammensetzung, bei dem man einen pharmazeutisch unbedenklichen Träger innig mit einer therapeutisch wirksamen Menge einer Verbindung nach einem der Ansprüche 1 bis 10 mischt.

## Revendications

1. Composé répondant à la formule (I) 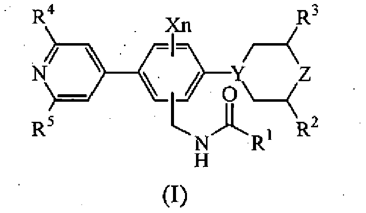 ou de l'un des stéréoisomères de celui-ci, où
n est égal à 0, 1 ou 2 ;
X représente un groupement fluoro ou chloro ;
Y représente N ou CH ;
Z représente O ou CH₂ ;
R¹ représente un groupement alkyle en C₁₋₈ ; alkyle en C₁₋₈ substitué par 1, 2 ou 3 substituants halogène ; cycloalkyle en C₃₋₆ ; (cycloalkyle en C₃₋₆)-alkyle en C₁₋₆ ; (alkoxy en C₁₋₆)-alkyle en C₁₋₆ ; (trihalogénoalkoxy en C₁₋₄)-alkyle en C₁₋₆ ; tétrahydrofuryle ; tétrahydropyranyle ; phényle ; ou phényle substitué par 1, 2 ou 3 substituants choisis parmi les atomes d'halogène et les groupements trifluorométhyle, trifluorométhoxy, cyano, alkyle en C₁₋₆ et alkoxy en C₁₋₄ ; ou un radical hétérocyclique monocyclique aromatique contenant au moins un hétéroatome choisi parmi N, O et S, éventuellement substitué par 1, 2 ou, lorsque cela est possible, 3 substituants choisis parmi les atomes d'halogène et les groupements alkyle en C₁₋₄, alkoxy en C₁₋₄, cycloalkyle en C₃₋₆ et trifluorométhyle ;
chacun des radicaux R² et R³ représente indépendamment H ou un groupement alkyle en C₁₋₄ ou trifluorométhyle ;
ou R² et R³ forment ensemble un groupement 1,2-éthanediyle ou 1,3-propanediyle ;
chacun des radicaux R⁴ et R⁵ représente indépendamment H ou un groupement alkyle en C₁₋₄, trifluorométhyle, cycloalkyle en C₃₋₆ ou alkoxy en C₁₋₄ ;
ou l'un des sels d'addition acide de celui-ci, ou l'un des solvates de celui-ci.

2. Composé selon la revendication 1, où
R¹ représente un groupement alkyle en C₁₋₆ ; alkyle en C₁₋₄ substitué par 3 substituants fluoro ; cycloalkyle en C₃₋₆; (cycloalkyle en C₃₋₆) -alkyle en C₁₋₂ ; méthoxyméthyle ; méthoxyéthyle ; tétrahydropyranyle ; phényle ; phényle substitué par 1, 2 ou 3 substituants choisis parmi les groupements fluoro, chloro, trifluorométhyle, trifluorométhoxy, cyano, méthyle et méthoxy ;
ou un groupement furanyle, oxazolyle, isoxazolyle, oxadiazolyle, pyrrolyle, pyrazolyle, imidazolyle, pyridinyle, pyridiminyle, pyrazinyle, pyridazinyle, thiényle, 1,2,3-thiadiazolyle, thiazolyle ou benzisoxazolyle, chacun étant non substitué ou substitué par 1, 2 ou, lorsque cela est possible, 3 substituants choisis parmi les groupements méthyle, éthyle, propyle, isopropyle, butyle, isobutyle, tert-butyle, cyclopropyle, méthoxy ou trifluorométhyle.

3. Composé selon la revendication 1, où R² représente un atome d'hydrogène ou un groupement méthyle ou trifluorométhyle.

4. Composé selon la revendication 1, où R³ représente un atome d'hydrogène ou un groupement méthyle ou trifluorométhyle.

5. Composé selon la revendication 1, où R⁴ représente un atome d'hydrogène ou un groupement méthyle.

6. Composé selon la revendication 1, où R⁵ représente un atome d'hydrogène ou un groupement méthyle.

7. Composé selon la revendication 1, où R¹ représente un groupement méthyle, éthyle, *n*-propyle, isopropyle, *n*-butyle, isobutyle, sec-butyle, tert-butyle, 2,2,2-trifluoroéthyle, 3,3,3-trifluoropropyle, 2-méthoxyéthyle, cyclopropyle, cyclobutyle, cyclopentyle, 1-(cyclopropyl)éthyle, (cyclopropyl)méthyle, (cyclobutyl)méthyle, 4-fluoro-2-méthylphényle, 3-méthyl-isoxazol-5-yle, 3-méthyl-isoxazol-4-yle, 5-méthyl-isoxazol-3-yle, 2-méthyl-5-trifluorométhyl-oxazol-4-yle, 2-méthyl-oxazol-4-yle.

8. Composé selon la revendication 1, où chacun des radicaux R² et R³ représente un groupement méthyle ou trifluorométhyle et présente la configuration cis.

9. Composé selon la revendication 1, où chacun des radicaux R⁴ et R⁵ représente un groupement méthyle.

10. Composé selon la revendication 1, où le composé est le N-[[5-[(2R,6S)-2,6-diméthyl-4-morpholinyl]-2-(2,6-diméthyl-4-pyridinyl)phényl]méthyl]-2-méthyl-propanamide.

11. Composition pharmaceutique comprenant au titre de principe actif le composé selon l'une quelconque des revendications 1 à 10.

12. Produit comprenant
(a) un composé de formule (I) selon la revendication 1, et
(b) un agoniste de récepteur α7-nicotinique choisi parmi les suivants :
Acide 1,4-diazabicyclo[3.2.2]nonane-4-carboxylique, ester de 4-bromophényle, monochlorhydrate (SSR180711A) ;
(-)-spiro[1-azabicyclo[2.2.2.]octane-3,5'-oxazolidine]-2'-one ;
(+)-N-(1-azabicyclo[2.2.2]oct-3-yl)benzo[b]furan-2-carboxamide ;
3-[(2,4-Diméthoxy)benzylidène]-anabaséine dichlorhydrate (GTS-21) ;
[N-[(3R)-1-Azabicyclo[2.2.2]oct-3-yl]-4-chlorobenzamide chlorhydrate] PNU-282987 ;
Nicotine ;
Varénicline ;
A-582941 ;
AR-R17779 ;
TC-1698 ;
PHA-709829 ;
tropisétron ;
WAY-317538 ;
MEM3454 ;
EVP-6124 ;
TC-5619 ;
MEM-63908 ;
et AZD-0328, sous forme d'une préparation combinée pour utilisation simultanée, séparée ou séquentielle dans le traitement prophylactique ou thérapeutique des troubles psychotiques, des troubles de dysfonctionnement intellectuel, ou des maladies inflammatoires.

13. Composé selon l'une quelconque des revendications 1 à 10 pour utilisation en tant que médicament.

14. Composé selon l'une quelconque des revendications 1 à 10 pour utilization dans le traitement des maladies suivantes : maladie d'Alzheimer, démence à corps de Lewy, trouble de déficit d'attention avec hyperactivité, anxiété, schizophrénie, manie, trouble maniaco-dépressif, maladie de Parkinson, chorée de Huntington, syndrome de Gilles de la Tourette, traumatismes cérébraux, décalage horaire, addiction à la nicotine et la douleur ; endotoxémie, choc endotoxique, sepsie, polyarthrite rhumatoïde, asthme, sclérose en plaques, psoriasis, urticaire, maladie inflammatoire chronique de l'intestin, maladie inflammatoire des voies biliaires, maladie de Crohn, rectocolite hémorragique, occlusion intestinale postopératoire, pancréatite, crise cardiaque, lésion pulmonaire aiguë ou rejet d'allogreffe ; dysfonctionnement cognitif dans la schizophrénie, dysfonctionnement cognitif dans la maladie d'Alzheimer, déficit cognitif léger ou arthrite.

15. Procédé d'élaboration d'une composition pharmaceutique selon la revendication 11, comprenant l'étape de mélangeage intime d'un vecteur pharmaceutiquement acceptable à une quantité thérapeutiquement active d'un composé selon l'une quelconque des revendications 1 à 10.
